# EUROPEAN PATENT SPECIFICATION

(11) **EP 1 007 691 B1**
(45) Date of publication and mention of the grant of the patent: **22.09.2004**
(21) Application number: 98918175.5
(22) Date of filing: 10.04.1998
(51) Int. Cl.: C12N 15/53, C12N 15/83, C12N 5/10, C12P 7/64, C11B 1/00, A61K 31/20, A23L 1/30, A23K 1/00

(54) **METHODS AND COMPOSITIONS FOR SYNTHESIS OF LONG CHAIN POLYUNSATURATED FATTY ACIDS**
VERFAHREN UND ZUSAMMENSETZUNGENFÜR DIE SYNTHESE VON LANGKETTIGEN, MEHRFACH UNGESÄTTIGTEN FETTSÄUREN
PROCEDES ET COMPOSITIONS POUR LA SYNTHESE D'ACIDES GRAS POLYINSATURES A CHAINE LONGUE

(30) Priority: 11.04.1997 US 833610
(43) Date of publication of application: 14.06.2000
(73) Proprietor: Calgene LLC, Davis, California 95616 (US); ABBOTT LABORATORIES, Abbott Park, Illinois 60064-3500 (US)
(72) Inventor: KNUTZON, Deborah, Granite Bay, CA 95746 (US); MUKERJI, Pradip, Gahanna, OH 43230 (US); HUANG, Yung-Sheng, Upper Arlington, OH 43220 (US); THURMOND, Jennifer, Columbus, OH 43231 (US); CHAUDHARY, Sunita, Pearland, TX 77584 (US); LEONARD, Amanda, Eun-Yeong, Gahanna, OH 43230 (US)
(74) Representative: Brasnett, Adrian Hugh
(86) International application number: PCT/US1998/007422
(87) International publication number: WO 1998/046765

(56) References cited:
- EP-A- 0 561 569
- WO-A-93/06712
- WO-A-94/18337
- WO-A-96/21022
- COVELLO P. ET AL.: "Functional expression of the extraplastidial Arabidopsis thaliana oleate desaturase gene (FAD2) in Saccharomyces cerevisiae" PLANT PHYSIOLOGY, vol. 111, no. 1, May 1996, pages 223-226, XP002075211
- SPYCHALLA J. ET AL.: "Identification of an animal w3 fatty acid desaturase by heterologous expression in Arabidopsis" PNAS,U.S.A., vol. 94, no. 4, 18 February 1997, pages 1142-1147, XP002076628
- HILLIER L. ET AL.: "The WashU-Merck EST Project, AC W49761" EMBL DATABASE, 30 May 1996, XP002076629 Heidelberg
- NATHANS J.: "Adult human retina cDNA, AC W28140" EMBL DATABASE, 14 May 1996, XP002076630 Heidelberg
- HILLIER L. ET AL.: "The WashU-Merck EST Project, AC W67716" EMBL DATABASE, 16 June 1996, XP002076631 Heidelberg
- HILLIER L. ET AL.: "The WashU-Merck EST Project, AC H17219" EMBL DATABSE, 1 July 1995, XP002076632 Heidelberg
- HILLIER L. ET AL.: "The WashU-Merck EST Project, AC H19385" EMBL DATABASE, 7 July 1995, XP002076633 Heidelberg
- YOSHINOR. ET AL.: "AC C25549" EMBL DATABASE, 24 July 1997, XP002076634 Heidelberg
- CADENA D. ET AL.: "AC AF002668" EMBL DATABASE, 4 July 1997, XP002076635 Heidelberg
- MICHAELSON L. ET AL.: "Isolation of a delta5-fatty acid desaturase gene from Mortierella alpina" JOURNAL OF BIOLOGICAL CHEMISTRY, vol. 273, no. 30, 24 July 1998, pages 19055-19059, XP002076636

### SEQUENCE LISTING

(1) GENERAL INFORMATION:
   (i) APPLICANT: KNUTZON, DEBORAH
      MURKERJI, PRADIP
      HUANG, YUNG-SHENG
      THURMOND, JENNIFER
      CHAUDHARY, SUNITA
      LEONARD, AMANDA
   (ii) TITLE OF INVENTION: METHODS AND COMPOSITIONS FOR SYNTHESIS OF LONG CHAIN POLY-UNSATURATED FATTY ACIDS
   (iii) NUMBER OF SEQUENCES: 34
   (iv) CORRESPONDENCE ADDRESS:
      (A) ADDRESSEE: LIMBACH & LIMBACH LLP
      (B) STREET: 2001 FERRY BUILDING
      (C) CITY: SAN FRANCISCO
      (D) STATE: CALIFORNIA
      (E) COUNTRY: USA
      (F) ZIP: 94111
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: MICHAEL R. WARD
      (B) REGISTRATION NUMBER: 38,651
      (C) REFERENCE/DOCKET NUMBER: CGAB-110
   (ix) TELECOMMUNICATION INFORMATION:
      (A) TELEPHONE: (415) 433-4150
      (B) TELEFAX: (415) 433-8716
      (C) TELEX: N/A
(2) INFORMATION FOR SEQ ID NO:1:
   (i) SEQUENCE CHARACTERISTICS:
      (A) LENGTH: 1483 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
   GCTTCCTCCA GTTCATCCTC CATTTCGCCA CCTGCATTCT TTACGACCGT TAAGCAAGAT 60
   GGGAACGGAC CAAGGAAAAA CCTTCACCTG GGAAGAGCTG GCGGCCCATA ACACCAAGGA 120
   CGACCTACTC TTGGCCATCC GCGGCAGGGT GTACGATGTC ACAAAGTTCT TGAGCCGCCA   180
   TCCTGGTGGA GTGGACACTC TCCTGCTCGG AGCTGGCCGA GATGTTACTC CGGTCTTTGA   240
   GATGTATCAC GCGTTTGGGG CTGCAGATGC CATTATGAAG AAGTACTATG TCGGTACACT   300
   GGTCTCGAAT GAGCTGCCCA TCTTCCCGGA GCCAACGGTG TTCCACAAAA CCATCAAGAC   360
   GAGAGTCGAG GGCTACTTTA CGGATCGGAA CATTGATCCC AAGAATAGAC CAGAGATCTG   420
   GGGACGATAC GCTCTTATCT TTGGATCCTT GATCGCTTCC TACTACGCGC AGCTCTTTGT   480
   GCCTTTCGTT GTCGAACGCA CATGGCTTCA GGTGGTGTTT GCAATCATCA TGGGATTTGC   540
   GTGCGCACAA GTCGGACTCA ACCCTCTTCA TGATGCGTCT CACTTTTCAG TGACCCACAA   600
   CCCCACTGTC TGGAAGATTC TGGGAGCCAC GCACGACTTT TTCAACGGAG CATCGTACCT   660
   GGTGTGGATG TACCAACATA TGCTCGGCCA TCACCCCTAC ACCAACATTG CTGGAGCAGA   720
   TCCCGACGTG TCGACGTCTG AGCCCGATGT TCGTCGTATC AAGCCCAACC AAAAGTGGTT   780
   TGTCAACCAC ATCAACCAGC ACATGTTTGT TCCTTTCCTG TACGGACTGC TGGCGTTCAA   840
   GGTGCGCATT CAGGACATCA ACATTTTGTA CTTTGTCAAG ACCAATGACG CTATTCGTGT   900
   CAATCCCATC TCGACATGGC ACACTGTGAT GTTCTGGGGC GGCAAGGCTT TCTTTGTCTG   960
   GTATCGCCTG ATTGTTCCCC TGCAGTATCT GCCCCTGGGC AAGGTGCTGC TCTTGTTCAC  1020
   GGTCGCGGAC ATGGTGTCGT CTTACTGGCT GGCGCTGACC TTCCAGGCGA ACCACGTTGT  1080
   TGAGGAAGTT CAGTGGCCGT TGCCTGACGA GAACGGGATC ATCCAAAAGG ACTGGGCAGC  1140
   TATGCAGGTC GAGACTACGC AGGATTACGC ACACGATTCG CACCTCTGGA CCAGCATCAC  1200
   TGGCAGCTTG AACTACCAGG CTGTGCACCA TCTGTTCCCC AACGTGTCGC AGCACCATTA  1260
   TCCCGATATT CTGGCCATCA TCAAGAACAC CTGCAGCGAG TACAAGGTTC CATACCTTGT  1320
   CAAGGATACG TTTTGGCAAG CATTTGCTTC ACATTTGGAG CACTTGCGTG TTCTTGGACT  1380
   CCGTCCCAAG GAAGAGTAGA AGAAAAAAAG CGCCGAATGA AGTATTGCCC CCTTTTTCTC  1440
   CAAGAATGGC AAAAGGAGAT CAAGTGGACA TTCTCTATGA AGA                    1483
(2) INFORMATION FOR SEQ ID NO:2:
   (i) SEQUENCE CHARACTERISTICS:
      (A) LENGTH: 446 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:2:
   Met Gly Thr Asp Gln Gly Lys Thr Phe Thr Trp Glu Glu Leu Ala Ala
   1 5 10 15
   His Asn Thr Lys Asp Asp Leu Leu Leu Ala Ile Arg Gly Arg Val Tyr
   20 25 30
   Asp Val Thr Lys Phe Leu Ser Arg His Pro Gly Gly Val Asp Thr Leu
   35 40 45
   Leu Leu Gly Ala Gly Arg Asp Val Thr Pro Val Phe Glu Met Tyr His
   50 55 60
   Ala Phe Gly Ala Ala Asp Ala Ile Met Lys Lys Tyr Tyr Val Gly Thr
   65 70 75 80
   Leu Val Ser Asn Glu Leu Pro Ile Phe Pro Glu Pro Thr Val Phe His
   85 90 95
   Lys Thr Ile Lys Thr Arg Val Glu Gly Tyr Phe Thr Asp Arg Asn Ile
   100 105 110
   Asp Pro Lys Asn Arg Pro Glu Ile Trp Gly Arg Tyr Ala Leu Ile Phe
   115 120 125
   Gly Ser Leu Ile Ala Ser Tyr Tyr Ala Gln Leu Phe Val Pro Phe Val
   130 135 140
   Val Glu Arg Thr Trp Leu Gln Val Val Phe Ala Ile Ile Met Gly Phe
   145 150 155 160
   Ala Cys Ala Gln Val Gly Leu Asn Pro Leu His Asp Ala Ser His Phe
   165 170 175
   Ser Val Thr His Asn Pro Thr Val Trp Lys Ile Leu Gly Ala Thr His
   180 185 190
   Asp Phe Phe Asn Gly Ala Ser Tyr Leu Val Trp Met Tyr Gln His Met
   195 200 205
   Leu Gly His His Pro Tyr Thr Asn Ile Ala Gly Ala Asp Pro Asp Val
   210 215 220
   Ser Thr Ser Glu Pro Asp Val Arg Arg Ile Lys Pro Asn Gln Lys Trp
   225 230 235 240
   Phe Val Asn His Ile Asn Gln His Met Phe Val Pro Phe Leu Tyr Gly
   245 250 255
   Leu Leu Ala Phe Lys Val Arg Ile Gln Asp Ile Asn Ile Leu Tyr Phe
   260 265 270
   Val Lys Thr Asn Asp Ala Ile Arg Val Asn Pro Ile Ser Thr Trp His
   275 280 285
   Thr Val Met Phe Trp Gly Gly Lys Ala Phe Phe Val Trp Tyr Arg Leu
   290 295 300
   Ile Val Pro Leu Gln Tyr Leu Pro Leu Gly Lys Val Leu Leu Leu Phe
   305 310 315 320
   Thr Val Ala Asp Met Val Ser Ser Tyr Trp Leu Ala Leu Thr Phe Gln
   325 330 335
   Ala Asn His Val Val Glu Glu Val Gln Trp Pro Leu Pro Asp Glu Asn
   340 345 350
   Gly Ile Ile Gln Lys Asp Trp Ala Ala Met Gln Val Glu Thr Thr Gln
           355                 360                 365
   Asp Tyr Ala His Asp Ser His Leu Trp Thr Ser Ile Thr Gly Ser Leu
       370                 375                 380
   Asn Tyr Gln Ala Val His His Leu Phe Pro Asn Val Ser Gln His His
   385                 390                 395                 400
   Tyr Pro Asp Ile Leu Ala Ile Ile Lys Asn Thr Cys Ser Glu Tyr Lys
                   405                 410                 415
   Val Pro Tyr Leu Val Lys Asp Thr Phe Trp Gln Ala Phe Ala Ser His
               420                 425                 430
   Leu Glu His Leu Arg Val Leu Gly Leu Arg Pro Lys Glu Glu
           435                 440                 445
(2) INFORMATION FOR SEQ ID NO:3:
   (i) SEQUENCE CHARACTERISTICS:
      (A) LENGTH: 186 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:3:
   Leu His His Thr Tyr Thr Asn Ile Ala Gly Ala Asp Pro Asp Val Ser
   1               5                   10                  15
   Thr Ser Glu Pro Asp Val Arg Arg Ile Lys Pro Asn Gln Lys Trp Phe
               20                  25                  30
   Val Asn His Ile Asn Gln His Met Phe Val Pro Phe Leu Tyr Gly Leu
           35                  40                  45
   Leu Ala Phe Lys Val Arg Ile Gln Asp Ile Asn Ile Leu Tyr Phe Val
       50                  55                  60
   Lys Thr Asn Asp Ala Ile Arg Val Asn Pro Ile Ser Thr Trp His Thr
   65                  70                  75                  80
   Val Met Phe Trp Gly Gly Lys Ala Phe Phe Val Trp Tyr Arg Leu Ile
                   85                  90                  95
   Val Pro Leu Gln Tyr Leu Pro Leu Gly Lys Val Leu Leu Leu Phe Thr
               100                 105                 110
   Val Ala Asp Met Val Ser Ser Tyr Trp Leu Ala Leu Thr Phe Gln Ala
           115                 120                 125
   Asn Tyr Val Val Glu Glu Val Gln Trp Pro Leu Pro Asp Glu Asn Gly
       130                 135                 140
   Ile Ile Gln Lys Asp Trp Ala Ala Met Gln Val Glu Thr Thr Gln Asp
   145                 150                 155                 160
   Tyr Ala His Asp Ser His Leu Trp Thr Ser Ile Thr Gly Ser Leu Asn
                   165                 170                 175
   Tyr Gln Xaa Val His His Leu Phe Pro His
               180                 185
(2) INFORMATION FOR SEQ ID NO:4:
   (i) SEQUENCE CHARACTERISTICS:
      (A) LENGTH: 457 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:4:
   Met Ala Ala Ala Pro Ser Val Arg Thr Phe Thr Arg Ala Glu Val Leu
   1               5                   10                  15
   Asn Ala Glu Ala Leu Asn Glu Gly Lys Lys Asp Ala Glu Ala Pro Phe
               20                  25                  30
   Leu Met Ile Ile Asp Asn Lys Val Tyr Asp Val Arg Glu Phe Val Pro
           35                  40                  45
   Asp His Pro Gly Gly Ser Val Ile Leu Thr His Val Gly Lys Asp Gly
       50                  55                  60
   Thr Asp Val Phe Asp Thr Phe His Pro Glu Ala Ala Trp Glu Thr Leu
   65                  70                  75                  80
   Ala Asn Phe Tyr Val Gly Asp Ile Asp Glu Ser Asp Arg Asp Ile Lys
                   85                  90                  95
   Asn Asp Asp Phe Ala Ala Glu Val Arg Lys Leu Arg Thr Leu Phe Gln
               100                 105                 110
   Ser Leu Gly Tyr Tyr Asp Ser Ser Lys Ala Tyr Tyr Ala Phe Lys Val
           115                 120                 125
   Ser Phe Asn Leu Cys Ile Trp Gly Leu Ser Thr Val Ile Val Ala Lys
       130                 135                 140
   Trp Gly Gln Thr Ser Thr Leu Ala Asn Val Leu Ser Ala Ala Leu Leu
   145                 150                 155                 160
   Gly Leu Phe Trp Gln Gln Cys Gly Trp Leu Ala His Asp Phe Leu His
                   165                 170                 175
   His Gln Val Phe Gln Asp Arg Phe Trp Gly Asp Leu Phe Gly Ala Phe
               180                 185                 190
   Leu Gly Gly Val Cys Gln Gly Phe Ser Ser Ser Trp Trp Lys Asp Lys
           195                 200                 205
   His Asn Thr His His Ala Ala Pro Asn Val His Val Glu Asp Pro Asp
       210                 215                 220
   Ile Asp Thr His Pro Leu Leu Thr Trp Ser Glu His Ala Leu Glu Met
   225                 230                 235                 240
   Phe Ser Asp Val Pro Asp Glu Glu Leu Thr Arg Met Trp Ser Arg Phe
   245 250 255
   Met Val Leu Asn Gln Thr Trp Phe Tyr Phe Pro Ile Leu Ser Phe Ala
   260 265 270
   Arg Leu Ser Trp Cys Leu Gln Ser Ile Leu Phe Val Leu Pro Asn Gly
   275 280 285
   Gln Ala His Lys Pro Ser Gly Ala Arg Val Pro Ile Ser Leu Val Glu
   290 295 300
   Gln Leu Ser Leu Ala Met His Trp Thr Trp Tyr Leu Ala Thr Met Phe
   305 310 315 320
   Leu Phe Ile Lys Asp Pro Val Asn Met Leu Val Tyr Phe Leu Val Ser
   325 330 335
   Gln Ala Val Cys Gly Asn Leu Leu Ala Ile Val Phe Ser Leu Asn His
   340 345 350
   Asn Gly Met Pro Val Ile Ser Lys Glu Glu Ala Val Asp Met Asp Phe
   355 360 365
   Phe Thr Lys Gln Ile Ile Thr Gly Arg Asp Val His Pro Gly Leu Phe
   370 375 380
   Ala Asn Trp Phe Thr Gly Gly Leu Asn Tyr Gln Ile Glu His His Leu
   385 390 395 400
   Phe Pro Ser Met Pro Arg His Asn Phe Ser Lys Ile Gln Pro Ala Val
   405 410 415
   Glu Thr Leu Cys Lys Lys Tyr Asn Val Arg Tyr His Thr Thr Gly Met
   420 425 430
   Ile Glu Gly Thr Ala Glu Val Phe Ser Arg Leu Asn Glu Val Ser Lys
   435 440 445
   Ala Ala Ser Lys Met Gly Lys Ala Gln
   450 455
(2) INFORMATION FOR SEQ ID NO:S:
   (i) SEQUENCE CHARACTERISTICS:
      (A) LENGTH: 446 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 359 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:6:
   Met Leu Thr Ala Glu Arg Ile Lys Phe Thr Gln Lys Arg Gly Phe Arg
   1               5                   10                  15
   Arg Val Leu Asn Gln Arg Val Asp Ala Tyr Phe Ala Glu His Gly Leu
               20                  25                  30
   Thr Gln Arg Asp Asn Pro Ser Met Tyr Leu Lys Thr Leu Ile Ile Val
           35                  40                  45
   Leu Trp Leu Phe Ser Ala Trp Ala Phe Val Leu Phe Ala Pro Val Ile
       50                  55                  60
   Phe Pro Val Arg Leu Leu Gly Cys Met Val Leu Ala Ile Ala Leu Ala
   65                  70                  75                  80
   Ala Phe Ser Phe Asn Val Gly His Asp Ala Asn His Asn Ala Tyr Ser
                   85                  90                  95
   Ser Asn Pro His Ile Asn Arg Val Leu Gly Met Thr Tyr Asp Phe Val
               100                 105                 110
   Gly Leu Ser Ser Phe Leu Trp Arg Tyr Arg His Asn Tyr Leu His His
           115                 120                 125
   Thr Tyr Thr Asn Ile Leu Gly His Asp Val Glu Ile His Gly Asp Gly
       130                 135                 140
   Ala Val Arg Met Ser Pro Glu Gln Glu His Val Gly Ile Tyr Arg Phe
   145                 150                 155                 160
   Gln Gln Phe Tyr Ile Trp Gly Leu Tyr Leu Phe Ile Pro Phe Tyr Trp
                   165                 170                 175
   Phe Leu Tyr Asp Val Tyr Leu Val Leu Asn Lys Gly Lys Tyr His Asp
               180                 185                 190
   His Lys Ile Pro Pro Phe Gln Pro Leu Glu Leu Ala Ser Leu Leu Gly
   195 200 205
   Ile Lys Leu Leu Trp Leu Gly Tyr Val Phe Gly Leu Pro Leu Ala Leu
   210 215 220
   Gly Phe Ser Ile Pro Glu Val Leu Ile Gly Ala Ser Val Thr Tyr Met
   225 230 235 240
   Thr Tyr Gly Ile Val Val Cys Thr Ile Phe Met Leu Ala His Val Leu
   245 250 255
   Glu Ser Thr Glu Phe Leu Thr Pro Asp Gly Glu Ser Gly Ala Ile Asp
   260 265 270
   Asp Glu Trp Ala Ile Cys Gln Ile Arg Thr Thr Ala Asn Phe Ala Thr
   275 280 285
   Asn Asn Pro Phe Trp Asn Trp Phe Cys Gly Gly Leu Asn His Gln Val
   290 295 300
   Thr His His Leu Phe Pro Asn Ile Cys His Ile His Tyr Pro Gln Leu
   305 310 315 320
   Glu Asn Ile Ile Lys Asp Val Cys Gln Glu Phe Gly Val Glu Tyr Lys
   325 330 335
   Val Tyr Pro Thr Phe Lys Ala Ala Ile Ala Ser Asn Tyr Arg Trp Leu
   340 345 350
   Glu Ala Met Gly Lys Ala Ser
   355
(2) INFORMATION FOR SEQ ID NO:7:
   (i) SEQUENCE CHARACTERISTICS:
      (A) LENGTH: 365 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:7:
   Met Thr Ser Thr Thr Ser Lys Val Thr Phe Gly Lys Ser Ile Gly Phe
   1               5                   10                  15
   Arg Lys Glu Leu Asn Arg Arg Val Asn Ala Tyr Leu Glu Ala Glu Asn
               20                  25                  30
   Ile Ser Pro Arg Asp Asn Pro Pro Met Tyr Leu Lys Thr Ala Ile Ile
           35                  40                  45
   Leu Ala Trp Val Val Ser Ala Trp Thr Phe Val Val Phe Gly Pro Asp
       50                  55                  60
   Val Leu Trp Met Lys Leu Leu Gly Cys Ile Val Leu Gly Phe Gly Val
   65                  70                  75                  80
   Ser Ala Val Gly Phe Asn Ile Ser His Asp Gly Asn His Gly Gly Tyr
                   85                  90                  95
   Ser Lys Tyr Gln Trp Val Asn Tyr Leu Ser Gly Leu Thr His Asp Ala
               100                 105                 110
   Ile Gly Val Ser Ser Tyr Leu Trp Lys Phe Arg His Asn Val Leu His
           115                 120                 125
   His Thr Tyr Thr Asn Ile Leu Gly His Asp Val Glu Ile His Gly Asp
       130                 135                 140
   Glu Leu Val Arg Met Ser Pro Ser Met Glu Tyr Arg Trp Tyr His Arg
   145                 150                 155                 160
   Tyr Gln His Trp Phe Ile Trp Phe Val Tyr Pro Phe Ile Pro Tyr Tyr
                   165                 170                 175
   Trp Ser Ile Ala Asp Val Gln Thr Met Leu Phe Lys Arg Gln Tyr His
               180                 185                 190
   Asp His Glu Ile Pro Ser Pro Thr Trp Val Asp Ile Ala Thr Leu Leu
           195                 200                 205
   Ala Phe Lys Ala Phe Gly Val Ala Val Phe Leu Ile Ile Pro Ile Ala
       210                 215                 220
   Val Gly Tyr Ser Pro Leu Glu Ala Val Ile Gly Ala Ser Ile Val Tyr
   225                 230                 235                 240
   Met Thr His Gly Leu Val Ala Cys Val Val Phe Met Leu Ala His Val
                   245                 250                 255
   Ile Glu Pro Ala Glu Phe Leu Asp Pro Asp Asn Leu His Ile Asp Asp
               260                 265                 270
   Glu Trp Ala Ile Ala Gln Val Lys Thr Thr Val Asp Phe Ala Pro Asn
           275                 280                 285
   Asn Thr Ile Ile Asn Trp Tyr Val Gly Gly Leu Asn Tyr Gln Thr Val
       290                 295                 300
   His His Leu Phe Pro His Ile Cys His Ile His Tyr Pro Lys Ile Ala
   305                 310                 315                 320
   Pro Ile Leu Ala Glu Val Cys Glu Glu Phe Gly Val Asn Tyr Ala Val
                   325                 330                 335
   His Gln Thr Phe Phe Gly Ala Leu Ala Ala Asn Tyr Ser Trp Leu Lys
               340                 345                 350
   Lys Met Ser Ile Asn Pro Glu Thr Lys Ala Ile Glu Gln
           355                 360                 365
(2) INFORMATION FOR SEQ ID NO:8:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic oligonucleotide"
   (ix) FEATURE:
      (A) NAME/KEY: misc_feature
      (B) LOCATION: 21
      (D) OTHER INFORMATION: /number= 1 /note= "N=Inosine or Cytosine"
   (ix) FEATURE:
      (A) NAME/KEY: misc_feature
      (B) LOCATION: 27
      (D) OTHER INFORMATION: /number= 2 /note= "N=Inosine or Cytosine"
   (xi) SEQUENCE DESCRIPTION: SEQ ID NO:8:
   CUACUACUAC UACAYCAYAC NTAYACNAAY AT 32
(2) INFORMATION FOR SEQ ID NO:9:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic oligonucleotide"
   (ix) FEATURE:
      (A) NAME/KEY: misc_feature
      (B) LOCATION: 13
      (D) OTHER INFORMATION: /number= 1 /note= "N=Inosine or Cytosine"
   (ix) FEATURE:
      (A) NAME/KEY: misc_feature
      (B) LOCATION: 19
      (D) OTHER INFORMATION: /number= 2 /note= "N=Inosine or Cytosine"
   (xi) SEQUENCE DESCRIPTION: SEQ ID NO:9:
   CAUCAUCAUC AUNGGRAANA RRTGRTG 27
(2) INFORMATION FOR SEQ ID NO:10:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:10:
   CCAAGCTTCT GCAGGAGCTC TTTTTTTTTT TTTTT          35
(2) INFORMATION FOR SEQ ID NO:11:
   (i) SEQUENCE CHARACTERISTICS:
      (A) LENGTH: 5 amino acids
      (B) TYPE: amino-acid
      (C) STRANDEDNESS: not relevant
      (D) TOPOLOGY: linear
   (ii) MOLECULE TYPE: peptide
   (xi) SEQUENCE DESCRIPTION: SEQ ID NO:11:
   His Xaa Xaa His His
   1               5
(2) INFORMATION FOR SEQ ID NO:12:
   (i) SEQUENCE CHARACTERISTICS:
      (A) LENGTH: 5 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:12:
   Gln Xaa Xaa His His
   1               5
(2) INFORMATION FOR SEQ ID NO:13:
   (i) SEQUENCE CHARACTERISTICS:
      (A) LENGTH: 746 nucleic acids
      (B) TYPE: nucleic acid
      (C) STRANDEDNESS: not relevant
      (D) TOPOLOGY: linear
   (ii) MOLECULE TYPE: nucleic acid
   (xi) SEQUENCE DESCRIPTION: SEQ ID NO:13:
   CGTATGTCAC TCCATTCCAA ACTCGTTCAT GGTATCATAA ATATCAACAC ATTTACGCTC     60
   CACTCCTCTA TGGTATTTAC ACACTCAAAT ATCGTACTCA AGATTGGGAA GCTTTTGTAA    120
   AGGATGGTAA AAATGGTGCA ATTCGTGTTA GTGTCGCCAC AAATTTCGAT AAGGCCGCTT    180
   ACGTCATTGG TAAATTGTCT TTTGTTTTCT TCCGTTTCAT CCTTCCACTC CGTTATCATA    240
   GCTTTACAGA TTTAATTTGT TATTTCCTCA TTGCTGAATT CGTCTTTGGT TGGTATCTCA    300
   CAATTAATTT CCAAGTTAGT CATGTCGCTG AAGATCTCAA ATTCTTTGCT ACCCCTGAAA    360
   GACCAGATGA ACCATCTCAA ATCAATGAAG ATTGGGCAAT CCTTCAACTT AAAACTACTC    420
   AAGATTATGG TCATGGTTCA CTCCTTTGTA CCTTTTTTAG TGGTTCTTTA AATCATCAAG    480
   TTGTTCATCA TTTATTCCCA TCAATTGCTC AAGATTTCTA CCCACAACTT GTACCAATTG    540
   TAAAAGAAGT TTGTAAAGAA CATAACATTA CTTACCACAT TAAACCAAAC TTCACTGAAG    600
   CTATTATGTC ACACATTAAT TACCTTTACA AAATGGGTAA TGATCCAGAT TATGTTAAAA    660
   AACCATTAGC CTCAAAAGAT GATTAAATGA AATAACTTAA AAACCAATTA TTTACTTTTG    720
   ACAAACAGTA ATATTAATAA ATACAA                                         746
(2) INFORMATION FOR SEQ ID NO:14:
   (i) SEQUENCE CHARACTERISTICS:
      (A) LENGTH: 227 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:14:
(2) INFORMATION FOR SEQ ID NO:15:
   (i) SEQUENCE CHARACTERISTICS:
      (A) LENGTH: 494 nucleic acids
      (B) TYPE: nucleic acid
      (C) STRANDEDNESS: not relevant
      (D) TOPOLOGY: linear
   (ii) MOLECULE TYPE: nucleic acid
   (xi) SEQUENCE DESCRIPTION: SEQ ID NO:15:
   TTTTGGAAGG NTCCAAGTTN ACCACGGANT NGGCAAGTTN ACGGGGCGGA AANCGGTTTT    60
   CCCCCCAAGC CTTTTGTCGA CTGGTTCTGT GGTGGCTTCC AGTACCAAGT CGACCACCAC   120
   TTATTCCCCA GCCTGCCCCG ACACAATCTG GCCAAGACAC ACGCACTGGT CGAATCGTTC   180
   TGCAAGGAGT GGGGTGTCCA GTACCACGAA GCCGACCTCG TGGACGGGAC CATGGAAGTC   240
   TTGCACCATT TGGGCAGCGT GGCCGGCGAA TTCGTCGTGG ATTTTGTACG CGACGGACCC   300
   GCCATGTAAT CGTCGTTCGT GACGATGCAA GGGTTCACGC ACATCTACAC ACACTCACTC   360
   ACACAACTAG TGTAACTCGT ATAGAATTCG GTGTCGACCT GGACCTTGTT TGACTGGTTG   420
   GGGATAGGGT AGGTAGGCGG ACGCGTGGGT CGNCCCCGGG AATTCTGTGA CCGGTACCTG   480
   GCCCGCGTNA AAGT                                                     494
(2) INFORMATION FOR SEQ ID NO:16:
   (i) SEQUENCE CHARACTERISTICS:
      (A) LENGTH: 87 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:16:
(2) INFORMATION FOR SEQ ID NO:17:
   (i) SEQUENCE CHARACTERISTICS:
      (A) LENGTH: 520 nucleic acids
      (B) TYPE: amino acid
      (C) STRANDEDNESS: not relevant
      (D) TOPOLOGY: linear
   (ii) MOLECULE TYPE: nucleic acid
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 153 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:18:
   Met Glu Phe Val Trp Ile Ala Val Arg Tyr Ala Thr Trp Phe Lys
   1               5                   10                  15
   Arg His Gly Cys Ala Trp Val His Ala Gly Ala Val Val Gly His
                   20                  25                  30
   Val Leu Val Arg Leu Trp Ser Arg Leu His Leu His Phe Ser Ala
                   35                  40                  45
   Val Arg Arg Lys Ser His Pro Phe Ala Arg Glu Gln Pro Gly Gly
                   50                  55                  60
   Ser Ala Ala Leu Ala Arg Val Arg Ala Asp His Thr Val Asn Ile
                   65                  70                  75
   Ser Thr Lys Ser Trp Phe Val Thr Trp Trp Met Ser Asn Leu Asn
                   80                  85                  90
   Phe Gln Ile Glu His His Leu Phe Pro Thr Ala Pro Gln Phe Arg
                   95                  100                 105
   Phe Lys Glu Ile Ser Pro Arg Val Glu Ala Leu Phe Lys Arg His
                   110                 115                 120
   Gly Leu Pro Tyr Tyr Asp Met Pro Tyr Thr Ser Ala Val Ser Thr
                   125                 130                 135
   Thr Phe Ala Asn Leu Tyr Ser Val Gly His Ser Val Gly Asp Ala
                   140                 145                 150
   Lys Arg Asp
(2) INFORMATION FOR SEQ ID NO:19:
   (i) SEQUENCE CHARACTERISTICS:
      (A) LENGTH: 429 nucleic acids
      (B) TYPE: nucleic acid
      (C) STRANDEDNESS: not relevant
      (D) TOPOLOGY: linear
   (ii) MOLECULE TYPE: nucleic acid
   (xi) SEQUENCE DESCRIPTION: SEQ ID NO:19:
(2) INFORMATION FOR SEQ ID NO:20:
   (i) SEQUENCE CHARACTERISTICS:
      (A) LENGTH: 125 amino acids
      (B) TYPE: amino acid
      (C) STRANDEDNESS: not relevant
      (D) TOPOLOGY: linear
   (ii) MOLECULE TYPE: peptide
   (xi) SEQUENCE DESCRIPTION: SEQ ID NO:20:
(2) INFORMATION FOR SEQ ID NO:21:
   (i) SEQUENCE CHARACTERISTICS:
      (A) LENGTH: 1219 base pairs
      (B) TYPE: nucleic acid
      (C) STRANDEDNESS: single
      (D) TOPOLOGY: linear
   (ii) MOLECULE TYPE: other nucleic acid (Edited Contig 2692004)
   (xi) SEQUENCE DESCRIPTION: SEQ ID NO:21:
   GCACGCCGAC CGGCGCCGGG AGATCCTGGC AAAGTATCCA GAGATAAAGT CCTTGATGAA     60
   ACCTGATCCC AATTTGATAT GGATTATAAT TATGATGGTT CTCACCCAGT TGGGTGCATT    120
   TTACATAGTA AAAGACTTGG ACTGGAAATG GGTCATATTT GGGGCCTATG CGTTTGGCAG    180
   TTGCATTAAC CACTCAATGA CTCTGGCTAT TCATGAGATT GCCCACAATG CTGCCTTTGG    240
   CAACTGCAAA GCAATGTGGA ATCGCTGGTT TGGAATGTTT GCTAATCTTC CTATTGGGAT    300
   TCCATATTCA ATTTCCTTTA AGAGGTATCA CATGGATCAT CATCGGTACC TTGGAGCTGA    360
   TGGCGTCGAT GTAGATATTC CTACCGATTT TGAGGGCTGG TTCTTCTGTA CCGCTTTCAG    420
   AAAGTTTATA TGGGTTATTC TTCAGCCTCT CTTTTATGCC TTTCGACCTC TGTTCATCAA    480
   CCCCAAACCA ATTACGTATC TGGAAGTTAT CAATACCGTG GCACAGGTCA CTTTTGACAT    540
   TTTAATTTAT TACTTTTTGG GAATTAAATC CTTAGTCTAC ATGTTGGCAG CATCTTTACT    600
   TGGCCTGGGT TTGCACCCAA TTTCTGGACA TTTTATAGCT GAGCATTACA TGTTCTTAAA    660
   GGGTCATGAA ACTTACTCAT ATTATGGGCC TCTGAATTTA CTTACCTTCA ATGTGGGTTA    720
   TCATAATGAA CATCATGATT TCCCCAACAT TCCTGGAAAA AGTCTTCCAC TGGTGAGGAA    780
   AATAGCAGCT GAATACTATG ACAACCTCCC TCACTACAAT TCCTGGATAA AAGTACTGTA    840
   TGATTTTGTG ATGGATGATA CAATAAGTCC CTACTCAAGA ATGAAGAGGC ACCAAAAAGG    900
   AGAGATGGTG CTGGAGTAAA TATCATTAGT GCCAAAGGGA TTCTTCTCCA AAACTTTAGA    960
   TGATAAAATG GAATTTTTGC ATTATTAAAC TTGAGACCAG TGATGCTCAG AAGCTCCCCT   1020
   GGCACAATTT CAGAGTAAGA GCTCGGTGAT ACCAAGAAGT GAATCTGGCT TTTAAACAGT   1080
   CAGCCTGACT CTGTACTGCT CAGTTTCACT CACAGGAAAC TTGTGACTTG TGTATTATCG   1140
   TCATTGAGGA TGTTTCACTC ATGTCTGTCA TTTTATAAGC ATATCATTTA AAAAGCTTCT   1200
   AAAAAGCTAT TTCGCCAGG                                                1219
(2) INFORMATION FOR SEQ ID NO:22:
   (i) SEQUENCE CHARACTERISTICS:
      (A) LENGTH: 655 base pairs
      (B) TYPE: nucleic acid
      (C) STRANDEDNESS: single
      (D) TOPOLOGY: linear
   (ii) MOLECULE TYPE: other nucleic acid (Edited Contig 2153526)
   (xi) SEQUENCE DESCRIPTION: SEQ ID NO:22:
   TTACCTTCTA CGTCCGCTTC TTCCTCACTT ATGTGCCACT ATTGGGGCTG AAAGCTTCCT    60
   GGGCCTTTTC TTCATAGTCA GGTTCCTGGA AAGCAACTGG TTTGTGTGGG TGACACAGAT   120
   GAACCATATT CCCATGCACA TTGATCATGA CCGGAACATG GACTGGGTTT CCACCCAGCT   180
   CCAGGCCACA TGCAATGTCC ACAAGTCTGC CTTCAATGAC TGGTTCAGTG GACACCTCAA   240
   CTTCCAGATT GAGCACCATC TTTTTCCCAC GATGCCTCGA CACAATTACC ACAAAGTGGC   300
   TCCCCTGGTG CAGTCCTTGT GTGCCAAGCA TGGCATAGAG TACCAGTCCA AGCCCCTGCT   360
   GTCAGCCTTC GCCGACATCA TCCACTCACT AAAGGAGTCA GGGCAGCTCT GGCTAGATGC   420
   CTATCTTCAC CAATAACAAC AGCCACCCTG CCCAGTCTGG AAGAAGAGGA GGAAGACTCT   480
   GGAGCCAAGG CAGAGGGGAG CTTGAGGGAC AATGCCACTA TAGTTTAATA CTCAGAGGGG   540
   GTTGGGTTTG GGGACATAAA GCCTCTGACT CAAACTCCTC CCTTTTATCT TCTAGCCACA   600
   GTTCTAAGAC CCAAAGTGGG GGGTGGACAC AGAAGTCCCT AGGAGGGAAG GAGCT        655
(2) INFORMATION FOR SEQ ID NO:23:
   (i) SEQUENCE CHARACTERISTICS:
      (A) LENGTH: 304 base pairs
      (B) TYPE: nucleic acid
      (C) STRANDEDNESS: single
      (D) TOPOLOGY: linear
   (ii) MOLECULE TYPE: other nucleic acid (Edited Contig 3506132)
   (xi) SEQUENCE DESCRIPTION: SEQ ID NO:23:
   GTCTTTTACT TTGGCAATGG CTGGATTCCT ACCCTCATCA CGGCCTTTGT CCTTGCTACC    60
   TCTCAGGCCC AAGCTGGATG GCTGCAACAT GATTATGGCC ACCTGTCTGT CTACAGAAAA   120
   CCCAAGTGGA ACCACCTTGT CCACAAATTC GTCATTGGCC ACTTAAAGGG TGCCTCTGCC   180
   AACTGGTGGA ATCATCGCCA CTTCCAGCAC CACGCCAAGC CTAACATCTT CCACAAGGAT   240
   CCCGATGTGA ACATGCTGCA CGTGTTTGTT CTGGGCGAAT GGCAGCCCAT CGAGTACGGC   300
   AAGA                                                                304
(2) INFORMATION FOR SEQ ID NO:24:
   (i) SEQUENCE CHARACTERISTICS:
      (A) LENGTH: 918 base pairs
      (B) TYPE: nucleic acid
      (C) STRANDEDNESS: single
      (D) TOPOLOGY: linear
   (ii) MOLECULE TYPE: other nucleic acid (Edited Contig 3854933)
   (xi) SEQUENCE DESCRIPTION: SEQ ID NO:24:
   CAGGGACCTA CCCCGCGCTA CTTCACCTGG GACGAGGTGG CCCAGCGCTC AGGGTGCGAG    60
   GAGCGGTGGC TAGTGATCGA CCGTAAGGTG TACAACATCA GCGAGTTCAC CCGCCGGCAT   120
   CCAGGGGGCT CCCGGGTCAT CAGCCACTAC GCCGGGCAGG ATGCCACGGA TCCCTTTGTG   180
   GCCTTCCACA TCAACAAGGG CCTTGTGAAG AAGTATATGA ACTCTCTCCT GATTGGAGAA   240
   CTGTCTCCAG AGCAGCCCAG CTTTGAGCCC ACCAAGAATA AAGAGCTGAC AGATGAGTTC   300
   CGGGAGCTGC GGGCCACAGT GGAGCGGATG GGGCTCATGA AGGCCAACCA TGTCTTCTTC   360
   CTGCTGTACC TGCTGCACAT CTTGCTGCTG GATGGTGCAG CCTGGCTCAC CCTTTGGGTC   420
   TTTGGGACGT CCTTTTTGCC CTTCCTCCTC TGTGCGGTGC TGCTCAGTGC AGTTCAGGCC   480
   CAGGCTGGCT GGCTGCAGCA TGACTTTGGG CACCTGTCGG TCTTCAGCAC CTCAAAGTGG   540
   AACCATCTGC TACATCATTT TGTGATTGGC CACCTGAAGG GGGCCCCCGC CAGTTGGTGG   600
   AACCACATGC ACTTCCAGCA CCATGCCAAG CCCAACTGCT TCCGCAAAGA CCCAGACATC   660
   AACATGCATC CCTTCTTCTT TGCCTTGGGG AAGATCCTCT CTGTGGAGCT TGGGAAACAG   720
   AAGAAAAAAT ATATGCCGTA CAACCACCAG CACARATACT TCTTCCTAAT TGGGCCCCCA   780
   GCCTTGCTGC CTCTCTACTT CCAGTGGTAT ATTTTCTATT TTGTTATCCA GCGAAAGAAG   840
   TGGGTGGACT TGGCCTGGAT CAGCAAACAG GAATACGATG AAGCCGGGCT TCCATTGTCC   900
   ACCGCAAATG CTTCTAAA                                                 918
(2) INFORMATION FOR SEQ ID NO:25:
   (i) SEQUENCE CHARACTERISTICS:
      (A) LENGTH: 1686 base pairs
      (B) TYPE: nucleic acid
      (C) STRANDEDNESS: single
      (D) TOPOLOGY: linear
   (ii) MOLECULE TYPE: other nucleic acid (Edited Contig 2511785)
   (xi) SEQUENCE DESCRIPTION: SEQ ID NO:25:
   GCCACTTAAA GGGTGCCTCT GCCAACTGGT GGAATCATCG CCACTTCCAG CACCACGCCA 60
   AGCCTAACAT CTTCCACAAG GATCCCGATG TGAACATGCT GCACGTGTTT GTTCTGGGCG 120
   AATGGCAGCC CATCGAGTAC GGCAAGAAGA AGCTGAAATA CCTGCCCTAC AATCACCAGC 180
   ACGAATACTT CTTCCTGATT GGGCCGCCGC TGCTCATCCC CATGTATTTC CAGTACCAGA 240
   TCATCATGAC CATGATCGTC CATAAGAACT GGGTGGACCT GGCCTGGGCC GTCAGCTACT 300
   ACATCCGGTT CTTCATCACC TACATCCCTT TCTACGGCAT CCTGGGAGCC CTCCTTTTCC 360
   TCAACTTCAT CAGGTTCCTG GAGAGCCACT GGTTTGTGTG GGTCACACAG ATGAATCACA 420
   TCGTCATGGA GATTGACCAG GAGGCCTACC GTGACTGGTT CAGTAGCCAG CTGACAGCCA 480
   CCTGCAACGT GGAGCAGTCC TTCTTCAACG ACTGGTTCAG TGGACACCTT AACTTCCAGA 540
   TTGAGCACCA CCTCTTCCCC ACCATGCCCC GGCACAACTT ACACAAGATC GCCCCGCTGG 600
   TGAAGTCTCT ATGTGCCAAG CATGGCATTG AATACCAGGA GAAGCCGCTA CTGAGGGCCC 660
   TGCTGGACAT CATCAGGTCC CTGAAGAAGT CTGGGAAGCT GTGGCTGGAC GCCTACCTTC 720
   ACAAATGAAG CCACAGCCCC CGGGACACCG TGGGGAAGGG GTGCAGGTGG GGTGATGGCC 780
   AGAGGAATGA TGGGCTTTTG TTCTGAGGGG TGTCCGAGAG GCTGGTGTAT GCACTGCTCA 840
   CGGACCCCAT GTTGGATCTT TCTCCCTTTC TCCTCTCCTT TTTCTCTTCA CATCTCCCCC 900
   ATAGCACCCT GCCCTCATGG GACCTGCCCT CCCTCAGCCG TCAGCCATCA GCCATGGCCC 960
   TCCCAGTGCC TCCTAGCCCC TTCTTCCAAG GAGCAGAGAG GTGGCCACCG GGGGTGGCTC 1020
   TGTCCTACCT CCACTCTCTG CCCCTAAAGA TGGGAGGAGA CCAGCGGTCC ATGGGTCTGG 1080
   CCTGTGAGTC TCCCCTTGCA GCCTGGTCAC TAGGCATCAC CCCCGCTTTG GTTCTTCAGA 1140
   TGCTCTTGGG GTTCATAGGG GCAGGTCCTA GTCGGGCAGG GCCCCTGACC CTCCCGGCCT 1200
   GGCTTCACTC TCCCTGACGG CTGCCATTGG TCCACCCTTT CATAGAGAGG CCTGCTTTGT 1260
   TACAAAGCTC GGGTCTCCCT CCTGCAGCTC GGTTAAGTAC CCGAGGCCTC TCTTAAGATG 1320
   TCCAGGGCCC CAGGCCCGCG GGCACAGCCA GCCCAAACCT TGGGCCCTGG AAGAGTCCTC 1380
   CACCCCATCA CTAGAGTGCT CTGACCCTGG GCTTTCACGG GCCCCATTCC ACCGCCTCCC 1440
   CAACTTGAGC CTGTGACCTT GGGACCAAAG GGGGAGTCCC TCGTCTCTTG TGACTCAGCA 1500
   GAGGCAGTGG CCACGTTCAG GGAGGGGCCG GCTGGCCTGG AGGCTCAGCC CACCCTCCAG 1560
   CTTTTCCTCA GGGTGTCCTG AGGTCCAAGA TTCTGGAGCA ATCTGACCCT TCTCCAAAGG 1620
   CTCTGTTATC AGCTGGGCAG TGCCAGCCAA TCCCTGGCCA TTTGGCCCCA GGGGACGTGG 1680
   GCCCTG 1686
(2) INFORMATION FOR SEQ ID NO:26:
   (i) SEQUENCE CHARACTERISTICS:
      (A) LENGTH: 1843 base pairs
      (B) TYPE: nucleic acid
      (C) STRANDEDNESS: single
      (D) TOPOLOGY: linear
   (ii) MOLECULE TYPE: other nucleic acid (Contig 2535)
   (xi) SEQUENCE DESCRIPTION: SEQ ID NO:26:
   GTCTTTTACT TTGGCAATGG CTGGATTCCT ACCCTCATCA CGGCCTTTGT CCTTGCTACC 60
   TCTCAGGCCC AAGCTGGATG GCTGCAACAT GATTATGGCC ACCTGTCTGT CTACAGAAAA 120
   CCCAAGTGGA ACCACCTTGT CCACAAATTC GTCATTGGCC ACTTAAAGGG TGCCTCTGCC 180
   AACTGGTGGA ATCATCGCCA CTTCCAGCAC CACGCCAAGC CTAACATCTT CCACAAGGAT 240
   CCCGATGTGA ACATGCTGCA CGTGTTTGTT CTGGGCGAAT GGCAGCCCAT CGAGTACGGC 300
   AAGAAGAAGC TGAAATACCT GCCCTACAAT CACCAGCACG AATACTTCTT CCTGATTGGG 360
   CCGCCGCTGC TCATCCCCAT GTATTTCCAG TACCAGATCA TCATGACCAT GATCGTCCAT 420
   AAGAACTGGG TGGACCTGGC CTGGGCCGTC AGCTACTACA TCCGGTTCTT CATCACCTAC 480
   ATCCCTTTCT ACGGCATCCT GGGAGCCCTC CTTTTCCTCA ACTTCATCAG GTTCCTGGAG 540
   AGCCACTGGT TTGTGTGGGT CACACAGATG AATCACATCG TCATGGAGAT TGACCAGGAG 600
   GCCTACCGTG ACTGGTTCAG TAGCCAGCTG ACAGCCACCT GCAACGTGGA GCAGTCCTTC 660
   TTCAACGACT GGTTCAGTGG ACACCTTAAC TTCCAGATTG AGCACCACCT CTTCCCCACC 720
   ATGCCCCGGC ACAACTTACA CAAGATCGCC CCGCTGGTGA AGTCTCTATG TGCCAAGCAT 780
   GGCATTGAAT ACCAGGAGAA GCCGCTACTG AGGGCCCTGC TGGACATCAT CAGGTCCCTG 840
   AAGAAGTCTG GGAAGCTGTG GCTGGACGCC TACCTTCACA AATGAAGCCA CAGCCCCCGG 900
   GACACCGTGG GGAAGGGGTG CAGGTGGGGT GATGGCCAGA GGAATGATGG GCTTTTGTTC 960
   TGAGGGGTGT CCGAGAGGCT GGTGTATGCA CTGCTCACGG ACCCCATGTT GGATCTTTCT 1020
   CCCTTTCTCC TCTCCTTTTT CTCTTCACAT CTCCCCCATA GCACCCTGCC CTCATGGGAC 1080
   CTGCCCTCCC TCAGCCGTCA GCCATCAGCC ATGGCCCTCC CAGTGCCTCC TAGCCCCTTC 1140
   TTCCAAGGAG CAGAGAGGTG GCCACCGGGG GTGGCTCTGT CCTACCTCCA CTCTCTGCCC 1200
   CTAAAGATGG GAGGAGACCA GCGGTCCATG GGTCTGGCCT GTGAGTCTCC CCTTGCAGCC 1260
   TGGTCACTAG GCATCACCCC CGCTTTGGTT CTTCAGATGC TCTTGGGGTT CATAGGGGCA 1320
   GGTCCTAGTC GGGCAGGGCC CCTGACCCTC CCGGCCTGGC TTCACTCTCC CTGACGGCTG 1380
   CCATTGGTCC ACCCTTTCAT AGAGAGGCCT GCTTTGTTAC AAAGCTCGGG TCTCCCTCCT 1440
   GCAGCTCGGT TAAGTACCCG AGGCCTCTCT TAAGATGTCC AGGGCCCCAG GCCCGCGGGC 1500
   ACAGCCAGCC CAAACCTTGG GCCCTGGAAG AGTCCTCCAC CCCATCACTA GAGTGCTCTG 1560
   ACCCTGGGCT TTCACGGGCC CCATTCCACC GCCTCCCCAA CTTGAGCCTG TGACCTTGGG 1620
   ACCAAAGGGG GAGTCCCTCG TCTCTTGTGA CTCAGCAGAG GCAGTGGCCA CGTTCAGGGA 1680
   GGGGCCGGCT GGCCTGGAGG CTCAGCCCAC CCTCCAGCTT TTCCTCAGGG TGTCCTGAGG 1740
   TCCAAGATTC TGGAGCAATC TGACCCTTCT CCAAAGGCTC TGTTATCAGC TGGGCAGTGC 1800
   CAGCCAATCC CTGGCCATTT GGCCCCAGGG GACGTGGGCC CTG 1843
(2) INFORMATION FOR SEQ ID NO:27:
   (i) SEQUENCE CHARACTERISTICS:
      (A) LENGTH: 2257 base pairs
      (B) TYPE: nucleic acid
      (C) STRANDEDNESS: single
      (D) TOPOLOGY: linear
   (ii) MOLECULE TYPE: other nucleic acid (Edited Contig 253538a)
   (xi) SEQUENCE DESCRIPTION: SEQ ID NO:27:
   CAGGGACCTA CCCCGCGCTA CTTCACCTGG GACGAGGTGG CCCAGCGCTC AGGGTGCGAG 60
   GAGCGGTGGC TAGTGATCGA CCGTAAGGTG TACAACATCA GCGAGTTCAC CCGCCGGCAT 120
   CCAGGGGGCT CCCGGGTCAT CAGCCACTAC GCCGGGCAGG ATGCCACGGA TCCCTTTGTG 180
   GCCTTCCACA TCAACAAGGG CCTTGTGAAG AAGTATATGA ACTCTCTCCT GATTGGAGAA 240
   CTGTCTCCAG AGCAGCCCAG CTTTGAGCCC ACCAAGAATA AAGAGCTGAC AGATGAGTTC 300
   CGGGAGCTGC GGGCCACAGT GGAGCGGATG GGGCTCATGA AGGCCAACCA TGTCTTCTTC 360
   CTGCTGTACC TGCTGCACAT CTTGCTGCTG GATGGTGCAG CCTGGCTCAC CCTTTGGGTC 420
   TTTGGGACGT CCTTTTTGCC CTTCCTCCTC TGTGCGGTGC TGCTCAGTGC AGTTCAGCAG 480
   GCCCAAGCTG GATGGCTGCA ACATGATTAT GGCCACCTGT CTGTCTACAG AAAACCCAAG 540
   TGGAACCACC TTGTCCACAA ATTCGTCATT GGCCACTTAA AGGGTGCCTC TGCCAACTGG 600
   TGGAATCATC GCCACTTCCA GCACCACGCC AAGCCTAACA TCTTCCACAA GGATCCCGAT 660
   GTGAACATGC TGCACGTGTT TGTTCTGGGC GAATGGCAGC CCATCGAGTA CGGCAAGAAG 720
   AAGCTGAAAT ACCTGCCCTA CAATCACCAG CACGAATACT TCTTCCTGAT TGGGCCGCCG 780
   CTGCTCATCC CCATGTATTT CCAGTACCAG ATCATCATGA CCATGATCGT CCATAAGAAC 840
   TGGGTGGACC TGGCCTGGGC CGTCAGCTAC TACATCCGGT TCTTCATCAC CTACATCCCT 900
   TTCTACGGCA TCCTGGGAGC CCTCCTTTTC CTCAACTTCA TCAGGTTCCT GGAGAGCCAC 960
   TGGTTTGTGT GGGTCACACA GATGAATCAC ATCGTCATGG AGATTGACCA GGAGGCCTAC 1020
   CGTGACTGGT TCAGTAGCCA GCTGACAGCC ACCTGCAACG TGGAGCAGTC CTTCTTCAAC 1080
   GACTGGTTCA GTGGACACCT TAACTTCCAG ATTGAGCACC ACCTCTTCCC CACCATGCCC 1140
   CGGCACAACT TACACAAGAT CGCCCCGCTG GTGAAGTCTC TATGTGCCAA GCATGGCATT 1200
   GAATACCAGG AGAAGCCGCT ACTGAGGGCC CTGCTGGACA TCATCAGGTC CCTGAAGAAG 1260
   TCTGGGAAGC TGTGGCTGGA CGCCTACCTT CACAAATGAA GCCACAGCCC CCGGGACACC 1320
   GTGGGGAAGG GGTGCAGGTG GGGTGATGGC CAGAGGAATG ATGGGCTTTT GTTCTGAGGG 1380
   GTGTCCGAGA GGCTGGTGTA TGCACTGCTC ACGGACCCCA TGTTGGATCT TTCTCCCTTT 1440
   CTCCTCTCCT TTTTCTCTTC ACATCTCCCC CATAGCACCC TGCCCTCATG GGACCTGCCC 1500
   TCCCTCAGCC GTCAGCCATC AGCCATGGCC CTCCCAGTGC CTCCTAGCCC CTTCTTCCAA 1560
   GGAGCAGAGA GGTGGCCACC GGGGGTGGCT CTGTCCTACC TCCACTCTCT GCCCCTAAAG  1620
   ATGGGAGGAG ACCAGCGGTC CATGGGTCTG GCCTGTGAGT CTCCCCTTGC AGCCTGGTCA  1680
   CTAGGCATCA CCCCCGCTTT GGTTCTTCAG ATGCTCTTGG GGTTCATAGG GGCAGGTCCT  1740
   AGTCGGGCAG GGCCCCTGAC CCTCCCGGCC TGGCTTCACT CTCCCTGACG GCTGCCATTG  1800
   GTCCACCCTT TCATAGAGAG GCCTGCTTTG TTACAAAGCT CGGGTCTCCC TCCTGCAGCT  1860
   CGGTTAAGTA CCCGAGGCCT CTCTTAAGAT GTCCAGGGCC CCAGGCCCGC GGGCACAGCC  1920
   AGCCCAAACC TTGGGCCCTG GAAGAGTCCT CCACCCCATC ACTAGAGTGC TCTGACCCTG  1980
   GGCTTTCACG GGCCCCATTC CACCGCCTCC CCAACTTGAG CCTGTGACCT TGGGACCAAA  2040
   GGGGGAGTCC CTCGTCTCTT GTGACTCAGC AGAGGCAGTG GCCACGTTCA GGGAGGGGCC  2100
   GGCTGGCCTG GAGGCTCAGC CCACCCTCCA GCTTTTCCTC AGGGTGTCCT GAGGTCCAAG  2160
   ATTCTGGAGC AATCTGACCC TTCTCCAAAG GCTCTGTTAT CAGCTGGGCA GTGCCAGCCA  2220
   ATCCCTGGCC ATTTGGCCCC AGGGGACGTG GGCCCTG                           2257
(2) INFORMATION FOR SEQ ID NO:28:
   (i) SEQUENCE CHARACTERISTICS:
      (A) LENGTH: 411 amino acids
      (B) TYPE: amino acid
      (C) STRANDEDNESS: single
      (D) TOPOLOGY: linear
   (ii) MOLECULE TYPE: amino acid (Translation of Contig 2692004)
   (xi) SEQUENCE DESCRIPTION: SEQ ID NO:28:
(2) INFORMATION FOR SEQ ID NO:29:
   (i) SEQUENCE CHARACTERISTICS:
      (A) LENGTH: 218 amino acids
      (B) TYPE: amino acid
      (C) STRANDEDNESS: single
      (D) TOPOLOGY: linear
   (ii) MOLECULE TYPE: amino acid (Translation of Contig 2153526)
   (xi) SEQUENCE DESCRIPTION: SEQ ID NO:29:
   Tyr Leu Leu Arg Pro Leu Leu Pro His Leu Cys Ala Thr Ile Gly
   1               5                   10                  15
   Ala Glu Ser Phe Leu Gly Leu Phe Phe Ile Val Arg Phe Leu Glu
                   20                  25                  30
   Ser Asn Trp Phe Val Trp Val Thr Gln Met Asn His Ile Pro Met
                   35                  40                  45
   His Ile Asp His Asp Arg Asn Met Asp Trp Val Ser Thr Gln Leu
                   50                  55                  60
   Gln Ala Thr Cys Asn Val His Lys Ser Ala Phe Asn Asp Trp Phe
                   65                  70                  75
   Ser Gly His Leu Asn Phe Gln Ile Glu His His Leu Phe Pro Thr
                   80                  85                  90
   Met Pro Arg His Asn Tyr His Lys Val Ala Pro Leu Val Gln Ser
                   95                  100                 105
   Leu Cys Ala Lys His Gly Ile Glu Tyr Gln Ser Lys Pro Leu Leu
                   110                 115                 120
   Ser Ala Phe Ala Asp Ile Ile His Ser Leu Lys Glu Ser Gly Gln
                   125                 130                 135
   Leu Trp Leu Asp Ala Tyr Leu His Gln Xaa Gln Gln Pro Pro Cys
                   140                 145                 150
   Pro Val Trp Lys Lys Arg Arg Lys Thr Leu Glu Pro Arg Gln Arg
                   155                 160                 165
   Gly Ala Xaa Gly Thr Met Pro Leu Xaa Phe Asn Thr Gln Arg Gly
                   170                 175                 180
   Leu Gly Leu Gly Thr Xaa Ser Leu Xaa Leu Lys Leu Leu Pro Phe
                   185                 190                 195
   Ile Phe Xaa Pro Gln Phe Xaa Asp Pro Lys Trp Gly Val Asp Thr
                   200                 205                 210
   Glu Val Pro Arg Arg Glu Gly Ala
                   215
(2) INFORMATION FOR SEQ ID NO:30:
   (i) SEQUENCE CHARACTERISTICS:
      (A) LENGTH: 71 amino acids
      (B) TYPE: amino acid
      (C) STRANDEDNESS: single
      (D) TOPOLOGY: linear
   (ii) MOLECULE TYPE: amino acid (Translation of Contig 3506132)
   (xi) SEQUENCE DESCRIPTION: SEQ ID NO:30:
(2) INFORMATION FOR SEQ ID NO:31:
   (i) SEQUENCE CHARACTERISTICS:
      (A) LENGTH: 306 amino acids
      (B) TYPE: amino acid
      (C) STRANDEDNESS: single
      (D) TOPOLOGY: linear
   (ii) MOLECULE TYPE: amino acid (Translation of Contig 3854933)
   (xi) SEQUENCE DESCRIPTION: SEQ ID NO:31:
   Gln Gly Pro Thr Pro Arg Tyr Phe Thr Trp Asp Glu Val Ala Gln
   1               5                   10                  15
   Arg Ser Gly Cys Glu Glu Arg Trp Leu Val Ile Asp Arg Lys Val
                   20                  25                  30
   Tyr Asn Ile Ser Glu Phe Thr Arg Arg His Pro Gly Gly Ser Arg
                   35                  40                  45
   Val Ile Ser His Tyr Ala Gly Gln Asp Ala Thr Asp Pro Phe Val
                   50                  55                  60
   Ala Phe His Ile Asn Lys Gly Leu Val Lys Lys Tyr Met Asn Ser
                   65                  70                  75
   Leu Leu Ile Gly Glu Leu Ser Pro Glu Gln Pro Ser Phe Glu Pro
                   80                  85                  90
   Thr Lys Asn Lys Glu Leu Thr Asp Glu Phe Arg Glu Leu Arg Ala
                   95                  100                 105
   Thr Val Glu Arg Met Gly Leu Met Lys Ala Asn His Val Phe Phe
                   110                 115                 120
   Leu Leu Tyr Leu Leu His Ile Leu Leu Leu Asp Gly Ala Ala Trp
                   125                 130                 135
   Leu Thr Leu Trp Val Phe Gly Thr Ser Phe Leu Pro Phe Leu Leu
                   140                 145                 150
   Cys Ala Val Leu Leu Ser Ala Val Gln Ala Gln Ala Gly Trp Leu
                   155                 160                 165
   Gln His Asp Phe Gly His Leu Ser Val Phe Ser Thr Ser Lys Trp
                   170                 175                 180
   Asn His Leu Leu His His Phe Val Ile Gly His Leu Lys Gly Ala
                   185                 190                 195
   Pro Ala Ser Trp Trp Asn His Met His Phe Gln His His Ala Lys
                   200                 205                 210
   Pro Asn Cys Phe Arg Lys Asp Pro Asp Ile Asn Met His Pro Phe
                   215                 220                 225
   Phe Phe Ala Leu Gly Lys Ile Leu Ser Val Glu Leu Gly Lys Gln
                   230                 235                 240
   Lys Lys Lys Tyr Met Pro Tyr Asn His Gln His Xaa Tyr Phe Phe
                   245                 250                 255
   Leu Ile Gly Pro Pro Ala Leu Leu Pro Leu Tyr Phe Gln Trp Tyr
                   260                 265                 270
   Ile Phe Tyr Phe Val Ile Gln Arg Lys Lys Trp Val Asp Leu Ala
                   275                 280                 285
   Trp Ile Ser Lys Gln Glu Tyr Asp Glu Ala Gly Leu Pro Leu Ser
                   290                 295                 300
   Thr Ala Asn Ala Ser Lys
                   305
(2) INFORMATION FOR SEQ ID NO:32:
   (i) SEQUENCE CHARACTERISTICS:
      (A) LENGTH: 566 amino acids
      (B) TYPE: amino acid
      (C) STRANDEDNESS: single
      (D) TOPOLOGY: linear
   (ii) MOLECULE TYPE: amino acid (Translation of Contig 2511785)
   (xi) SEQUENCE DESCRIPTION: SEQ TD NO:32:
(2) INFORMATION FOR SEQ ID NO:33:
   (i) SEQUENCE CHARACTERISTICS:
      (A) LENGTH: 619 amino acids
      (B) TYPE: amino acid
      (C) STRANDEDNESS: single
      (D) TOPOLOGY: linear
   (ii) MOLECULE TYPE: amino acid (Translation of Contig 2535)
   (xi) SEQUENCE DESCRIPTION: SEQ ID NO:33:
(2) INFORMATION FOR SEQ ID NO:34:
   (i) SEQUENCE CHARACTERISTICS:
      (A) LENGTH: 757 amino acids
      (B) TYPE: amino acid
      (C) STRANDEDNESS: single
      (D) TOPOLOGY: linear
   (ii) MOLECULE TYPE: amino acid (Translation of Contig 253538a)
   (xi) SEQUENCE DESCRIPTION: SEQ ID NO:34:

## Description

### RELATED APPLICATION

This application is a continuation in part application of Serial Number 08/833,610 filed April 11, 1997.

### INTRODUCTION

### Field of the Invention

This invention relates to modulating levels of enzymes and/or enzyme components relating to production of long chain poly-unsaturated fatty acids (PUFAs) in a microorganism or animal.

### Background

Two main families of polyunsaturated fatty acids (PUFAs) are the ω3 fatty acids, exemplified by eicosapentaenoic acid (EPA), and the ω6 fatty acids, exemplified by arachidonic acid (ARA). PUFAs are important components of the plasma membrane of the cell, where they may be found in such forms as phospholipids. PUFAs are necessary for proper development, particularly in the developing infant brain, and for tissue formation and repair. PUFAs also serve as precursors to other molecules of importance in human beings and animals, including the prostacyclins, eicosanoids, leukotrienes and prostaglandins.

Four major long chain PUFAs of importance include docosahexaenoic acid (DHA) and EPA, which are primarily found in different types of fish oil, gamma-linolenic acid (GLA), which is found in the seeds of a number of plants, including evening primrose (*Oenothera biennis*), borage (*Borago officinalis*) and black currants (*Ribes nigrum*), and stearidonic acid (SDA), which is found in marine oils and plant seeds. Both GLA and another important long chain PUFA, arachidonic acid (ARA), are found in filamentous fungi. ARA can be purified from animal tissues including liver and adrenal gland. GLA, ARA, EPA and SDA are themselves, or are dietary precursors to, important long chain fatty acids involved in prostaglandin synthesis, in treatment of heart disease, and in development of brain tissue.

Polyunsaturated fatty acids have a number of pharmaceutical and medical applications including treatment of heart disease, cancer and arthritis.

For DHA, a number of sources exist for commercial production including a variety of marine organisms, oils obtained from cold water marine fish, and egg yolk fractions. For ARA, microorganisms including the genera *Mortierella, Entomophthora, Phytium* and *Porphyridium* can be used for commercial production. Commercial sources of SDA include the genera *Trichodesma and Echium*. Commercial sources of GLA include evening primrose, black currants and borage. However, there are several disadvantages associated with commercial production of PUFAs from natural sources. Natural sources of PUFAs, such as animals and plants, tend to have highly heterogeneous oil compositions. The oils obtained from these sources therefore can require extensive purification to separate out one or more desired PUFAs or to produce an oil which is enriched in one or more PUFA. Natural sources also are subject to uncontrollable fluctuations in availability. Fish stocks may undergo natural variation or may be depleted by overfishing. Fish oils have unpleasant tastes and odors, which may be impossible to economically separate from the desired product, and can render such products unacceptable as food supplements. Animal oils, and particularly fish oils, can accumulate environmental pollutants. Weather and disease can cause fluctuation in yields from both fish and plant sources. Cropland available for production of alternate oil-producing crops is subject to competition from the steady expansion of human populations and the associated increased need for food production on the remaining arable land. Crops which do produce PUFAs, such as borage, have not been adapted to commercial growth and may not perform well in monoculture. Growth of such crops is thus not economically competitive where more profitable and better established crops can be grown. Large scale fermentation of organisms such as *Mortierella* is also expensive. Natural animal tissues contain low amounts of ARA and are difficult to process. Microorganisms such as *Porphyridium* and *Mortierella* are difficult to cultivate on a commercial scale.

Dietary supplements and pharmaceutical formulations containing PUFAs can retain the disadvantages of the PUFA source. Supplements such as fish oil capsules can contain low levels of the particular desired component and thus require large dosages. High dosages result in ingestion of high levels of undesired components, including contaminants. Unpleasant tastes and odors of the supplements can make such regimens undesirable, and may inhibit compliance by the patient. Care must be taken in providing fatty acid supplements, as overaddition may result in suppression of endogenous biosynthetic pathways and lead to competition with other necessary fatty acids in various lipid fractions *in vivo*, leading to undesirable results. For example, Eskimos having a diet high in ω3 fatty acids have an increased tendency to bleed (U.S. Pat. No. 4,874,603).

A number of enzymes are involved in PUFA biosynthesis. Linolenic acid (LA, 18:2 Δ9, 12) is produced from oleic acid (18:1 Δ°) by a Δ12-desaturase. GLA (18:3 Δ6, 9, 12) is produced from linoleic acid (LA, 18:2 Δ9, 12) by a Δ6-desaturase. ARA (20:4 Δ5, 8, 11, 14) production from dihomo-gamma-linolenic acid (DGLA, 20:3 Δ8, 11, 14) is catalyzed by a Δ5-desaturase. However, animals cannot desaturate beyond the Δ9 position and therefore cannot convert oleic acid (18:1 Δ9) into linolenic acid (18:2 Δ912). Likewise, α-linoleic acid (ALA, 18:3 Δ9, 12, 15) cannot be synthesized by mammals. Other eukaryotes, including fungi and plants, have enzymes which desaturate at positions Δ12 and Δ15. The major poly-unsaturated fatty acids of animals therefore are either derived from diet and/or from desaturation and elongation of linoleic acid (18:2 Δ9, 12) or α-linolenic acid (18:3 Δ9, 12, 15). Therefore it is of interest to obtain genetic material involved in PUFA biosynthesis from species that naturally produce these fatty acids and to express the isolated material in a microbial or animal system which can be manipulated to provide production of commercial quantities of one or more PUFAs. Thus there is a need for fatty acid desaturases, genes encoding them, and recombinant methods of producing them. A need further exists for oils containing higher relative proportions of and/or enriched in specific PUFAs. A need also exists for reliable economical methods of producing specific PUFAs.

### Relevant Literature

Production of gamma-linolenic acid by a Δ6-desaturase is described in USPN 5,552,306. Production of 8, 11-eicosadienoic acid using *Mortierella alpina* is disclosed in USPN 5,376,541. Production of docosahexaenoic acid by dinoflagellates is described in USPN 5,407,957. Cloning of a Δ6-palmitoylacyl carrier protein desaturase is described in PCT publication WO 96/13591 and USPN 5,614,400. Cloning of a Δ6-desaturase from borage is described in PCT publication WO 96/21022. Cloning of Δ9-desaturases is described in the published patent applications PCT WO 91/13972, EP 0 550 162 A1, EP 0 561 569 A2, EP 0 644 263 A2, and EP 0 736 598 A1, and in USPN 5,057,419. Cloning of Δ12-desaturases from various organisms is described in PCT publication WO 94/11516 and USPN 5,443,974. Cloning of Δ15-desaturases from various organisms is described in PCT publication WO 93/11245. All publications and U.S. patents or applications referred to herein are hereby incorporated in their entirety by reference.

### Summary of the Invention

The present invention relates to novel compositions and methods for preparation of poly-unsaturated long chain fatty acids or PUFAs. The compositions include nucleic acids encoding a Δ5-desaturase and/or polypeptides having Δ5-desaturase activity, the polypeptides, and probes for isolating and detecting the same. The methods involve growing a host microorganism or animal which contains and expresses one or more transgenes encoding a Δ5-desaturase and/or a polypeptide having Δ5-desaturase activity. Expression of the desaturase polypeptide provides for a relative increase in Δ5-desaturated PUFA, or metabolic progeny therefrom, as a result of altered concentrations of enzymes and substrates involved in PUFA biosynthesis. The invention finds use for example in the large scale production of PUFA containing oils which include, for example, ARA, EPA and/or DHA.

The present invention provides a purified or isolated polypeptide having Δ5 desaturase activity, which polypeptide is capable of desaturating a fatty acid molecule at carbon 5 from the carboxyl end of said fatty acid, said polypeptide having an amino acid sequence which has at least 60%, preferably at least 80%, and more preferably at least 90%, homology to the 446 amino acid sequence of SEQ ID NO:2.

The invention further provides a purified or isolated polypeptide having Δ5 desaturase activity, which polypeptide is a deletion mutant of SEQ ID NO: 2, said polypeptide being capable of desaturating a fatty acid molecule at carbon 5 from the carboxyl end of said fatty acid.

The polypeptide of the invention may include an amino acid motif selected from the group consisting of residues 30-38, 41-44, 171-175, 203-212 and 387-394 of SEQ ID NO:2. The polypeptide of the invention may comprise residues 30-38, 41-44, 171-175, 203-212 and 387-394 of SEQ ID NO:2. The polypeptide of the invention may comprise SEQ ID NO:2.

The invention further provides an isolated nucleic acid encoding a polypeptide of the invention. The isolated nucleic acid may comprise SEQ ID NO:1.

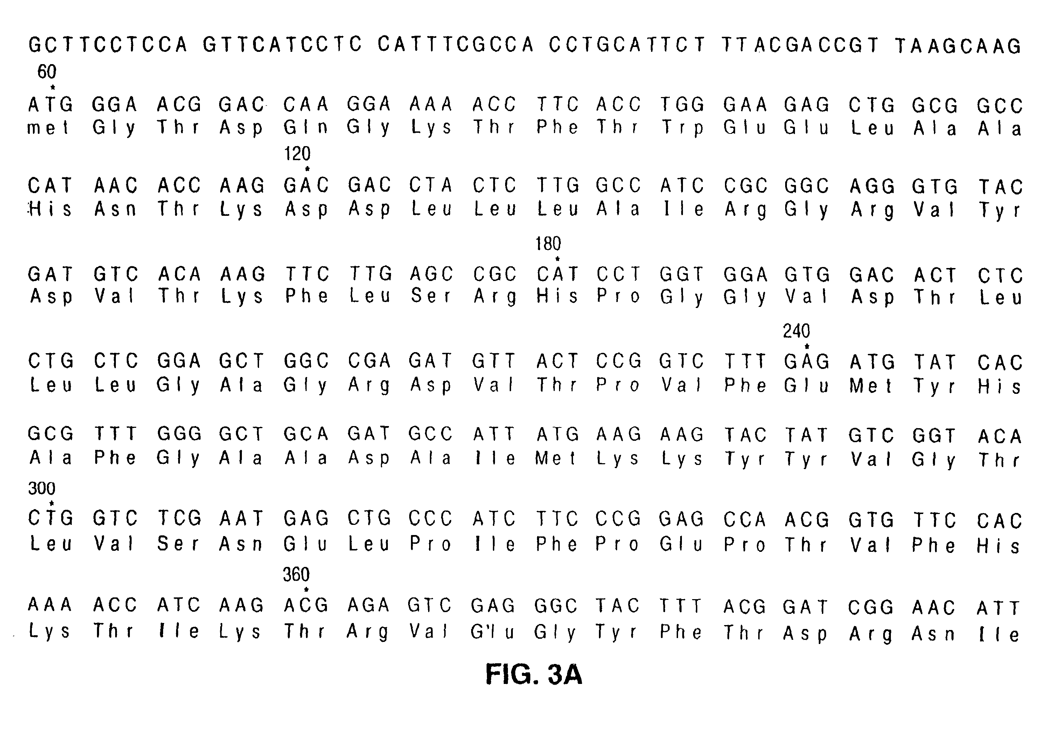

The nucleic acid is preferably derived from a eukaryotic cell, such as a fungal cell, or a fungal cell of the genus *Mortierella,* or of the genus/species *Mortierella alpina*. Also preferred is an isolated nucleic acid comprising a sequence which anneals to a nucleotide sequence depicted in FIG. 3A-3D (SEQ ID NO: 1). Also provided is an isolated nucleic acid sequence which hybridizes to a nucleotide sequence depicted in FIG. 3A-D (SEQ ID NO: 1).

The invention further provides a nucleic acid construct comprising a nucleic acid of the invention operably linked to a promoter. The present invention includes a nucleic acid construct comprising a nucleotide sequence depicted in a FIG. 3A-D (SEQ ID NO: 1) linked to a heterologous nucleic acid; a nucleic acid construct comprising a nucleotide sequence depicted in a FIG. 3A-D (SEQ ID NO: 1) operably linked to a promoter; and a nucleic acid construct comprising a nucleotide sequence depicted in a FIG. 3A-D (SEQ ID NO: 1) operably linked to a promoter which is functional in a microbial cell. In a preferred embodiment, the microbial cell is a yeast cell, and the nucleotide sequence is derived from a fungus, such as a fungus of the genus *Mortierella*, particularly a fungus of the species *Mortierella alpina*.

In another embodiment of the invention, a nucleic acid construct is provided which comprises a nucleotide sequence which encodes a polypeptide comprising an amino acid sequence which corresponds to an amino acid sequence depicted in FIG. 3A-D (SEQ ID NO: 2), wherein the nucleotide sequence is operably linked to a promoter which is functional in a host cell, and wherein the nucleotide sequence encodes a polypeptide which desaturates a fatty acid molecule at carbon 5 from the carboxyl end of a fatty acid molecule. Additionally, provided by the invention is a nucleic acid construct comprising a nucleotide sequence which encodes a functionally active Δ5-desaturase, where the desaturase includes an amino acid sequence which corresponds to all of or a portion of an amino acid sequence depicted in a FIG. 3A-D (SEQ ID NO: 2), wherein the nucleotide sequence is operably linked to a promoter functional in a host cell.

The invention also includes a host cell comprising a nucleic construct of the invention. The invention provides a recombinant host cell transformed with a nucleic acid of the invention or with a nucleic acid construct of the invention. The host cell may be a microbial host cell. The host cell may be a yeast cell.

In a preferred embodiment, a recombinant host cell is provided which comprises at least one copy of a DNA sequence which encodes a functionally active *Mortierella alpina* fatty acid desaturase having an amino acid sequence as depicted in FIG. 3A-D (SEQ ID NO: 2), wherein the cell or an ancestor of the cell was transformed with a vector comprising said DNA sequence, and wherein the DNA sequence is operably linked to a promoter. The host cell is either eukaryotic or prokaryotic. Preferred eukaryotic host cells are those selected from the group consisting of a mammalian cell, an insect cell, a fungal cell, and an algae cell. Preferred mammalian cells include an avian cell, a fungal cell such as a yeast, and a marine algae cell. Preferred prokaryotic cells include those selected from the group consisting of a bacteria, a cyanobacteria, cells which contain a bacteriophage, and/or a virus. The DNA sequence of the recombinant host cell preferably contains promoter which is functional in the host cell.

The host cells of the invention which contain the DNA sequences of the invention are enriched for fatty acids, such as 20:3 fatty acids. The invention provides a host cell which is enriched for 20:3, 20:4 or ω-3 20:4 fatty acids compared to a cell untransformed with a nucleic acid construct of the invention or a nucleic acid of the invention. A recombinant host cell of the invention may comprise a fatty acid selected from the group consisting of a dihomo-.gamma.-linoleic acid, n-6 eicosatrienoic acid, 20:3n-6 acid and 20:3 (8,11,14) acid.

The present invention also includes a method for the production of the fatty acid arachidonic acid which method comprises: growing a host cell of the invention in the presence of dihomo-γ-linolenic acid, under conditions wherein said acid is converted to arachidonic acid by the expression of a polypeptide of the invention; and recovering the fatty acid arachidonic acid from the culture.

The polypeptide of the invention is an enzyme which desaturates a fatty acid molecule at carbon 5 from the carboxyl end of the fatty acid molecule. In preferred embodiments of the invention the nucleic acid is derived from a *Mortierella sp*.; and the substrate for said polypeptide is exogenously supplied. The microbial cells used in the methods can be either eukaryotic cells or prokaryotic cells. The preferred eukaryotic cells are those selected from the group consisting of a mammalian cell, an insect cell, a fungal cell, and an algae cell. Preferred mammalian cells include an avian cell, a preferred fungal cell is a yeast, and the preferred algae cell is a marine algae cell. The preferred prokaryotic cells include those selected from the group consisting of a bacteria, a cyanobacteria, cells which contain a bacteriophage, and/or a virus. The nucleic acid sequence encoding the polypeptide of the microbial cell preferably contains a promoter which is functional in the host cell which optionally is an inducible promoter for example by components of the culture broth. The preferred microbial cells used in the methods are yeast cells, such as *Saccharomyces* cells.

A recombinant yeast cell of the invention may convert greater than about 5% of 20:3 fatty acid substrate to a 20:4 fatty acid product.

Also obtainable by methods of the invention is an oil comprising one or more PUFA. The amount of said one or more PUFAs is approximately 0.3-30% arachidonic acid (ARA), approximately 0.2-30% dihomo-.gamma.-linoleic acid (DGLA), and approximately 0.2-30% .gamma.-linoleic acid (GLA). The oil may have a ratio of ARA:DGLA:GLA that is approximately 1.0: 19.0:30 to 6.0:1.0:0.2.

Methods of the invention may further comprise formulating the fatty acid into a product selected from the group: a pharmaceutical composition comprising a pharmaceutically acceptable carrier; a nutritional formula; an infant formula; a dietary supplement; a dietary substitute; a cosmetic; and an animal feed. In a preferred embodiment, the nutritional compositions of the invention are in a liquid form or a solid form. The nutritional compositions of the invention preferably are administered to a mammalian host parenterally or internally.

The methods and materials of the present invention may be used in desaturating a fatty acid, by culturing a recombinant microbial cell of the invention under conditions suitable for expression of a polypeptide encoded by the nucleic acid, wherein the host cell further comprises a fatty acid substrate of the polypeptide. A fatty acid desaturated by methods of the invention may be formulated into an oil.

Purified nucleotide and peptide sequences related to nucleic acids and polypeptides of the invention are presented in SEQ ID NO:1-34. The sequences presented in SEQ ID NO:1-34 may be used as probes to identify related sequences, as components of expression systems and as components of systems useful for producing transgenic oil.

The present invention is further directed to methods of obtaining altered long chain poly unsaturated fatty acid biosystems by growing transgenic microbes which encode transgene expression products which desaturate a fatty acid molecule at carbon 5 from the carboxyl end of the fatty acid molecule.

The methods of the present invention may provide formulas, dietary supplements or dietary supplements in the form of a liquid or a solid containing the long chain fatty acids as defined in the claims. These formulas and supplements may be administered to a human or an animal.

The formulas and supplements produced by methods of the invention may further comprise at least one macronutrient selected from the group consisting of coconut oil, soy oil, canola oil, mono- and diglycerides, glucose, edible lactose, electrodialysed whey, electrodialysed skim milk, milk whey, soy protein, and other protein hydrolysates.

The formulas produced by methods of the present invention may further include at least one vitamin selected from the group consisting of Vitamins A, C, D, E, and B complex; and at least one mineral selected from the group consisting of calcium, magnesium, zinc, manganese, sodium, potassium, phosphorus, copper, chloride, iodine, selenium, and iron.

The present invention also provides uses of the host cell of the invention as set out in the claims.

The present invention may be used in a method of treating a patient having a condition caused by insuffient intake or production of polyunsaturated fatty acids comprising administering to the patient a dietary substitute of the invention in an amount sufficient to effect treatment of the patient.

The present invention may be used to make cosmetic and pharmaceutical compositions of the material of the invention.

Sequences having regions of similarity with the nucleotide sequences of the present invention are shown in the group consisting of: SEQ ID NO:13; SEQ ID NO:15; SEQ ID NO:17; SEQ ID NO:19; SEQ ID NO:21; SEQ ID NO:22; SEQ ID NO:22; SEQ ID NO:24; SEQ ID NO:25; SEQ ID NO:26 and SEQ ID NO:17.

Sequences having regions of similarity with polypeptides of the present invention are shown in the group consisting of: SEQ ID NO:14; SEQ ID NO:16; SEQ ID NO:18; SEQ ID NO:20; SEQ ID NO:28; SEQ ID NO:29; SEQ ID NO:30; SEQ ID NO:31; SEQ ID NO:32; SEQ ID NO:33 and SEQ ID NO:34.

Methods of the present invention may be used to produce transgenic oils in pharmaceutically acceptable carriers, and nutritional supplements, cosmetic agents and infant formulae containing transgenic oils.

The present invention may be used in a method for obtaining altered long chain polyunsaturated fatty acid biosynthesis comprising the steps of: growing a microbe having cells which contain a transgene which encodes a polypeptide of the invention, wherein the trangene is operably associated with an expression control sequence, under conditions whereby the transgene is expressed, whereby long chain polyunsaturated fatty acid biosynthesis in the cells is altered.

The present invention also relates to making chain polyunsaturated fatty acid selected from the group consisting of ARA, DGLA and EPA.

Methods of the present invention may produce pharmaceutical compositions comprising at least one nutrient selected from the group consisting of a vitamin, a mineral, a carbohydrate, a sugar, an amino acid, a free fatty acid, a phospholipid, an antioxidant, and a phenolic compound.

### Brief Description of the Drawings

Figure 1 shows possible pathways for the synthesis of arachidonic acid (20:4 Δ5, 8, 11, 14) and stearidonic acid (18:4 Δ6, 9, 12, 15) from palmitic acid (C₁₆) from a variety of organisms, including algae, *Mortierella* and humans. These PUFAs can serve as precursors to other molecules important for humans and other animals, including prostacyclins, leukotrienes, and prostaglandins, some of which are shown.
Figure 2 shows possible pathways for production of PUFAs in addition to ARA, including EPA and DHA, for a variety of organisms.
Figure 3A-D shows the DNA sequence of the *Mortierella alpina* Δ5-desaturase and the deduced amino acid sequence.

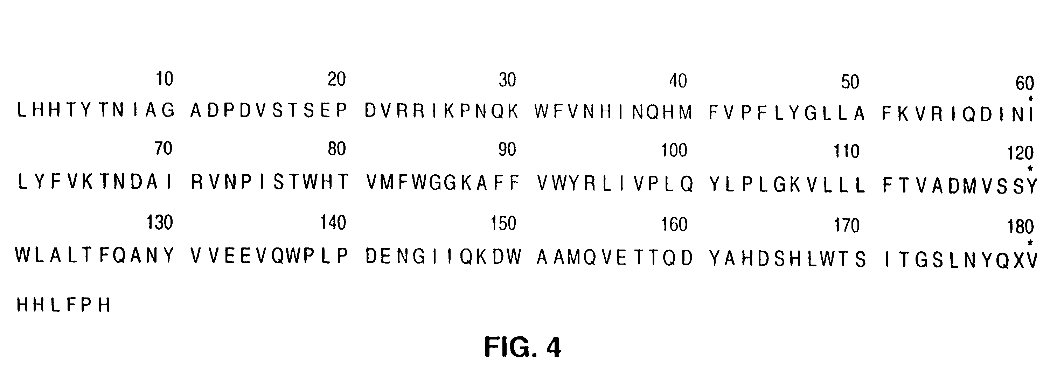

Figure 4 shows the deduced amino acid sequence of the PCR fragment (see Example 1)
Figure 5A and 5B show alignments of the protein sequence of the Δ5-desaturase with Δ6-desaturases.
Figure 6A and 6B show the effect of the timing of substrate addition relative to induction on conversion of substrate to product in SC334 containing the Δ5-desaturase gene.
Figure 7A and 7B show the effect of inducer concentration on Δ5-desaturase expression in SC334.
Figure 8A and 8B show the effect of induction temperature on Δ5-desaturase activity in SC334.
Figure 9A and 9B show the effect of host strain on the conversion of substrate to product in strains expressing the Δ5-desaturase gene at 15°C.
Figure 10A and 10B show the effect of host strain on the conversion of substrate to product in strains expressing the Δ5-desaturase gene at 30°C.
Figure 11 shows the effect of a host strain expressing choline transferase as well as the Δ5-desaturase gene on the conversion of substrate to product.
Figure 12A and 12B show the effect of media composition and temperature on the conversion of substrate to product in two host strains expressing the Δ5-desaturase gene.
Figure 13 shows alignment of the protein sequence of Ma 29 and contig 253538a.
Figure 14 shows alignment of the protein sequence of Ma 524 and contig 253538a.

### Brief Description of the Sequence Listings

SEQ ID NO:1 shows a DNA sequence of the *Mortierella alpina* Δ5-desaturase.

SEQ ID NO:2 shows an amino acid sequence of *Mortierella alpina* Δ5-desaturase.

SEQ ID NO: 3 shows the deduced amino acid sequence of the *M*. *alpina* PCR fragment (see Example 1).

SEQ ID NO: 4 - SEQ ID NO: 7 show the deduced amino acid sequences of various Δ6-desaturases.

SEQ ID NO: 8 and SEQ ID NO: 9 show PCR primer sequences for Δ6-desaturases

SEQ ID NO: 10 shows a primer for reverse transcription of total RNA.

SEQ ID NO: 11 and SEQ ID NO: 12 show amino acid motifs for desaturase sequences.

SEQ ID NO: 13 and SEQ ID NO: 14 show the nucleotide and amino acid sequence of a *Dictyostelium discoideum* desaturase sequence.

SEQ ID NO: 15 and SEQ ID NO: 16 show the nucleotide and amino acid sequence of a *Phaeodactylum tricornutum* desaturase sequence.

SEQ ID NO: 17-20 show the nucleotide and deduced amino acid sequence of a Schizochytrium cDNA clone.

SEQ ID NO: 21-27 show nucleotide sequences for human desaturases.

SEQ ID NO: 28 - SEQ ID NO: 34 show peptide sequences for human desaturases.

### Detailed Description of the Invention

In order to ensure a complete understanding of the invention, the following definitions are provided:
**Δ5-Desaturase:** Δ5 desaturase is an enzyme which introduces a double bond between carbons 5 and 6 from the carboxyl end of a fatty acid molecule.
**Δ6-Desaturase:** Δ6-desaturase is an enzyme which introduces a double bond between carbons 6 and 7 from the carboxyl end of a fatty acid molecule.
**Δ9-Desaturase:** Δ9-desaturase is an enzyme which introduces a double bond between carbons 9 and 10 from the carboxyl end of a fatty acid molecule.
**Δ12-Desaturase:** Δ12-desaturase is an enzyme which introduces a double bond between carbons 12 and 13 from the carboxyl end of a fatty acid molecule.

**Fatty Acids:** Fatty acids are a class of compounds containing a long hydrocarbon chain and a terminal carboxylate group. Fatty acids include the following:

| **Fatty Acid** | | |
|---|---|---|
| 12:0 | lauric acid | |
| 16:0 | palmitic acid | |
| 16:1 | palmitoleic acid | |
| 18:0 | stearic acid | |
| 18:1 | oleic acid | Δ9-18:1 |
| 18:2 Δ5,9 | taxoleic acid | Δ5,9-18:2 |
| 18:2 Δ6,9 | 6,9-octadecadienoic acid | Δ6,9-18:2 |
| 18:2 | linoleic acid | Δ9,12-18:2 (LA) |
| 18:3 Δ6,9,12 | gamma-linolenic acid | Δ6,9,12-18:3 (GLA) |
| 18:3 Δ5,9,12 | pinolenic acid | Δ5,9,12-18:3 |
| 18:3 | alpha-linoienic acid | Δ9,12,15-18:3 (ALA) |
| 18:4 | stearidonic acid | Δ6,9,12,15-18:4 (SDA) |
| 20:0 | Arachidic acid | |
| 20:1 | Eicoscenic Acid | |
| 22:0 | behehic acid | |
| 22:1 | erucic acid | |
| 22:2 | Docasadienoic acid | |
| 20:4 ω6 | arachidonic acid | Δ5,8,11,14-20:4 (ARA) |
| 20:3 w6 | ω6-eicosatrienoic dihomo-gamma linolenic | Δ8,11,14-20:3 (DGLA) |
| 20:5 ω3 | Eicosapentanoic (Timnodonic acid) | Δ5,8,11,14,17-20:5 (EPA) |
| 20:3 ω3 | ω3-eicosatrienoic | Δ11,16,17-20:3 |
| 20:4 ω3 | ω3-eicosatetraenoic | Δ8,11,14,17-20:4 |
| 22:5 ω3 | Docasapentaenoic | Δ7,10,13,16,19-22:5 (ω3DPA) |
| 22:6 ω3 | Docosahexaenoic (cervonic acid) | Δ4,7,10,13,16,19-22:6 (DHA) |
| 24:0 | Lignoceric acid | |

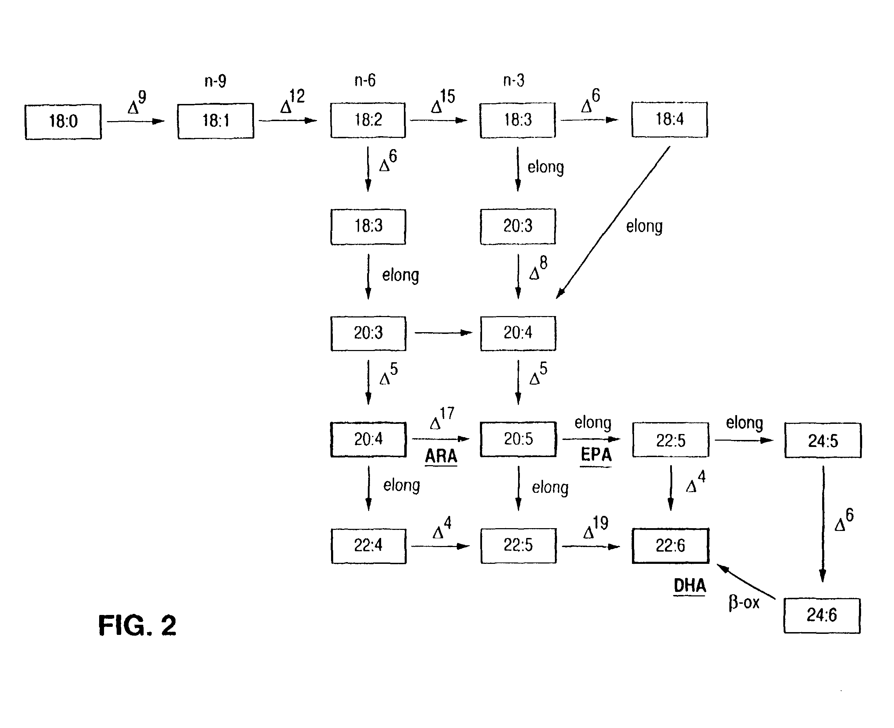

Taking into account these definitions, the present invention is directed to novel DNA sequences, DNA constructs, methods and compositions are provided which permit modification of the poly-unsaturated long chain fatty acid content of, for example, microbial cells or animals. Host cells are manipulated to express a sense or antisense transcript of a DNA encoding a polypeptide(s) which catalyzes the conversion of DGLA to ARA. The substrate(s) for the expressed enzyme may be produced by the host cell or may be exogenously supplied. To achieve expression, the transformed DNA is operably associated with transcriptional and translational initiation and termination regulatory regions that are functional in the host cell. Constructs comprising the gene to be expressed can provide for integration into the genome of the host cell or can autonomously replicate in the host cell. For production of ARA, the expression cassettes generally used include a cassette which provides for Δ5-desaturase activity, particularly in a host cell which produces or can take up DGLA. Production of ω6-type unsaturated fatty acids, such as ARA, is favored in a host microorganism or animal which is substantially free of ALA. The host is selected or obtained by removing or inhibiting activity of a Δ15- or ω3- type desaturase (see Figure 2). The endogenous desaturase activity can be affected by providing an expression cassette for an antisense Δ15 or ω3 transcript, by disrupting a target Δ15- or ω3-desaturase gene through insertion, substitution and/or deletion of all or part of the target gene, or by adding a Δ15-or ω3-desaturase inhibitor. Production of LA also can be increased by providing expression cassettes for Δ9 and/or Δ12-desaturases where their respective enzymatic activities are limiting.

### MICROBIAL PRODUCTION OF FATTY ACIDS

Microbial production of fatty acids has several advantages over purification from natural sources such as fish or plants. Many microbes are known with greatly simplified oil compositions compared with those of higher organisms, making purification of desired components easier. Microbial production is not subject to fluctuations caused by external variables such as weather and food supply. Microbially produced oil is substantially free of contamination by environmental pollutants. Additionally, microbes can provide PUFAs in particular forms which may have specific uses. For example, *Spirulina* can provide PUFAs predominantly at the first and third positions of triglycerides; digestion by pancreatic lipases preferentially releases fatty acids from these positions. Following human or animal ingestion of triglycerides derived from *Spirulina*, these PUFAs are released by pancreatic lipases as free fatty acids and thus are directly available, for example, for infant brain development. Additionally, microbial oil production can be manipulated by controlling culture conditions, notably by providing particular substrates for microbially expressed enzymes, or by addition of compounds which suppress undesired biochemical pathways. In addition to these advantages, production of fatty acids from recombinant microbes provides the ability to alter the naturally occurring microbial fatty acid profile by providing new synthetic pathways in the host or by suppressing undesired pathways, thereby increasing levels of desired PUFAs, or conjugated forms thereof, and decreasing levels of undesired PUFAs.

### PRODUCTION OF FATTY ACIDS IN ANIMALS

Production of fatty acids in animals also presents several advantages. Expression of desaturase genes in animals can produce greatly increased levels of desired PUFAs in animal tissues, making recovery from those tissues more economical. For example, where the desired PUFAs are expressed in the breast milk of animals, methods of isolating PUFAs from animal milk are well established. In addition to providing a source for purification of desired PUFAs, animal breast milk can be manipulated through expression of desaturase genes, either alone or in combination with other human genes, to provide animal milks with a PUFA composition substantially similar to human breast milk during the different stages of infant development. Humanized animal milks could serve as infant formulas where human nursing is impossible or undesired, or in cases of malnourishment or disease.

Depending upon the host cell, the availability of substrate, and the desired end product(s), several polypeptides, particularly desaturases, are of interest. By "desaturase" is intended a polypeptide which can desaturate one or more fatty acids to produce a mono- or poly-unsaturated fatty acid or precursor thereof of interest. Of particular interest are polypeptides which can catalyze the conversion of DGLA to produce ARA which includes enzymes which desaturate at the Δ5 position. By "polypeptide" is meant any chain of amino acids, regardless of length or post-translational modification, for example, glycosylation or phosphorylation. Considerations for choosing a specific polypeptide having desaturase activity include the pH optimum of the polypeptide, whether the polypeptide is a rate limiting enzyme or a component thereof, whether the desaturase used is essential for synthesis of a desired poly-unsaturated fatty acid, and/or co-factors required by the polypeptide. The expressed polypeptide preferably has parameters compatible with the biochemical environment of its location in the host cell. For example, the polypeptide may have to compete for substrate with other enzymes in the host cell. Analyses of the Kₘ and specific activity of the polypeptide in question therefore are considered in determining the suitability of a given polypeptide for modifying PUFA production in a given host cell. The polypeptide used in a particular situation is one which can function under the conditions present in the intended host cell but otherwise can be any polypeptide having desaturase activity which has the desired characteristic of being capable of modifying the relative production of a desired PUFA.

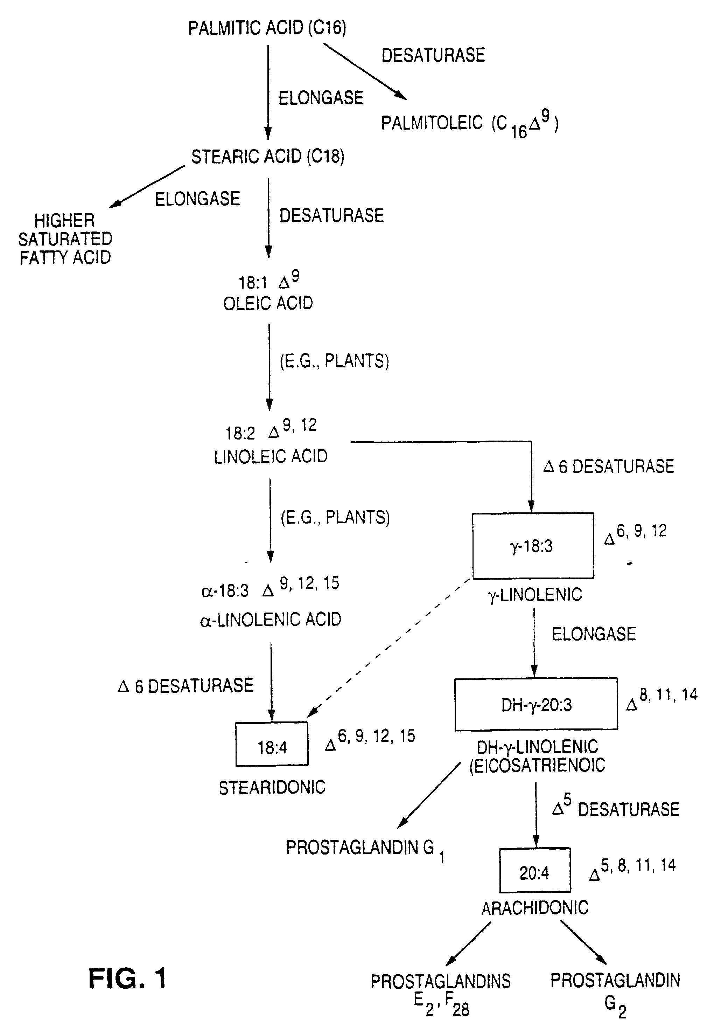

For production of ARA, the DNA sequence used encodes a polypeptide having Δ5-desaturase activity. In particular instances, this can be coupled with an expression cassette which provides for production of a polypeptide having Δ6-desaturase activity and the host cell can optionally be depleted of any Δ15-desaturase activity present, for example by providing a transcription cassette for production of antisense sequences to the Δ15-desaturase transcription product, by disrupting the Δ15-desaturase gene, or by using a host cell which naturally has, or has been mutated to have, low Δ15-desaturase activity. Inhibition of undesired desaturase pathways also can be accomplished through the use of specific desaturase inhibitors such as those described in U.S. Patent No. 4,778,630. The choice of combination of cassettes used can depend in part on the PUFA profile of the host cell. Where the host cell Δ5-desaturase activity is limiting, overexpression of Δ5-desaturase alone generally will be sufficient to provide for enhanced ARA production in the presence of an appropriate substrate such as DGLA. ARA production also can be increased by providing expression cassettes for Δ9- or Δ12-desaturase genes when the activities of those desaturases are limiting. A scheme for the synthesis of arachidonic acid (20:4 *Δ*^{*5, 8, 11, 14*}) from palmitic acid (C₁₆) is shown in Figure 1. A key enzyme in this pathway is a Δ5-desaturase which converts DH-γ-linolenic acid (DGLA, eicosatrienoic acid) to ARA. Conversion of α-linolenic acid (ALA) to stearidonic acid by a Δ6-desaturase is also shown. Production of PUFAs in addition to ARA, including EPA and DHA is shown in Figure 2.

### SOURCES OF POLYPEPTIDES HAVING DESATURASE ACTIVITY

A source of polypeptides having desaturase activity and oligonucleotides encoding such polypeptides are organisms which produce a desired poly-unsaturated fatty acid. As an example, microorganisms having an ability to produce ARA can be used as a source of Δ5-desaturase activity. Such microorganisms include, for example, those belonging to the genera *Mortierella, Conidiobolus, Pythium, Phytophathora, Penicillium, Porphyridium, Coidosporium, Mucor, Fusarium, Aspergillus, Rhodotorula*, and *Entomophthora*. Within the genus *Porphyridium*, of particular interest is *Porphyridium cruentum*. Within the genus *Mortierella*, of particular interest are *Mortierella elongata, Mortierella exigua, Mortierella hygrophila, Mortierella ramanniana*, var. angulispora, and *Mortierella alpina*. Within the genus *Mucor,* of particular interest are *Mucor circinelloides* and *Mucor javanicus*.

DNAs encoding desired desaturases can be identified in a variety of ways. As an example, a source of the desired desaturase, for example genomic or cDNA libraries from *Mortierella,* is screened with detectable enzymatically-or chemically-synthesized probes, which can be made from DNA, RNA, or non-naturally occurring nucleotides, or mixtures thereof. Probes may be enzymatically synthesized from DNAs of known desaturases for normal or reduced-stringency hybridization methods. Oligonucleotide probes also can be used to screen sources and can be based on sequences of known desaturases, including sequences conserved among known desaturases, or on peptide sequences obtained from the desired purified protein. Oligonucleotide probes based on amino acid sequences can be degenerate to encompass the degeneracy of the genetic code, or can be biased in favor of the preferred codons of the source organism. Oligonucleotides also can be used as primers for PCR from reverse transcribed mRNA from a known or suspected source; the PCR product can be the full length cDNA or can be used to generate a probe to obtain the desired full length cDNA. Alternatively, a desired protein can be entirely sequenced and total synthesis of a DNA encoding that polypeptide performed.

Once the desired genomic or cDNA has been isolated, it can be sequenced by known methods. It is recognized in the art that such methods are subject to errors, such that multiple sequencing of the same region is routine and is still expected to lead to measurable rates of mistakes in the resulting deduced sequence, particularly in regions having repeated domains, extensive secondary structure, or unusual base compositions, such as regions with high GC base content. When discrepancies arise, resequencing can be done and can employ special methods. Special methods can include altering sequencing conditions by using: different temperatures; different enzymes; proteins which alter the ability of oligonucleotides to form higher order structures; altered nucleotides such as ITP or methylated dGTP; different gel compositions, for example adding formamide; different primers or primers located at different distances from the problem region; or different templates such as single stranded DNAs. Sequencing of mRNA also can be employed.

For the most part, some or all of the coding sequence for the polypeptide having desaturase activity is from a natural source. In some situations, however, it is desirable to modify all or a portion of the codons, for example, to enhance expression, by employing host preferred codons. Host preferred codons can be determined from the codons of highest frequency in the proteins expressed in the largest amount in a particular host species of interest. Thus, the coding sequence for a polypeptide having desaturase activity can be synthesized in whole or in part. All or portions of the DNA also can be synthesized to remove any destabilizing sequences or regions of secondary structure which would be present in the transcribed mRNA. All or portions of the DNA also can be synthesized to alter the base composition to one more preferable in the desired host cell. Methods for synthesizing sequences and bringing sequences together are well established in the literature. *In vitro* mutagenesis and selection, site-directed mutagenesis, or other means can be employed to obtain mutations of naturally occurring desaturase genes to produce a polypeptide having desaturase activity *in vivo* with more desirable physical and kinetic parameters for function in the host cell, such as a longer half-life or a higher rate of production of a desired polyunsaturated fatty acid.

### Mortierella alpina Desaturase

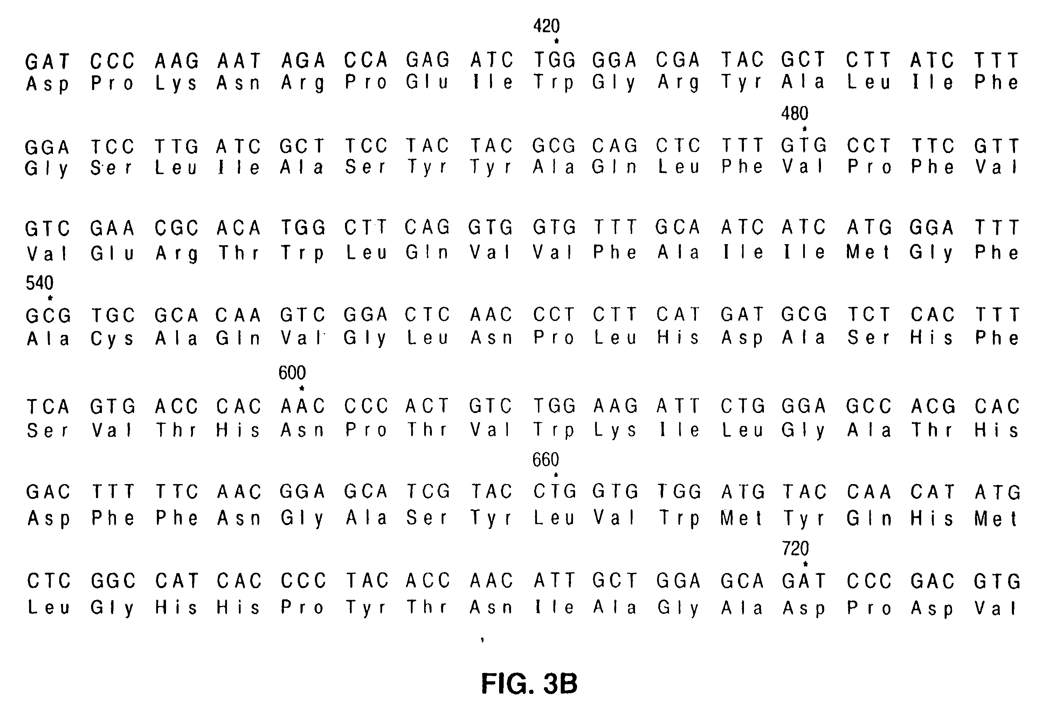

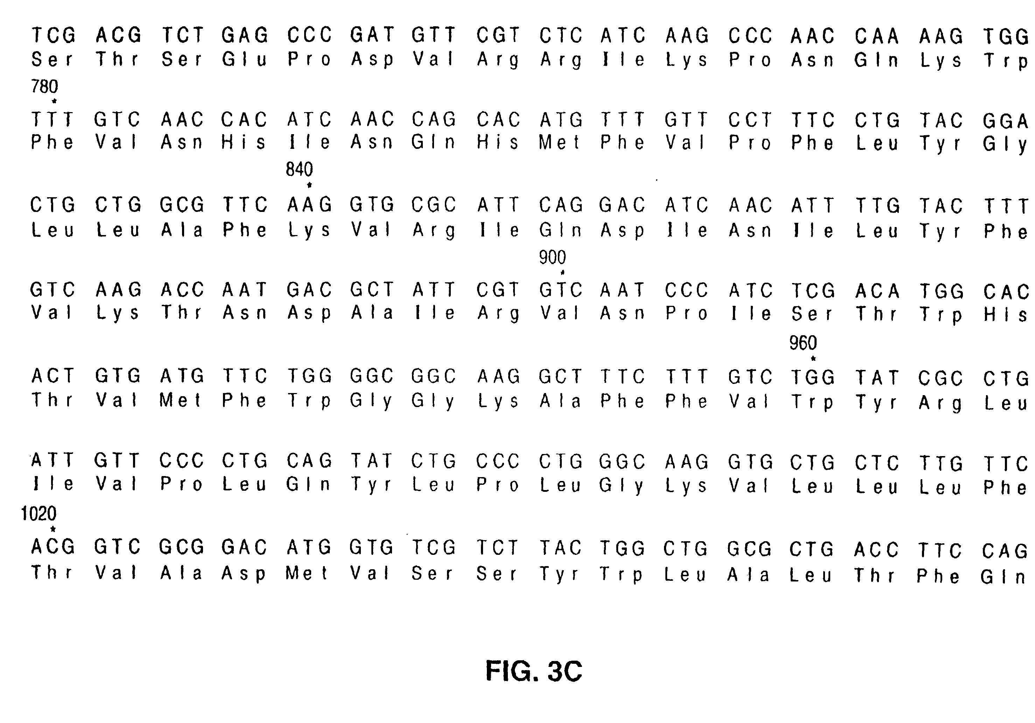

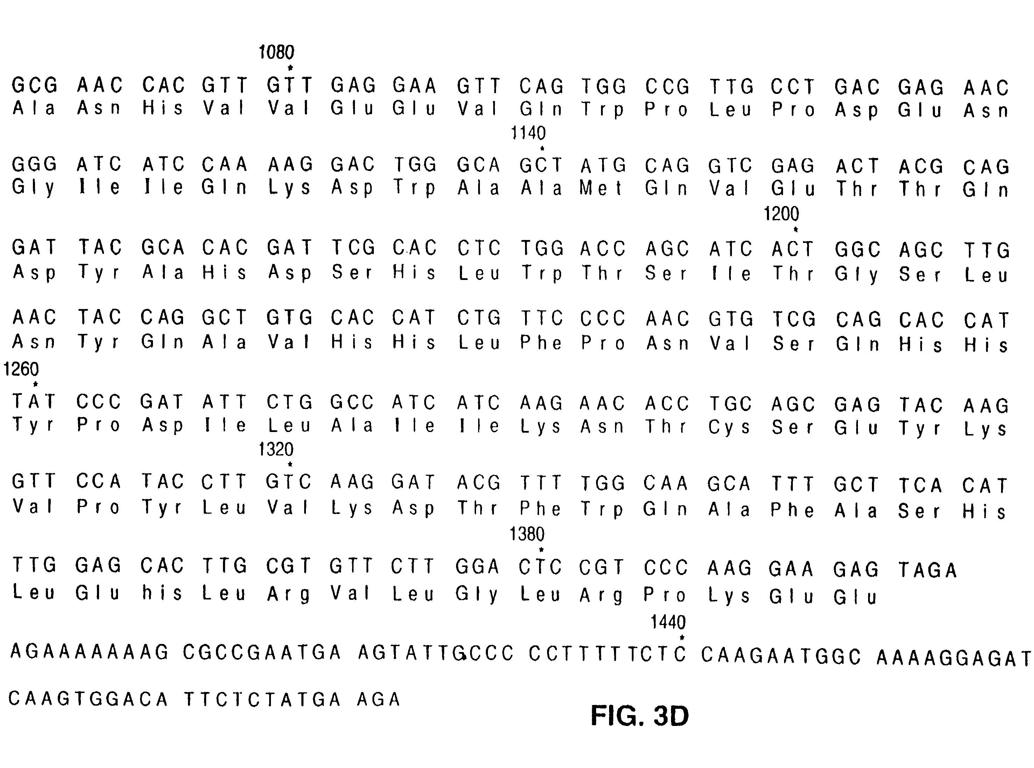

Of particular interest is the *Mortierella alpina* Δ5-desaturase which has 446 amino acids; the amino acid sequence is shown in Figure 3. The gene encoding the *Mortierella alpina* Δ5-desaturase can be expressed in transgenic microorganisms or animals to effect greater synthesis of ARA from DGLA. Other DNAs which are substantially identical to the *Mortierella alpina* Δ5-desaturase DNA, or which encode polypeptides which are substantially identical to the *Mortierella alpina* Δ5-desaturase polypeptide, also can be used. By substantially identical is intended an amino acid sequence or nucleic acid sequence exhibiting in order of increasing preference at least 60%, 80%, 90% or 95% homology to the *Mortierella alpina* Δ5-desaturase amino acid sequence or nucleic acid sequence encoding the amino acid sequence. For polypeptides, the length of comparison sequences generally is at least 16 amino acids, preferably at least 20 amino acids, or most preferably 35 amino acids. For nucleic acids, the length of comparison sequences generally is at least 50 nucleotides, preferably at least 60 nucleotides, and more preferably at least 75 nucleotides, and most preferably, 110 nucleotides. Homology typically is measured using sequence analysis software, for example, the Sequence Analysis software package of the Genetics Computer Group, University of Wisconsin Biotechnology Center, 1710 University Avenue, Madison, Wisconsin 53705, MEGAlign (DNAStar, Inc., 1228 S. Park St., Madison, Wisconsin 53715), and MacVector (Oxford Molecular Group, 2105 S. Bascom Avenue, Suite 200, Campbell, California 95008). Such software matches similar sequences by assigning degrees of homology to various substitutions, deletions, and other modifications. Conservative substitutions typically include substitutions within the following groups: glycine and alanine; valine, isoleucine and leucine; aspartic acid, glutamic acid, asparagine, and glutamine; serine and threonine; lysine and arginine; and phenylalanine and tyrosine. Substitutions may also be made on the basis of conserved hydrophobicity or hydrophilicity (Kyte and Doolittle, *J. Mol. Biol*. 157: 105-132, 1982), or on the basis of the ability to assume similar polypeptide secondary structure (Chou and Fasman, *Adv. Enzymol*. 47: 45-148, 1978).

### Other Desaturases

Encompassed by the present invention are related desaturases from the same or other organisms as defined in the claims. Such related desaturases include variants of the disclosed Δ5-desarurase naturally occurring within the same or different species of *Mortierella*, as well as homologues of the disclosed Δ5-desaturase from other species. Also included are desaturases which, although not substantially identical to the *Mortierella alpina* Δ5-desaturase, desaturate a fatty acid molecule at carbon 5 from the carboxyl end of a fatty acid molecule. Related desaturases can be identified by their ability to function substantially the same as the disclosed desaturases; that is, are still able to effectively convert DGLA to ARA. Related desaturases also can be identified by screening sequence databases for sequences homologous to the disclosed desaturase, by hybridization of a probe based on the disclosed desaturase to a library constructed from the source organism, or by RT-PCR using mRNA from the source organism and primers based on the disclosed desaturase. Such desaturases include those from humans, *Dictyostelium discoideum* and *Phaeodactylum tricornum*.

The regions of a desaturase polypeptide important for desaturase activity can be determined through routine mutagenesis, expression of the resulting mutant polypeptides and determination of their activities. Mutants may include deletions, insertions and point mutations, or combinations thereof. A typical functional analysis begins with deletion mutagenesis to determine the N- and C-terminal limits of the protein necessary for function, and then internal deletions, insertions or point mutants are made to further determine regions necessary for function. Other techniques such as cassette mutagenesis or total synthesis also can be used. Deletion mutagenesis is accomplished, for example, by using exonucleases to sequentially remove the 5' or 3' coding regions. Kits are available for such techniques. After deletion, the coding region is completed by ligating oligonucleotides containing start or stop codons to the deleted coding region after 5' or 3' deletion, respectively. Alternatively, oligonucleotides encoding start or stop codons are inserted into the coding region by a variety of methods including site-directed mutagenesis, mutagenic PCR or by ligation onto DNA digested at existing restriction sites. Internal deletions can similarly be made through a variety of methods including the use of existing restriction sites in the DNA, by use of mutagenic primers via site directed mutagenesis or mutagenic PCR. Insertions are made through methods such as linker-scanning mutagenesis, site-directed mutagenesis or mutagenic PCR. Point mutations are made through techniques such as site-directed mutagenesis or mutagenic PCR.

Chemical mutagenesis also can be used for identifying regions of a desaturase polypeptide important for activity. A mutated construct is expressed, and the ability of the resulting altered protein to function as a desaturase is assayed. Such structure-function analysis can determine which regions may be deleted, which regions tolerate insertions, and which point mutations allow the mutant protein to function in substantially the same way as the native desaturase. All such mutant proteins and nucleotide sequences encoding them are within the scope of the present invention.

### EXPRESSION OF DESATURASE GENES

Once the DNA encoding a desaturase polypeptide has been obtained, it is placed in a vector capable of replication in a host cell, or is propagated *in vitro* by means of techniques such as PCR or long PCR. Replicating vectors can include plasmids, phage, viruses, cosmids and the like. Desirable vectors include those useful for mutagenesis of the gene of interest or for expression of the gene of interest in host cells. The technique of long PCR has made *in vitro* propagation of large constructs possible, so that modifications to the gene of interest, such as mutagenesis or addition of expression signals, and propagation of the resulting constructs can occur entirely *in vitro* without the use of a replicating vector or a host cell.

For expression of a desaturase polypeptide, functional transcriptional and translational initiation and termination regions are operably linked to the DNA encoding the desaturase polypeptide. Expression of the polypeptide coding region can take place *in vitro* or in a host cell. Transcriptional and translational initiation and termination regions are derived from a variety of nonexclusive sources, including the DNA to be expressed, genes known or suspected to be capable of expression in the desired system, expression vectors, chemical synthesis, or from an endogenous locus in a host cell.

### Expression In Vitro

*In vitro* expression can be accomplished, for example, by placing the coding region for the desaturase polypeptide in an expression vector designed for *in vitro* use and adding rabbit reticulocyte lysate and cofactors; labeled amino acids can be incorporated if desired. Such *in vitro* expression vectors may provide some or all of the expression signals necessary in the system used. These methods are well known in the art and the components of the system are commercially available. The reaction mixture can then be assayed directly for the polypeptide, for example by determining its activity, or the synthesized polypeptide can be purified and then assayed.

### Expression In A Host Cell

Expression in a host cell can be accomplished in a transient or stable fashion. Transient expression can occur from introduced constructs which contain expression signals functional in the host cell, but which constructs do not replicate and rarely integrate in the host cell, or where the host cell is not proliferating. Transient expression also can be accomplished by inducing the activity of a regulatable promoter operably linked to the gene of interest, although such inducible systems frequently exhibit a low basal level of expression. Stable expression can be achieved by introduction of a construct that can integrate into the host genome or that autonomously replicates in the host cell. Stable expression of the gene of interest can be selected for through the use of a selectable marker located on or transfected with the expression construct, followed by selection for cells expressing the marker. When stable expression results from integration, integration of constructs can occur randomly within the host genome or can be targeted through the use of constructs containing regions of homology with the host genome sufficient to target recombination with the host locus. Where constructs are targeted to an endogenous locus, all or some of the transcriptional and translational regulatory regions can be provided by the endogenous locus.

When increased expression of the desaturase polypeptide in the source organism is desired, several methods can be employed. Additional genes encoding the desaturase polypeptide can be introduced into the host organism. Expression from the native desaturase locus also can be increased through . homologous recombination, for example by inserting a stronger promoter into the host genome to cause increased expression, by removing destabilizing sequences from either the mRNA or the encoded protein by deleting that information from the host genome, or by adding stabilizing sequences to the mRNA (USPN 4,910,141).

When it is desirable to express more than one different gene, appropriate regulatory regions and expression methods, introduced genes can be propagated in the host cell through use of replicating vectors or by integration into the host genome. Where two or more genes are expressed from separate replicating vectors. it is desirable that each vector has a different means of replication. Each introduced construct, whether integrated or not, should have a different means of selection and should lack homology to the other constructs to maintain stable expression and prevent reassortment of elements among constructs. Judicious choices of regulatory regions, selection means and method of propagation of the introduced construct can be experimentally determined so that all introduced genes are expressed at the necessary levels to provide for synthesis of the desired products.

As an example, where the host cell is a yeast, transcriptional and translational regions functional in yeast cells are provided, particularly from the host species. The transcriptional initiation regulatory regions can be obtained, for example from genes in the glycolytic pathway, such as alcohol dehydrogenase, glyceraldehyde-3-phosphate dehydrogenase (GPD), phosphoglucoisomerase, phosphoglycerate kinase, etc. or regulatable genes such as acid phosphatase, lactase, metallothionein, glucoamylase, etc. Any one of a number of regulatory sequences can be used in a particular situation, depending upon whether constitutive or induced transcription is desired, the particular efficiency of the promoter in conjunction with the open-reading frame of interest, the ability to join a strong promoter with a control region from a different promoter which allows for inducible transcription, ease of construction, and the like. Of particular interest are promoters which are activated in the presence of galactose. Galactose-inducible promoters (GAL1, GAL7, and GAL10) have been extensively utilized for high level and regulated expression of protein in yeast (Lue *et al., Mol. Cell. Biol*. Vol. 7, p. 3446, 1987; Johnston, *Microbiol. Rev*. Vol. 51, p. 458, 1987). Transcription from the GAL promoters is activated by the GAL4 protein, which binds to the promoter region and activates transcription when galactose is present. In the absence of galactose, the antagonist GAL80 binds to GAL4 and prevents GAL4 from activating transcription. Addition of galactose prevents GAL80 from inhibiting activation by GAL4.

Nucleotide sequences surrounding the translational initiation codon ATG have been found to affect expression in yeast cells. If the desired polypeptide is poorly expressed in yeast, the nucleotide sequences of exogenous genes can be modified to include an efficient yeast translation initiation sequence to obtain optimal gene expression. For expression in *Saccharomyces*, this can be done by site-directed mutagenesis of an inefficiently expressed gene by fusing it in-frame to an endogenous *Saccharomyces* gene, preferably a highly expressed gene, such as the lactase gene.

The termination region can be derived from the 3' region of the gene from which the initiation region was obtained or from a different gene. A large number of termination regions are known to and have been found to be satisfactory in a variety of hosts from the same and different genera and species. The termination region usually is selected more as a matter of convenience rather than because of any particular property. Preferably, the termination region is derived from a yeast gene, particularly *Saccharomyces, Schizosaccharomyces, Candida* or *Kluyveromyces*. The 3' regions of two mammalian genes, γ interferon and α2 interferon, are also known to function in yeast.

### INTRODUCTION OF CONSTRUCTS INTO HOST CELLS

Constructs comprising the gene of interest may be introduced into a host cell by standard techniques. These techniques include transformation, protoplast fusion, lipofection, transfection, transduction, conjugation, infection, bolistic impact, electroporation, microinjection, scraping, or any other method which introduces the gene of interest into the host cell. Methods of transformation which are used include lithium acetate transformation (*Methods in Enzymology*, Vol. 194, p. 186-187, 1991). For convenience, a host cell which has been manipulated by any method to take up a DNA sequence or construct will be referred to as "transformed" or "recombinant" herein.

The subject host will have at least have one copy of the expression construct and may have two or more, depending upon whether the gene is integrated into the genome, amplified, or is present on an extrachromosomal element having multiple copy numbers. Where the subject host is a yeast, four principal types of yeast plasmid vectors can be used: Yeast Integrating plasmids (YIps), Yeast Replicating plasmids (YRps), Yeast Centromere plasmids (YCps), and Yeast Episomal plasmids (YEps). YIps lack a yeast replication origin and must be propagated as integrated elements in the yeast genome. YRps have a chromosomally derived autonomously replicating sequence and are propagated as medium copy number (20 to 40), autonomously replicating, unstably segregating plasmids. YCps have both a replication origin and a centromere sequence and propagate as low copy number (10-20), autonomously replicating, stably segregating plasmids. YEps have an origin of replication from the yeast 2µm plasmid and are propagated as high copy number, autonomously replicating, irregularly segregating plasmids. The presence of the plasmids in yeast can be ensured by maintaining selection for a marker on the plasmid. Of particular interest are the yeast vectors pYES2 (a YEp plasmid available from Invitrogen, confers uracil prototrophy and a GAL1 galactose-inducible promoter for expression), pRS425-pG1 (a YEp plasmid obtained from Dr. T. H. Chang, Ass. Professor of Molecular Genetics, Ohio State University, containing a constitutive GPD promoter and conferring leucine prototrophy), and pYX424 (a YEp plasmid having a constitutive TP1 promoter and conferring leucine prototrophy; Alber, T. and Kawasaki, G. (1982). *J. Mol. & Appl*. *Genetics* 1: 419).

The transformed host cell can be identified by selection for a marker contained on the introduced construct. Alternatively, a separate marker construct may be introduced with the desired construct, as many transformation techniques introduce many DNA molecules into host cells. Typically, transformed hosts are selected for their ability to grow on selective media. Selective media may incorporate an antibiotic or lack a factor necessary for growth of the untransformed host, such as a nutrient or growth factor. An introduced marker gene therefor may confer antibiotic resistance, or encode an essential growth factor or enzyme, and permit growth on selective media when expressed in the transformed host Selection of a transformed host also can occur when the expressed marker protein can be detected, either directly or indirectly. The marker protein may be expressed alone or as a fusion to another protein. The marker protein can be detected by its enzymatic activity; for example β galactosidase can convert the substrate X-gal to a colored product, and luciferase can convert luciferin to a light-emitting product. The marker protein can be detected by its light-producing or modifying characteristics; for example, the green fluorescent protein of *Aequorea victoria* fluoresces when illuminated with blue light. Antibodies can be used to detect the marker protein or a molecular tag on, for example, a protein of interest. Cells expressing the marker protein or tag can be selected, for example, visually, or by techniques such as FACS or panning using antibodies. For selection of yeast transformants, any marker that functions in yeast may be used. Desirably, resistance to kanamycin and the amino glycoside G418 are of interest, as well as ability to grow on media lacking uracil, leucine, lysine or tryptophan.

The Δ5-desaturase-mediated production of PUFAs can be performed in either prokaryotic or eukaryotic host cells. Prokaryotic cells of interest include *Eschericia*, *Bacillus, Lactobacillus, cyanobacteria* and the like. Eukaryotic cells include mammalian cells such as those of lactating animals, avian cells such as of chickens, and other cells amenable to genetic manipulation including insect, fungal, and algae cells. The cells may be cultured or formed as part or all of a host organism including an animal. Viruses and bacteriophage also may be used with the cells in the production of PUFAs, particularly for gene transfer, cellular targeting and selection. In a preferred embodiment, the host is any microorganism or animal which produces DGLA and/or can assimilate exogenously supplied DGLA, and preferably produces large amounts of DGLA. Examples of host animals include mice, rats, rabbits, chickens, quail, turkeys, bovines, sheep, pigs, goats, yaks, etc., which are amenable to genetic manipulation and cloning for rapid expansion of the transgene expressing population. For animals, a Δ5-desaturase transgene can be adapted for expression in target organelles, tissues and body fluids through modification of the gene regulatory regions. Of particular interest is the production of PUFAs in the breast milk of the host animal.

### Expression In Yeast

Examples of host microorganisms include *Saccharomyces cerevisiae, Saccharomyces carlsbergensis*, or other yeast such as *Candida, Kluyveromyces* or other fungi, for example, filamentous fungi such as *Aspergillus, Neurospora*, *Penicillium*, etc. Desirable characteristics of a host microorganism are, for example, that it is genetically well characterized, can be used for high level expression of the product using ultra-high density fermentation, and is on the GRAS (generally recognized as safe) list since the proposed end product is intended for ingestion by humans. Of particular interest is use of a yeast, more particularly baker's yeast (*S. cerevisiae*), as a cell host in the subject invention. Strains of particular interest are SC334 (Mat α pep4-3 prb1-1122 ura3-52 leu2-3, 112 reg1-501 gal1; *Gene* 83:57-64, 1989, Hovland *P. et al*.), YTC34 (α ade2-101 his3Δ200 lys2-801 ura3-52; obtained from Dr. T. H. Chang, Ass. Professor of Molecular Genetics, Ohio State University), YTC41 (a/α ura3-52/ura3=52 lys2-801/1ys2-801 ade2-101/ade2-101 trp1-Δ1/trp1-Δ1 his3Δ200/his3Δ200 leu2Δ1/leu2Δ1; obtained from Dr. T. H. Chang, Ass. Professor of Molecular Genetics, Ohio State University), BJ1995 (obtained from the Yeast Genetic Stock Centre, 1021 Donner Laboratory, Berkeley, CA 94720), INVSC1 (Mat α hiw3Δ1 leu2 trp1-289 ura3-52; obtained from Invitrogen, 1600 Faraday Ave., Carlsbad, CA 92008) and INVSC2 (Mat α his3Δ200 ura3-167; obtained from Invitrogen).

### Expression In Avian Species

For producing PUFAs in avian species and cells, such as chickens, turkeys, quail and ducks, gene transfer can be performed by introducing a nucleic acid sequence encoding a Δ5-desaturase into the cells following procedures known in the art. If a transgenic animal is desired, pluripotent stem cells of embryos can be provided with a vector carrying a Δ5-desaturase encoding transgene and developed into adult animal (USPN 5,162,215; Ono *et al*. (1996) *Comparative Biochemistry and Physiology A 113*(3):287-292; WO 9612793; WO 9606160). In most cases, the transgene will be modified to express high levels of the desaturase in order to increase production of PUFAs. The transgene can be modified, for example, by providing transcriptional and/or translational regulatory regions that function in avian cells, such as promoters which direct expression in particular tissues and egg parts such as yolk. The gene regulatory regions can be obtained from a variety of sources, including chicken anemia or avian leukosis viruses or avian genes such as a chicken ovalbumin gene.

### Expression In Insect Cells

Production of PUFAs in insect cells can be conducted using baculovirus expression vectors harboring a Δ5-desaturase transgene. Baculovirus expression vectors are available from several commercial sources such as Clonetech. Methods for producing hybrid and transgenic strains of algae, such as marine algae, which contain and express a desaturase transgene also are provided. For example, transgenic marine algae may be prepared as described in USPN 5,426,040. As with the other expression systems described above, the timing, extent of expression and activity of the desaturase transgene can be regulated by fitting the polypeptide coding sequence with the appropriate transcriptional and translational regulatory regions selected for a particular use. Of particular interest are promoter regions which can be induced under preselected growth conditions. For example, introduction of temperature sensitive and/or metabolite responsive mutations into the desaturase transgene coding sequences, its regulatory regions, and/or the genome of cells into which the transgene is introduced can be used for this purpose.

### Expression In Plants

Production of PUFA's in plants can be conducted using various plant transformation systems such as the use of *Agrobacterium tumefaciens*, plant viruses, particle cell transformation and the like which are disclosed in Applicant's related applications U.S. Application Serial Nos. 08/834,033 and 08/956,985 and continuation-in-part applications filed simultaneously with this application all of which are hereby incorporated by reference.

The transformed host cell is grown under appropriate conditions adapted for a desired end result. For host cells grown in culture, the conditions are typically optimized to produce the greatest or most economical yield of PUFAs, which relates to the selected desaturase activity. Media conditions which may be optimized include: carbon source, nitrogen source, addition of substrate, final concentration of added substrate, form of substrate added, aerobic or anaerobic growth, growth temperature, inducing agent, induction temperature, growth phase at induction, growth phase at harvest, pH, density, and maintenance of selection. Microorganisms such as yeast, for example, are preferably grown using selected media of interest, which include yeast peptone broth (YPD) and minimal media (contains amino acids, yeast nitrogen base, and ammonium sulfate, and lacks a component for selection, for example uracil). Desirably, substrates to be added are first dissolved in ethanol. Where necessary, expression of the polypeptide of interest may be induced, for example by including or adding galactose to induce expression from a GAL promoter.

### Expression In An Animal

Expression in cells of a host animal can likewise be accomplished in a transient or stable manner. Transient expression can be accomplished via known methods, for example infection or lipofection, and can be repeated in order to maintain desired expression levels of the introduced construct (*see* Ebert, PCT publication WO 94/05782). Stable expression can be accomplished via integration of a construct into the host genome, resulting in a transgenic animal. The construct can be introduced, for example, by microinjection of the construct into the pronuclei of a fertilized egg, or by transfection, retroviral infection or other techniques whereby the construct is introduced into a cell line which may form or be incorporated into an adult animal (U.S. Patent No. 4,873,191; U.S. Patent No. 5,530,177; U.S. Patent No. 5,565,362; U.S. Patent No. 5,366,894; Wilmut *et al*. (1997) *Nature* 385:810). The recombinant eggs or embryos are transferred to a surrogate mother (U.S. Patent No. 4,873,191; U.S. Patent No. 5,530,177; U.S. Patent No. 5,565,362; U.S. Patent No. 5,366,894; Wilmut *et al*. (supra)).

After birth, transgenic animals are identified, for example, by the presence of an introduced marker gene, such as for coat color, or by PCR or Southern blotting from a blood, milk or tissue sample to detect the introduced construct, or by an immunological or enzymological assay to detect the expressed protein or the products produced therefrom (U.S. Patent No. 4,873,191; U.S. Patent No. 5,530,177; U.S. Patent No. 5,565,362; U.S. Patent No. 5,366,894; Wilmut *et al*. (supra)). The resulting transgenic animals may be entirely transgenic or may be mosaics, having the transgenes in only a subset of their cells. The advent of mammalian cloning, accomplished by fusing a nucleated cell with an enucleated egg, followed by transfer into a surrogate mother, presents the possibility of rapid, large-scale production upon obtaining a "founder" animal or cell comprising the introduced construct; prior to this, it was necessary for the transgene to be present in the germ line of the animal for propagation (Wilmut *et al*. (supra)).

Expression in a host animal presents certain efficiencies, particularly where the host is a domesticated animal. For production of PUFAs in a fluid readily obtainable from the host animal, such as milk, the desaturase transgene can be expressed in mammary cells from a female host, and the PUFA content of the host cells altered. The desaturase transgene can be adapted for expression so that it is retained in the mammary cells, or secreted into milk, to form the PUFA reaction products localized to the milk (PCT publication WO 95/24488). Expression can be targeted for expression in mammary tissue using specific regulatory sequences, such as those of bovine α-lactalbumin, α-casein, β-casein, γ-casein, κ-casein, β-lactoglobulin, or whey acidic protein, and may optionally include one or more introns and/or secretory signal sequences (U.S. Patent No. 5,530,177; Rosen, U.S. Patent No. 5,565,362; Clark *et al*., U.S. Patent No. 5,366,894; Garner *et al*., PCT publication WO 95/23868). Expression of desaturase transgenes, or antisense desaturase transcripts, adapted in this manner can be used to alter the levels of specific PUFAs, or derivatives thereof, found in the animals milk. Additionally, the Δ5-desaturase transgene can be expressed either by itself or with other transgenes, in order to produce animal milk containing higher proportions of desired PUFAs or PUFA ratios and concentrations that resemble human breast milk (Prieto *et al*., PCT publication WO 95/24494).

### PURIFICATION OF FATTY ACIDS

The fatty acids desaturated in the Δ5 position may be found in the host microorganism or animal as free fatty acids or in conjugated forms such as acylglycerols, phospholipids, sulfolipids or glycolipids, and may be extracted from the host cell through a variety of means well-known in the art. Such means may include extraction with organic solvents, sonication, supercritical fluid extraction using for example carbon dioxide, and physical means such as presses, or combinations thereof. Of particular interest is extraction with methanol and chloroform. Where desirable, the aqueous layer can be acidified to protonate negatively charged moieties and thereby increase partitioning of desired products into the organic layer. After extraction, the organic solvents can be removed by evaporation under a stream of nitrogen. When isolated in conjugated forms, the products may be enzymatically or chemically cleaved to release the free fatty acid or a less complex conjugate of interest, and can then be subject to further manipulations to produce a desired end product. Desirably, conjugated forms of fatty acids are cleaved with potassium hydroxide.

If further purification is necessary, standard methods can be employed. Such methods may include extraction, treatment with urea, fractional crystallization, HPLC, fractional distillation, silica gel chromatography, high speed centrifugation or distillation, or combinations of these techniques. Protection of reactive groups, such as the acid or alkenyl groups, may be done at any step through known techniques, for example alkylation or iodination. Methods used include methylation of the fatty acids to produce methyl esters. Similarly, protecting groups may be removed at any step. Desirably, purification of fractions containing ARA, DHA and EPA may be accomplished by treatment with urea and/or fractional distillation.

### USES OF FATTY ACIDS

There are several uses for fatty acids produced by the subject invention. Probes based on the DNAs of the present invention may find use in methods for isolating related molecules or in methods to detect organisms expressing desaturases. When used as probes, the DNAs or oligonucleotides must be detectable. This is usually accomplished by attaching a label either at an internal site, for example via incorporation of a modified residue, or at the 5' or 3' terminus. Such labels can be directly detectable, can bind to a secondary molecule that is detectably labeled, or can bind to an unlabelled secondary molecule and a detectably labeled tertiary molecule; this process can be extended as long as is practical to achieve a satisfactorily detectable signal without unacceptable levels of background signal. Secondary, tertiary, or bridging systems can include use of antibodies directed against any other molecule, including labels or other antibodies, or can involve any molecules which bind to each other, for example a biotin-streptavidin/avidin system. Detectable labels typically include radioactive isotopes, molecules which chemically or enzymatically produce or alter light, enzymes which produce detectable reaction products, magnetic molecules, fluorescent molecules or molecules whose fluorescence or light-emitting characteristics change upon binding. Examples of labelling methods can be found in USPN 5,011,770. Alternatively, the binding of target molecules can be directly detected by measuring the change in heat of solution on binding of probe to target via isothermal titration calorimetry, or by coating the probe or target on a surface and detecting the change in scattering of light from the surface produced by binding of target or probe, respectively, as may be done with the BIAcore system.

PUFAs produced by recombinant means find applications in a wide variety of areas. Supplementation of humans or animals with PUFAs in various forms can result in increased levels not only of the added PUFAs, but of their metabolic progeny as well. For example, where the inherent Δ5-desaturase pathway is dysfunctional in an individual, treatment with ARA can result not only in increased levels of ARA, but also of downstream products of ARA such as prostaglandins (see Figure 1). Complex regulatory mechanisms can make it desirable to combine various PUFAs, or to add different conjugates of PUFAs, in order to prevent, control or overcome such mechanisms to achieve the desired levels of specific PUFAs in an individual.

### NUTRITIONAL COMPOSITIONS

Methods of the present invention may provide nutritional compositions. Such compositions, for purposes of the present invention, include any food or preparation for human consumption including for enteral or parenteral consumption, which when taken into the body (a) serve to nourish or build up tissues or supply energy and/or (b) maintain, restore or support adequate nutritional status or metabolic function.

The nutritional composition comprises at least one oil or acid produced in accordance with the present invention and may either be in a solid or liquid form. Additionally, the composition may include edible macronutrients, vitamins and minerals in amounts desired for a particular use. The amount of such ingredients will vary depending on whether the composition is intended for use with normal, healthy infants, children or adults having specialized needs such as those which accompany certain metabolic conditions (e.g., metabolic disorders).

Examples of macronutrients which may be added to the composition include but are not limited to edible fats, carbohydrates and proteins. Examples of such edible fats include but are not limited to coconut oil, soy oil, and mono-and diglycerides. Examples of such carbohydrates include but are not limited to glucose, edible lactose and hydrolyzed search. Additionally, examples of proteins which may be utilized in the nutritional composition of the invention include but are not limited to soy proteins, electrodialysed whey , electrodialysed skim milk, milk whey, or the hydrolysates of these proteins.

With respect to vitamins and minerals, the following may be added to the nutritional compositions of the present invention: calcium, phosphorus, potassium, sodium, chloride, magnesium, manganese, iron, copper, zinc, selenium, iodine, and Vitamins A, E, D, C, and the B complex. Other such vitamins and minerals may also be added.

The components utilized in the nutritional compositions will be of semi-purified or purified origin. By semi-purified or purified is meant a material which has been prepared by purification of a natural material or by synthesis.

Examples of nutritional compositions of the present invention include but are not limited to infant formulas, dietary supplements, and rehydration compositions. Nutritional compositions of particular interest include but are not limited to those utilized for enteral and parenteral supplementation for infants, specialist infant formulae, supplements for the elderly, and supplements for those with gastrointestinal difficulties and/or malabsorption.

### Nutritional Compositions

A typical nutritional composition produced by the invention will contain edible macronutrients, vitamins and minerals in amounts desired for a particular use. The amounts of such ingredients will vary depending on whether the formulation is intended for use with normal, healthy individuals temporarily exposed to stress, or to subjects having specialized needs due to certain chronic or acute disease states (e.g., metabolic disorders). It will be understood by persons skilled in the art that the components utilized in the nutritional formulation are of semi-purified or purified origin. By semi-purified or purified is meant a material that has been prepared by purification of a natural material or by synthesis. These techniques are well known in the art (See, e.g., Code of Federal Regulations for Food Ingredients and Food Processing; Recommended Dietary Allowances, 10^{th} Ed., National Academy Press, Washington, D.C., 1989).

The nutritional formulation may be an enteral nutritional product; more preferably an adult or child enteral nutritional product. The formula may comprise, in addition to the PUFAs of the invention; macronutrients, vitamins and minerals in amounts designed to provide the daily nutritional requirements of adults.

The macronutritional components include edible fats, carbohydrates and proteins. Exemplary edible fats are coconut oil, soy oil, and mono- and diglycerides and the PUFA oils produced by the invention. Exemplary carbohydrates are glucose, edible lactose and hydrolyzed cornstarch. A typical protein source would be soy protein, electrodialysed whey or electrodialysed skim milk or milk whey, or the hydrolysates of these proteins, although other protein sources are also available and may be used. These macronutrients would be added in the form of commonly accepted nutritional compounds in amount equivalent to those present in human milk or an energy basis, i.e., on a per calorie basis.

Methods for formulating liquid and enteral nutritional formulas are well known in the art and are described in detail in the examples.

The enteral formula can be sterilized and subsequently utilized on a ready-to-feed (RTF) basis or stored in a concentrated liquid or a powder. The powder can be prepared by spray drying the enteral formula prepared as indicated above, and the formula can be reconstituted by rehydrating the concentrate. Adult and infant nutritional formulas are well known in the art and commercially available (e.g., Similac®, Ensure®, Jevity® and Alimentum® from Ross Products Division, Abbott Laboratories). An oil or acid of the present invention can be added to any of these formulas in the amounts described below.

The energy density of the nutritional composition when in liquid form, can typically range from about 0.6 Kcal to 3.0 Kcal per ml. When in solid or powdered form, the nutritional supplement can contain from about 1.2 to more than 9 Kcals per gm, preferably 3 to 7 Kcals per gram. In general, the osmolality of a liquid product should be less than 700 mOsm and more preferably less than 660 mOsm.

The nutritional formula would typically include vitamins and minerals, in addition to the PUFAs of the invention, in order to help the individual ingest the minimum daily requirements for these substances. In addition to the PUFAs listed above, it may also be desirable to supplement the nutritional composition with zinc, copper, and folic acid in addition to antioxidants. It is believed that these substances will also provide a boost to the stressed immune system and thus will provide further benefits to the individual. The presence of zinc, copper or folic acid is optional and is not required in order to gain the beneficial effects on immune suppression. Likewise a pharmaceutical composition can be supplemented with these same substances as well.

In a more preferred embodiment, the nutritional contains, in addition to the antioxidant system and the PUFA component, a source of carbohydrate wherein at least 5 weight % of said carbohydrate is an indigestible oligosaccharide. In yet a more preferred embodiment, the nutritional composition additionally contains protein, taurine and carnitine.

The PUFAs, or derivatives thereof, made by the disclosed method can be used as dietary substitutes, or supplements, particularly infant formulas, for patients undergoing intravenous feeding or for preventing or treating malnutrition. Typically, human breast milk has a fatty acid profile comprising from about 0.15 % to about 0.36 % as DHA, from about 0.03 % to about 0.13 % as EPA, from about 0.30 % to about 0.88 % as ARA, from about 0.22 % to about 0.67 % as DGLA, and from about 0.27 % to about 1.04 % as GLA. Additionally, the predominant triglyceride in human milk has been reported to be 1,3-di-oleoyl-2-palmitoyl, with 2-palmitoyl glycerides reported as better absorbed than 2-oleoyl or 2-lineoyl glycerides (USPN 4,876,107). Thus, fatty acids such as ARA, DGLA, GLA and/or EPA produced by the invention can be used to alter the composition of infant formulas to better replicate the PUFA composition of human breast milk. In particular, an oil composition for use in a pharmacologic or food supplement, particularly a breast milk substitute or supplement, will preferably comprise one or more of ARA, DGLA and GLA. More preferably the oil will comprise from about 0.3 to 30% ARA, from about 0.2 to 30% DGLA, and from about 0.2 to about 30% GLA.

In addition to the concentration, the ratios of ARA, DGLA and GLA can be adapted for a particular given end use. When formulated as a breast milk supplement, or substitute an oil composition which contains two or more of ARA, DGLA and GLA will be provided in a ratio of about 1:19:30 to about 6:1:0.2, respectively. For example, the breast milk of animals can vary in ratios of ARA:DGLA:DGL ranging from 1:19:30 to 6:1:0.2, which includes intermediate ratios which are preferably about 1:1:1, 1:2:1, 1:1:4. When produced together in a host cell, adjusting the rate and percent of conversion of a precursor substrate such as GLA and DGLA to ARA can be used to precisely control the PUFA ratios. For example, a 5% to 10% conversion rate of DGLA to ARA can be used to produce an ARA to DGLA ratio of about 1:19, whereas a conversion rate of about 75% to 80% can be used to produce an ARA to DGLA ratio of about 6:1. Therefore, whether in a cell culture system or in a host animal, regulating the timing, extent and specificity of desaturase expression as described can be used to modulate the PUFA levels and ratios. Depending on the expression system used, e.g., cell culture or an animal expressing oil(s) in its milk, the oils also can be isolated and recombined in the desired concentrations and ratios. Amounts of oils providing these ratios of PUFA can be determined following standard protocols. PUFAs, or host cells containing them, also can be used as animal food supplements to alter an animal's tissue or milk fatty acid composition to one more desirable for human or animal consumption.

For dietary supplementation, the purified PUFAs, or derivatives thereof, may be incorporated into cooking oils, fats or margarines formulated so that in normal use the recipient would receive the desired amount. The PUFAs may also be incorporated into infant formulas, nutritional supplements or other food products, and may find use as anti-inflammatory or cholesterol lowering agents.

### Pharmaceutical Compositions

A pharmaceutical composition comprising one or more of the acids and/or resulting oils may be produced in accordance with the methods described herein. More specifically, such a pharmaceutical composition may comprise one or more of the acids and/or oils as well as a standard, well-known, non-toxic pharmaceutically acceptable carrier, adjuvant or vehicle such as, for example, phosphate buffered saline, water, ethanol, polyols, vegetable oils, a wetting agent or an emulsion such as a water/oil emulsion. The composition may be in either a liquid or solid form. For example, the composition may be in the form of a tablet, capsule, ingestible liquid or powder, injectible, or topical ointment or cream.

Possible routes of administration include, for example, oral, rectal and parenteral. The route of administration will, of course, depend upon the desired effect. For example, if the composition is being utilized to treat rough, dry, or aging skin, to treat injured or burned skin, or to treat skin or hair affected by a disease or condition, it may perhaps be applied topically.

The dosage of the composition to be administered to the patient may be determined by one of ordinary skill in the art and depends upon various factors such as weight of the patient, age of the patient, immune status of the patient, etc.

With respect to form, the composition may be, for example, a solution, a dispersion, a suspension, an emulsion or a sterile powder which is then reconstituted.

Additionally, the composition may be utilized for cosmetic purposes. It may be added to pre-existing cosmetic compositions such that a mixture is formed or may be used as a sole composition.

Pharmaceutical compositions may be utilized to administer the PUFA component to an individual. Suitable pharmaceutical compositions may comprise physiologically acceptable sterile aqueous or non-aqueous solutions, dispersions, suspensions or emulsions and sterile powders for reconstitution into sterile solutions or dispersions for ingestion. Examples of suitable aqueous and non-aqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the maintenance of the required particle size in the case of dispersions and by the use of surfactants. It may also be desirable to include isotonic agents, for example sugars, sodium chloride and the like. Besides such inert diluents, the composition can also include adjuvants, such as wetting agents, emulsifying and suspending agents, sweetening, flavoring and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth or mixtures of these substances, and the like.

Solid dosage forms such as tablets and capsules can be prepared using techniques well known in the art. For example, PUFAs of the invention can be tableted with conventional tablet bases such as lactose, sucrose, and cornstarch in combination with binders such as acacia, cornstarch or gelatin, disintegrating agents such as potato starch or alginic acid and a lubricant such as stearic acid or magnesium stearate. Capsules can be prepared by incorporating these excipients into a gelatin capsule along with the antioxidants and the PUFA component. The amount of the antioxidants and PUPA component that should be incorporated into the pharmaceutical formulation should fit within the guidelines discussed above.

As used in this application, the term "treat" refers to either preventing, or reducing the incidence of, the undesired occurrence. For example, to treat immune suppression refers to either preventing the occurrence of this suppression or reducing the amount of such suppression. The terms "patient" and "individual" are being used interchangeably and both refer to an animal. The term "animal" as used in this application refers to any warm-blooded mammal including, but not limited to, dogs, humans, monkeys, and apes. As used in the application the term "about" refers to an amount varying from the stated range or number by a reasonable amount depending upon the context of use. Any numerical number or range specified in the specification should be considered to be modified by the term about.

"Dose" and "serving" are used interchangeably and refer to the amount of the nutritional or pharmaceutical composition ingested by the patient in a single setting and designed to deliver effective amounts of the antioxidants and the structured triglyceride. As will be readily apparent to those skilled in the art, a single dose or serving of the liquid nutritional powder should supply the amount of antioxidants and PUFAs discussed above. The amount of the dose or serving should be a volume that a typical adult can consume in one sitting. This amount can vary widely depending upon the age, weight, sex or medical condition of the patient. However as a general guideline, a single serving or dose of a liquid nutritional produce should be considered as encompassing a volume from 100 to 600 ml, more preferably from 125 to 500 ml and most preferably from 125 to 300 ml.

The PUFAs may also be added to food even when supplementation of the diet is not required. For example, the composition may be added to food of any type including but not limited to margarines, modified butters, cheeses, milk, yogurt, chocolate, candy, snacks, salad oils, cooking oils, cooking fats, meats, fish and beverages.

### Pharmaceutical Applications

For pharmaceutical use (human or veterinary), the compositions are generally administered orally but can be administered by any route by which they may be successfully absorbed, e.g., parenterally (i.e. subcutaneously, intramuscularly or intravenously), rectally or vaginally or topically, for example, as a skin ointment or lotion. The PUFAs produced by methods of the present invention may be administered alone or in combination with a pharmaceutically acceptable carrier or excipient. Where available, gelatin capsules are the preferred form of oral administration. Dietary supplementation as set forth above also can provide an oral route of administration. The unsaturated acids of the present invention may be administered in conjugated forms, or as salts, esters, amides or prodrugs of the fatty acids. Preferred pharmaceutically acceptable salts are the sodium, potassium or lithium salts. Also encompassed are the N-alkylpolyhydroxamine salts, such as N-methyl glucamine, found in PCT publication WO 96/33155. The preferred esters are the ethyl esters. As solid salts, the PUFAs also can be administered in tablet form. For intravenous administration, the PUFAs or derivatives thereof may be incorporated into commercial formulations such as Intralipids. The typical normal adult plasma fatty acid profile comprises 6.64 to 9.46% of ARA, 1.45 to 3.11% of DGLA, and 0.02 to 0.08% of GLA. These PUFAs or their metabolic precursors can be administered, either alone or in mixtures with other PUFAs, to achieve a normal fatty acid profile in a patient. Where desired, the individual components of formulations may be individually provided in kit form, for single or multiple use. A typical dosage of a particular fatty acid is from 0.1 mg to 20 g, or even 100 g daily, and is preferably from 10 mg to 1, 2, 5 or 10 g daily as required, or molar equivalent amounts of derivative forms thereof. Parenteral nutrition compositions produced by methods of the invention may comprise from about 2 to about 30 weight percent fatty acids calculated as triglycerides ; preferred is a composition having from about 1 to about 25 weight percent of the total PUFA composition as GLA (USPN 5,196,198). Other vitamins, and particularly fat-soluble vitamins such as vitamin A, D, E and L-carnitine can optionally be included. Where desired, a preservative such as α tocopherol may be added, typically at about 0.1% by weight.

Suitable pharmaceutical compositions may comprise physiologically acceptable sterile aqueous or non-aqueous solutions, dispersions, suspensions or emulsions and sterile powders for reconstitution into sterile injectible solutions or dispersions. Examples of suitable aqueous and non-aqeuous carriers, diluents, solvents or vehicles include water, ethanol, polyols (propylleneglyol, polyethylenegycol, glycerol, and the like), suitable mixtures thereof, vegetable oils (such as olive oil) and injectable organic esters such as ehyl oleate. Proper fluidity can be maintained, for example, by the maintenance of the required particle size in the case of dispersions and by the use of surfactants. It may also be desirable to include isotonic agents, for example sugars, sodium chloride and the like. Besides such inert diluents, the composition can also include adjuvants, such as wetting agents, emulsifying and suspending agents, sweetening, flavoring and perfuming agents.

Suspensions in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances and the like.

An especially preferred pharmaceutical composition contains diacetyltartaric acid esters of mono- and diglycerides dissolved in an aqueous medium or solvent. Diacetyltartaric acid esters of mono- and diglycerides have an HLB value of about 9-12 and are significantly more hydrophilic than existing antimicrobial lipids that have HLB values of 2-4. Those existing hydrophobic lipids cannot be formulated into aqueous compositions. As disclosed herein, those lipids can now be solubilized into aqueous media in combination with diacetyltartaric acid esters of mono-and diglycerides. In accordance with this embodiment, diacetyltartaric acid esters of mono- and diglycerides (e.g., DATEM-C12:0) is melted with other active antimicrobial lipids (e.g., 18:2 and 12:0 monoglycerides) and mixed to obtain a homogeneous mixture. Homogeneity allows for increased antimicrobial activity. The mixture can be completely dispersed in water. This is not possible without the addition of diacetyltartaric acid esters of mono- and diglycerides and premixing with other monoglycerides prior to introduction into water. The aqueous composition can then be admixed under sterile conditions with physiologically acceptable diluents, preservatives, buffers or propellants as may be required to form a spray or inhalant.

Supplementation with PUFAs produced using methods of the present invention can be used to treat restenosis after angioplasty. Symptoms of inflammation, rheumatoid arthritis, and asthma and psoriasis can be treated with the PUFAs of the present invention. Evidence indicates that PUFAs may be involved in calcium metabolism, suggesting that PUFAs of the present invention may be used in the treatment or prevention of osteoporosis and of kidney or urinary tract stones.

The PUFAs can be used in the treatment of cancer. Malignant cells have been shown to have altered fatty acid compositions; addition of fatty acids has been shown to slow their growth and cause cell death, and to increase their susceptibility to chemotherapeutic agents. GLA has been shown to cause reexpression on cancer cells of the E-cadherin cellular adhesion molecules, loss of which is associated with aggressive metastasis. Clinical testing of intravenous administration of the water soluble lithium salt of GLA to pancreatic cancer patients produced statistically significant increases in their survival. PUFA supplementation may also be useful for treating cachexia associated with cancer.

The PUFAs can also be used to treat diabetes (USPN 4,826,877; Horrobin *et al*., Am. J. Clin. Nutr. Vol. 57 (Suppl.), 732S-737S). Altered fatty acid metabolism and composition has been demonstrated in diabetic animals. These alterations have been suggested to be involved in some of the long-term complications resulting from diabetes, including retinopathy, neuropathy, nephropathy and reproductive system damage. Primrose oil, which contains GLA, has been shown to prevent and reverse diabetic nerve damage.

The PUFAs can be used to treat eczema, reduce blood pressure and improve math scores. Essential fatty acid deficiency has been suggested as being involved in eczema, and studies have shown beneficial effects on eczema from treatment with GLA. GLA has also been shown to reduce increases in blood pressure associated with stress, and to improve performance on arithmetic tests. GLA and DGLA have been shown to inhibit platelet aggregation, cause vasodilation, lower cholesterol levels and inhibit proliferation of vessel wall smooth muscle and fibrous tissue (Brenner *et al*., Adv. Exp. Med. Biol. Vol. 83, p. 85-101, 1976). Administration of GLA or DGLA, alone or in combination with EPA, has been shown to reduce or prevent gastro-intestinal bleeding and other side effects caused by non-steroidal anti-inflammatory drugs (USPN 4,666,701). GLA and DGLA have also been shown to prevent or treat endometriosis and premenstrual syndrome (USPN 4,758,592) and to treat myalgic encephalomyelitis and chronic fatigue after viral infections (USPN 5,116,871).

Further uses of the PUFAs produced by methods of this invention include use in treatment of AIDS, multiple schlerosis, acute respiratory syndrome, hypertension and inflammatory skin disorders. The PUFAs also can be used for formulas for general health as well as for geriatric treatments.

### Veterinary Applications

It should be noted that the above-described pharmaceutical and nutritional compositions may be utilized in connection with animals, as well as humans, as animals experience many of the same needs and conditions as human. For example, the oil or acids of the present invention may be utilized in animal feed supplements.

The following examples are presented by way of illustration, not of limitation.

### Examples

- Example 1: Isolation of a Δ5-desaturase Nucleotide Sequence from *Mortierella alpina*
- Example 2: Expression of *M. alpina* Δ5-desaturase Clones in Baker's Yeast
- Example 3: Initial Optimization of Culture Conditions
- Example 4: Distribution of PUFAs in Yeast Lipid Fractions
- Example 5: Further Culture Optimization
- Example 6: Identification of Homologues to *M*. *alpina* Δ5 and Δ6 desaturases
- Example 7: Identification of *M. alpina* Δ5 and Δ6 homologues in other PUFA-producing organisms
- Example 8: Identification of *M. alpina* Δ5 and Δ6 homologues in other PUFA-producing organisms
- Example 9: Human Desaturase Sequences
- Example 10: Nutritional Compositions

### Example 1

### Isolation of a Δ5-desaturase Nucleotide Sequence from Mortierella alpina

*Motierella alpina* produces arachidonic acid (ARA, 20:4) from the precursor 20:3 by a Δ5-desaturase. A nucleotide sequence encoding the Δ5-desaturase from *Mortierella alpina* was obtained through PCR amplification using *M. alpina* 1^{st} strand cDNA and degenerate oligonucleotide primers corresponding to amino acid sequences conserved between Δ6-desaturases from *Synechocystis* and *Spirulina*. The procedure used was as follows:

Total RNA was isolated from a 3 day old PUFA-producing culture of *Mortierella alpina* using the protocol of Hoge *et al*. (1982) *Experimental Mycology* 6:225-232. The RNA was used to prepare double-stranded cDNA using BRL's lambda-ZipLox system, following the manufacturer's instructions. Several size fractions of the *M*. *alpina* cDNA were packaged separately to yield libraries with different average-sized inserts. The "full-length" library contains approximately 3 x 10⁶ clones with an average insert size of 1.77 kb. The "sequencing-grade" library contains approximately 6 x 10⁵ clones with an average insert size of 1.1 kb.

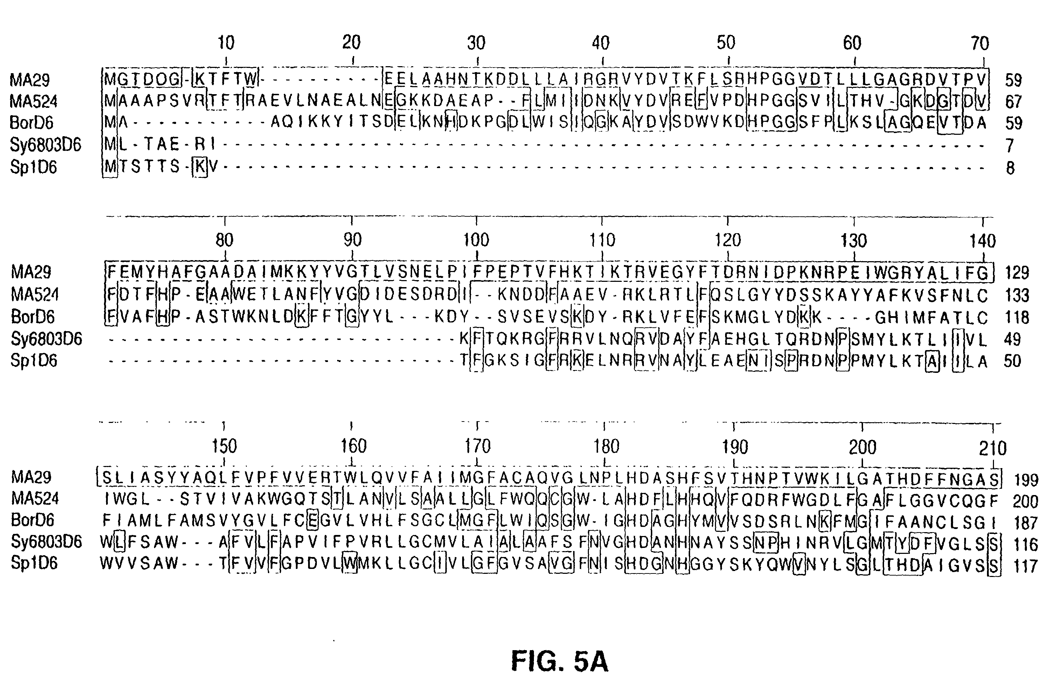

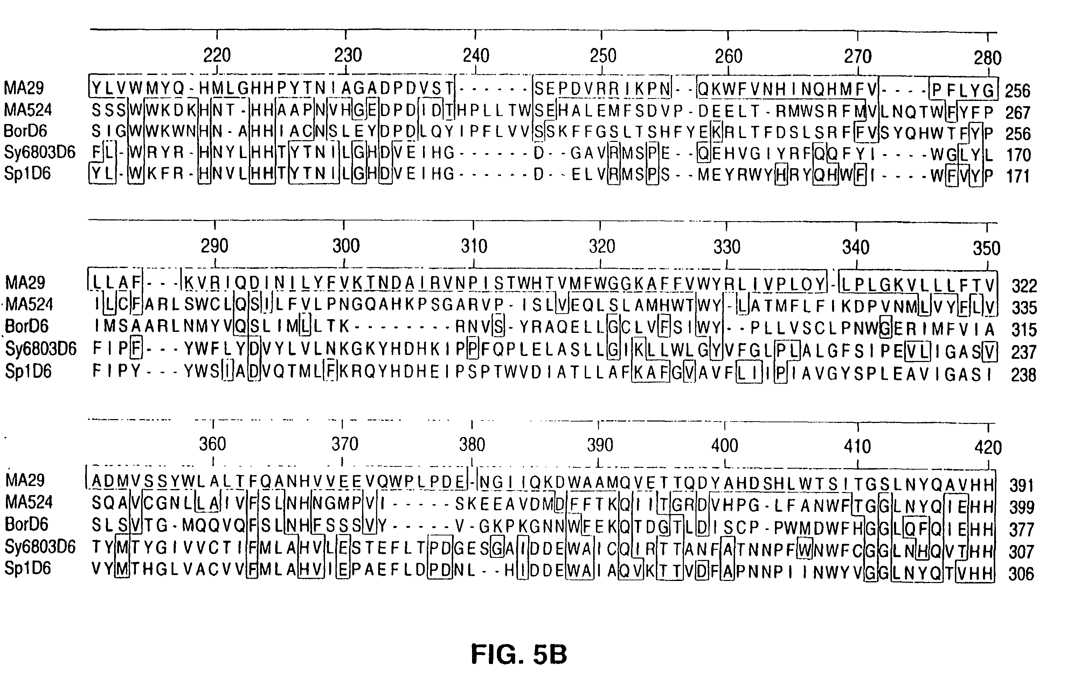

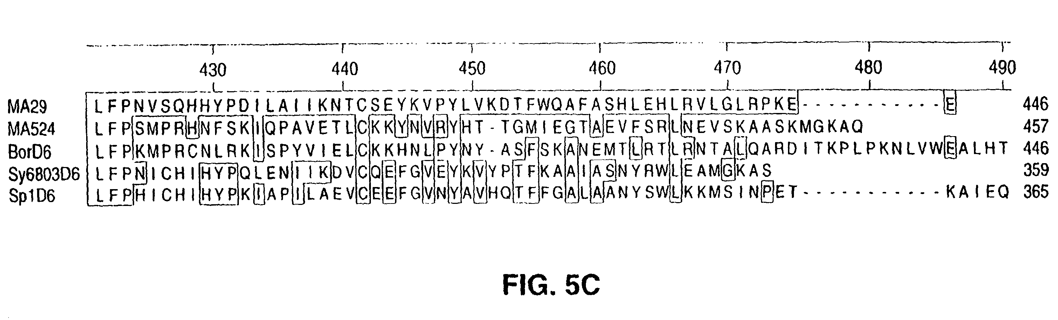

5µg of total RNA was reverse transcribed using BRL Superscript RTase and the primer TSyn (5'-CCAAGCTTCTGCAGGAGCTCTTTTTTT TTTTTTTT-3'), SEQ ID NO:10. Degenerate oligonucleotides were designed to regions conserved between the two cyanobacterial Δ6-desaturase sequences. The specific primers used were D6DESAT-F3 (SEQ ID NO:8) (5'-CUACUACUACUACAYCAYACOTAYACOAAYAT-3') and D6DESAT-R3 (SEQ ID NO:9) (5'-CAUCAUCAUCAUOGGRAAOARRTGRTG-3'), where Y=C+T, R=A+G, and O=I+C. PCR amplification was carried out in a 25µl volume containing: template derived from 40 ng total RNA, 2 pM each primer, 200 µM each deoxyribonucleotide triphosphate, 60 mM Tris-Cl, pH 8.5, 15 mM (NH₄)₂SO₄, 2 mM MgCl₂. Samples were subjected to an initial denaturation step of 95 degrees (all temperatures Celsius) for 5 minutes, then held at 72 degrees while 0.2 U of Taq polymerase were added. PCR thermocycling conditions were as follows: 94 degrees for 1 min., 45 degrees for 1.5 min., 72 degrees for 2 min. PCR was continued for 35 cycles. PCR using these primers on the *M. alpina* first-strand cDNA produced a 550 bp reaction product. Comparison of the deduced amino acid sequence of the *M. alpina* PCR fragment SEQ ID NO:3 revealed regions of homology with Δ6-desaturases (*see* Figure 5). However, there was only about 28% identity over the region compared.

The PCR product was used as a probe to isolate corresponding cDNA clones from a *M*. *alpina* library. The longest cDNA clone, Ma29, was designated pCGN5521 and has been completely sequenced on both strands. The cDNA is contained as a 1481 bp insert in the vector pZL1 (Bethesda Research Laboratories) and, beginning with the first ATG, contains an open reading frame encoding 446 amino acids. The reading frame contains the sequence deduced from the PCR fragment. The sequence of the cDNA insert was found to contain regions of homology to Δ6-desaturases (*see* Figure 5). For example, three conserved "histidine boxes" (that have been observed in membrane-bound desaturases (Okuley *et al*., (1994) *The Plant Cell 6*:147-158)) were found to be present in the *Mortierella* sequence at amino acid positions 171-175,207-212, and 387-391 (*see* Figure 3). However, the typical "HXXHH" amino acid motif for the third histidine box for the *Mortierella* desaturase was found to be QXXHH, SEQ ID NO:11-12. Surprisingly, the amino-terminus of the encoded protein, showed significant homology to cytochrome b5 proteins. Thus, the *Mortierella* cDNA clone appears to represent a fusion between a cytochrome b5 and a fatty acid desaturase. Since cytochrome b5 is believed to function as the electron donor for membrane-bound desaturase enzymes, it is possible that the N-terminal cytochrome b5 domain of this desaturase protein is involved in its function. This may be advantageous when expressing the desaturase in heterologous systems for PUFA production.

### Example 2

### Expression of M. alpina Desaturase Clones in Baker's Yeast

### Yeast Transformation

Lithium acetate transformation of yeast was performed according to standard protocols (*Methods in Enzymology,* Vol. 194, p. 186-187, 1991). Briefly, yeast were grown in YPD at 30°C. Cells were spun down, resuspended in TE, spun down again, resuspended in TE containing 100 mM lithium acetate, spun down again, and resuspended in TE/lithium acetate. The resuspended yeast were incubated at 30°C for 60 minutes with shaking. Carrier DNA was added, and the yeast were aliquoted into tubes. Transforming DNA was added, and the tubes were incubated for 30 min. at 30°C. PEG solution (35% (w/v) PEG 4000, 100 mM lithium acetate, TE pH7.5) was added followed by a 50 min. incubation at 30°C. A 5 min. heat shock at 42°C was performed, the cells were pelleted, washed with TE, pelleted again and resuspended in TE. The resuspended cells were then plated on selective media.

### Desaturase Expression in Transformed Yeast

The cDNA clones from *Mortierella alpina* were screened for desaturase activity in baker's yeast. A canola Δ15-desaturase (obtained by PCR using 1^{st} strand cDNA from *Brassica napus* cultivar 212/86 seeds using primers based on the published sequence (Arondel *et al. Science* 258:1353-1355)) was used as a positive control. The Δ15-desaturase gene and the gene from cDNA clone Ma29 was inserted into the expression vector pYES2 (Invitrogen), resulting in plasmids pCGR-2 and pCGR-4, respectively. These plasmids were transfected into *S. cerevisiae* yeast strain 334 and expressed after induction with galactose and in the presence of substrates that allowed detection of specific desaturase activity. The control strain was *S. cerevisiae* strain 334 containing the unaltered pYES2 vector. The substrates used, the products produced and the indicated desaturase activity were: DGLA (conversion to ARA would indicate Δ5-desaturase activity), linolenic acid (conversion to GLA would indicate Δ6-desaturase activity; conversion to ALA would indicate Δ15-desaturase activity), oleic acid (an endogenous substrate made by *S. cerevisiae*, conversion to linolenic acid would indicate Δ12-desaturase activity, which *S. cerevisiae* lacks), or ARA (conversion to EPA would indicate Δ17-desaturase activity). The results are provided in Table 1 below. The lipid fractions were extracted as follows: Cultures were grown for 48-52 hours at 15°C. Cells were pelleted by centrifugation, washed once with sterile ddH₂O, and repelleted. Pellets were vortexed with methanol; chloroform was added along with tritridecanoin (as an internal standard). The mixtures were incubated for at least one hour at room temperature or at 4°C overnight. The chloroform layer was extracted and filtered through a Whatman filter with one gram of anhydrous sodium sulfate to remove particulates and residual water. The organic solvents were evaporated at 40°C under a stream of nitrogen. The extracted lipids were then derivatized to fatty acid methyl esters (FAME) for gas chromatography analysis (GC) by adding 2 ml of 0.5 N potassium hydroxide in methanol to a closed tube. The samples were heated to 95°C to 100°C for 30 minutes and cooled to room temperature. Approximately 2 ml of 14 % boron trifluoride in methanol was added and the heating repeated. After the extracted lipid mixture cooled, 2 ml of water and 1 ml of hexane were added to extract the FAME for analysis by GC. The percent conversion was calculated by dividing the product produced by the sum of (the product produced and the substrate added) and then multiplying by 100. To calculate the oleic acid percent conversion, as no substrate was added, the total linolenic acid produced was divided by the sum of (oleic acid and linolenic acid produced), then multiplying by 100.

**Table 1**

| ***M*. *alpina Desaturase Expression in Baker's Yeast*** | | |
|---|---|---|
| CLONE | TYPE OF ENZYME ACTIVITY | % CONVERSION OF SUBSTRATE |
| pCGR-2 (canola Δ15 desaturase) | Δ6 | 0 (18:2 to 18:3ω6) |
| | Δ15 | 16.3 (18:2 to 18:3ω3) |
| | Δ5 | 2.0 (20:3 to 20:4ω6) |
| | Δ17 | 2.8 (20:4 to 20:5ω3) |
| | Δ12 | 1.8 (18:1 to 18:2ω6) |
| | | |
| pCGR-4 (*M*. *alpina* Ma29) | Δ6 | 0 |
| | Δ15 | 0 |
| | Δ5 | 15.3 |
| | Δ17 | 0.3 |
| | Δ12 | 3.3 |

The Δ15-desaturase control clone exhibited 16.3% conversion of the substrate. The pCGR-4 clone expressing the Ma29 cDNA converted 15.3% of the 20:3 substrate to 20:4ω6, indicating that the gene encodes a Δ5-desaturase. The background (non-specific conversion of substrate) was between 0-3% in these cases. We also found substrate inhibition of the activity by using different concentrations of the substrate. When substrate was added to 100 µM, the percent conversion to product dropped compared to when substrate was added to 25 µM (see below). Additionally, by varying the DGLA substrate concentrations, between about 5 µM to about 200 µM percent conversion of DGLA to ARA ranged from about 5% to 75% with the *M*. *alpina* Δ5-desaturase.

These data show that desaturases with different substrate specificities can be expressed in a heterologous system and used to produce poly-unsaturated long chain fatty acids.

Table 2 represents fatty acids of interest as a percent of the total lipid extracted from the yeast host *S. cerevisiae* 334 with the indicated plasmid. No glucose was present in the growth media. Affinity gas chromatography was used to separate the respective lipids. GC/MS was employed to verify the identity product(s). The expected product for the *B. napus* Δ15-desaturase, α-linolenic acid, was detected when its substrate, linolenic acid, was added exogenously to the induced yeast culture. This finding demonstrates that yeast expression of a desaturase gene can produce functional enzyme and detectable amounts of product under the current growth conditions. Both exogenously added substrates were taken up by yeast, although slightly less of the longer chain PUFA, dihomo-γ-linolenic acid (20:3), was incorporated into yeast than linolenic acid (18:2) when either was added in free form to the induced yeast cultures. Arachidonic acid was detected as a novel PUFA in yeast when dihomo-γ-linolenic acid was added as the substrate to *S. cerevisiae* 334 (pCGR-4). This identifies pCGR-4 (MA29) as the Δ5-desaturase from *M*. *alpina*. Prior to this, no isolation and expression of a Δ5-desaturase from any source has been reported.

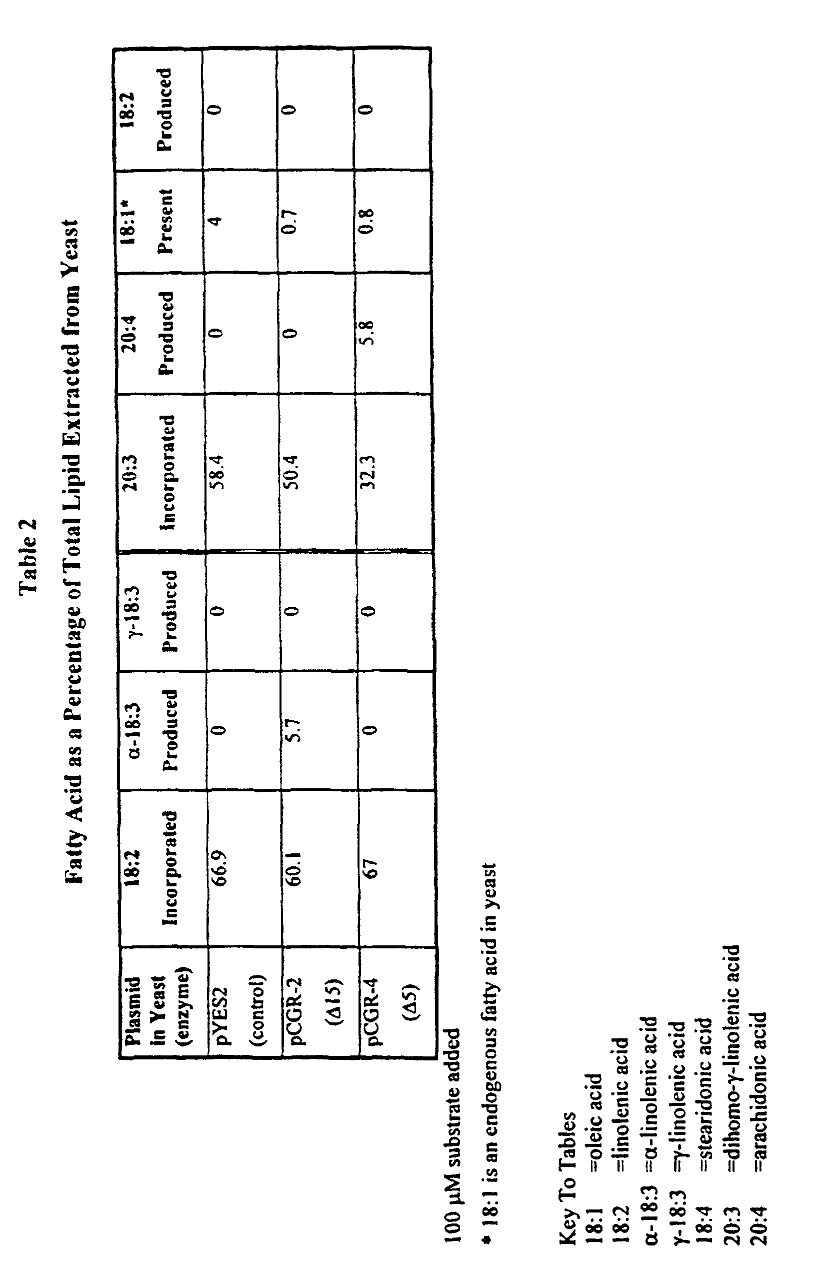

### Example 3

### Optimization of Culture Conditions

Table 3A shows the effect of exogenous free fatty acid substrate concentration on yeast uptake and conversion to fatty acid product as a percentage of the total yeast lipid extracted. In all instances, low amounts of exogenous substrate (1-10 µM) resulted in low fatty acid substrate uptake and product formation. Between 25 and 50 µM concentration of free fatty acid in the growth and induction media gave the highest percentage of fatty acid product formed, while the 100 µM concentration and subsequent high uptake into yeast appeared to decrease or inhibit the desaturase activity. The feedback inhibition of high fatty acid substrate concentration was well illustrated when the percent conversion rates of the respective fatty acid substrates to their respective products were compared in Table 3B. In all cases, 100 µM substrate concentration in the growth media decreased the percent conversion to product. The effect of media composition was also evident when glucose was present in the growth media for the Δ5-desaturase, since the percent of substrate uptake was decreased at 25 µM (Table 3A). However, the percent conversion by Δ5-desaturase increased by 18% and the percent product formed remained the same in the presence of glucose in the growth media.

**Table 3A**

| **Effect of Added Substrate on the Percentage of Incorporated Substrate and Product Formed in Yeast Extracts** | | |
|---|---|---|
| **Plasmid in Yeast** | **pCGR-2 (Δ15)** | **pCGR-4 (Δ5)** |
| substrate/product | 18:2 /α-18:3 | 20:3/20:4 |
| 1 µM sub. | ND | 0.5/1.7 |
| 10µM sub. | ND | 3.3/4 |
| 25 µ M sub. | ND | 5.1/6.1 |
| 25 µM◇ sub. | 36.6/7.2◇ | 9.3/5.4◇ |
| 50 µM sub. | 53.1/6.5◇ | ND |
| 100 µM sub. | 60.1/5.7◇ | 32.3/5.8◇ |

**Table 3B**

| **Effect of Substrate Concentration in Media on the Percent Conversion of Fatty Acid Substrate to Product in Yeast Extracts** | | |
|---|---|---|
| **Plasmid in Yeast** | **pCGR-2 (Δ15)** | **pCGR-4 (Δ5)** |
| substrate/product | 18:2 →α-18:3 | 20:3→20:4 |
| 1 µM sub. | ND | 77.3 |
| 10 µM sub. | ND | 54.8 |
| 25 µM sub. | ND | 54.2 |
| 25 µM◇ sub. | 16.4 | 36.7 |
| 50 µM sub. | 10.90 | ND |
| 100 µM sub. | 8.70 | 15.2◇ |
| ◇ no glucose in media | | |
| ⁺ Yeast peptone broth (YPD) | | |
| * 18:1 is an endogenous yeast lipid sub. is substrate concentration | | |
| ND (not done) | | |

Table 4 shows the amount of fatty acid produced by a recombinant desaturase from induced yeast cultures when different amounts of free fatty acid substrate were used. Fatty acid weight was determined since the total amount of lipid varied dramatically when the growth conditions were changed, such as the presence of glucose in the yeast growth and induction media. To better determine the conditions when the recombinant desaturase would produce the most PUFA product, the quantity of individual fatty acids were examined. The absence of glucose reduced the amount of arachidonic acid produced by Δ5-desaturase by half. For Δ5-desaturase the amount of total yeast lipid was decreased by almost half in the absence of glucose.

**Table 4**

| **Fatty Acid Produced in µg from Yeast Extracts** | | |
|---|---|---|
| **Plasmid in Yeast (enzyme)** | **pCGR-4 (Δ5)** | **pCGR-7 (Δ12)** |
| product | 20:4 | 18:2* |
| 1 µM sub. | 8.3 | ND |
| 10 µM sub. | 19.2 | ND |
| 25 µM sub. | 31.2 | 115.7 |
| 25 µM ◇ sub. | 16.8 | 39◇ |
| ◇ no glucose in media | | |
| sub. is substrate concentration | | |
| ND (not done) | | |
| *18:1, the substrate, is an endogenous yeast lipid | | |

### Example 4

### Distribution of PUFAs in Yeast Lipid Fractions

Table 5 illustrates the uptake of free fatty acids and their new products formed in yeast lipids as distributed in the major lipid fractions. A total lipid extract was prepared as described above. The lipid extract was separated on TLC plates, and the fractions were identified by comparison to standards. The bands were collected by scraping, and internal standards were added. The fractions were then saponified and methylated as above, and subjected to gas chromatography. The gas chromatograph calculated the amount of fatty acid by comparison to a standard. It would appear that the substrates are accessible in the phospholipid form to the desaturases.

**Table 5**

| **Fatty Acid Distribution in Various Yeast Lipid Fractions in µg** | | | | | |
|---|---|---|---|---|---|
| Fatty acid fraction | Phospholipid | Diglyceride | Free Fatty Acid | Triglyceride | Cholesterol Ester |
| SC (pCGR-4) substrate 20:3 | 15.1 | 1.9 | 22.9 | 12.6 | 3.3 |
| SC (pCGR-4) product 20:4 | 42.6 | 0.9 | 6.8 | 4.9 | 0.4 |
| SC = *S. cerevisiae* (plasmid) | | | | | |

### Example 5

### Further Culture Optimization

The growth and induction conditions for optimal activities of desaturases in *Saccharomyces cerevisiae* were evaluated. Various culture conditions that were manipulated for optimal activity were: I) induction temperature, ii) concentration of inducer, iii) timing of substrate addition, iv) concentration of substance, v) sugar source, vi) growth phase at induction. These studies were done using Δ5-desaturase gene from *Mortierella alpina* (MA 29). In addition, the effect of changing host strain on expression of the Δ5-desaturase gene was also determined.

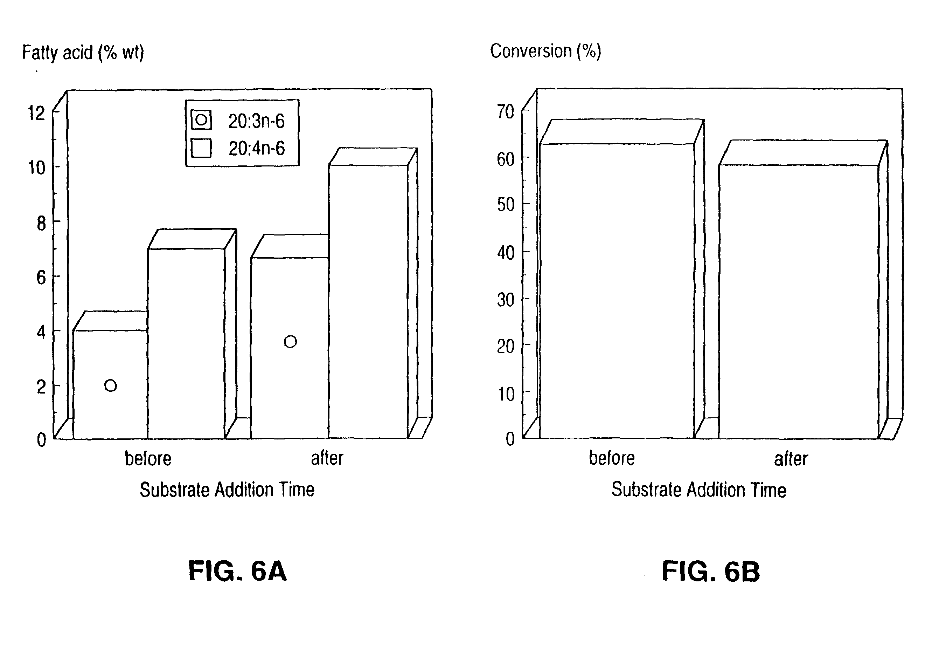

As described above, the best rate of conversion of substrate to ARA was observed at a substrate concentration of 1 µM, however, the percentage of ARA in the total fatty acids was highest at 25 µM substrate concentration. To determine if the substrate needed to be modified to a readily available form before it could be utilized by the desaturase, the substrate was added either 15 hours before induction or concomitant with inducer addition (indicated as after, in Figure 6A). As it can be seen in Figure 6A, addition of substrate before induction did not have a significant effect on the activity of Δ5-desaturase. In fact, addition of substrate along with the inducer was slightly better for expression/activity of Δ5-desaturase, as ARA levels in the total fatty acids were higher. However, the rate of conversion of substrate to product was slightly lower.

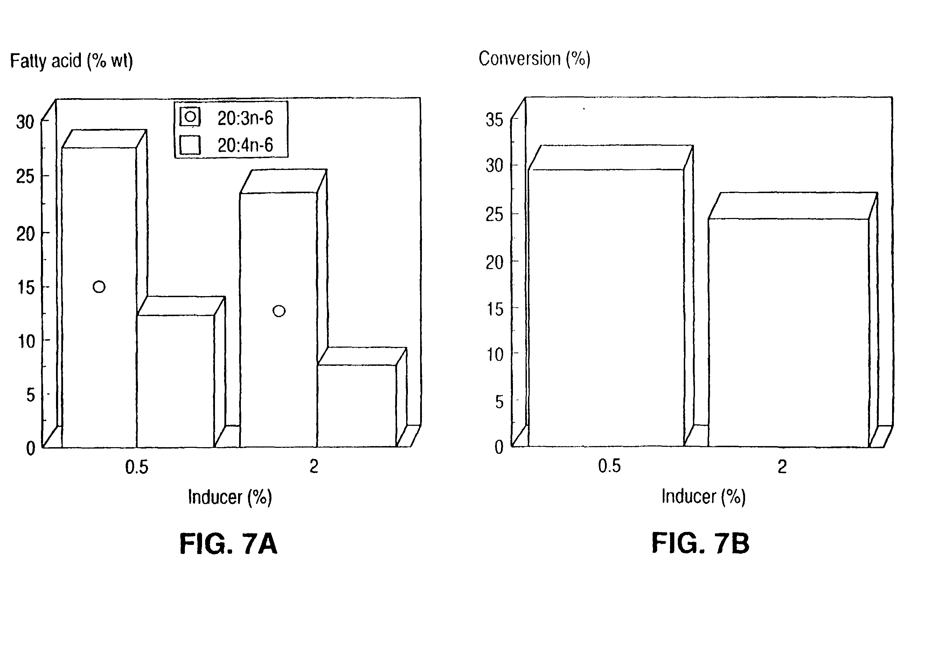

The effect of inducer concentration of expression/activity of *Mortierella* Δ5-desaturase was examined by inducing SC334/pCGR5 with 0.5 or 2% (w/v) of galactose. As shown in Figures 7A and 7B, expression of Δ5-desaturase was higher when induced with 0.5% galactose. Furthermore, rate of conversion of substrate to product was also better when SC334/pCGR5 was induced with 0.5% galactose vs 2% galactose.

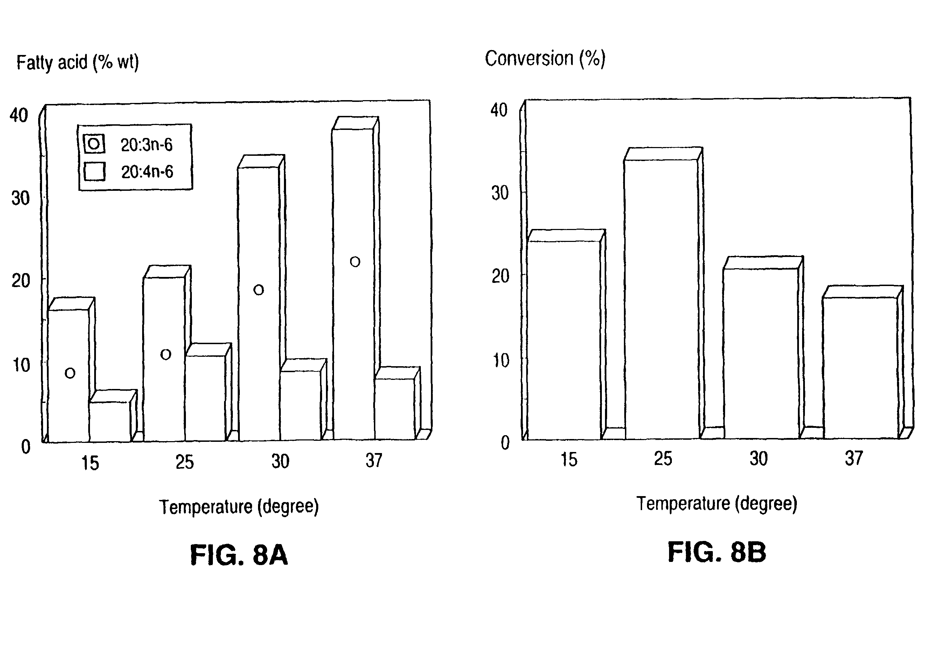

To determine the effect of temperature on Δ5-desaturase activity, the SC334 host strain, transformed with pCGR5 (SC334/pCGR5) was grown and induced at 15° C, 25°C, 30°C and 37°C. The quantity of ARA (20:4n6) produced in SC334/pCGR5 cultures, supplemental with substrate 20:3n6, was measured by fatty acid analysis. Figure 8A depicts the quantity of 20:3n6 and 20:4n6, expressed as percentage of total fatty acids. Figure 8B depicts the rate of conversion of substrate to product. Growth and induction of SC334/pCGR5 at 25°C, was the best for the expression of Δ5-desaturase as evidenced by the highest levels of arachidonic acid in the total fatty acids. Additionally the highest rate of conversion of substrate to product also occurred at 25°C. Growth and induction at 15°C gave the lowest expression of ARA, whereas at 37°C gave the lowest conversion of substrate to product.

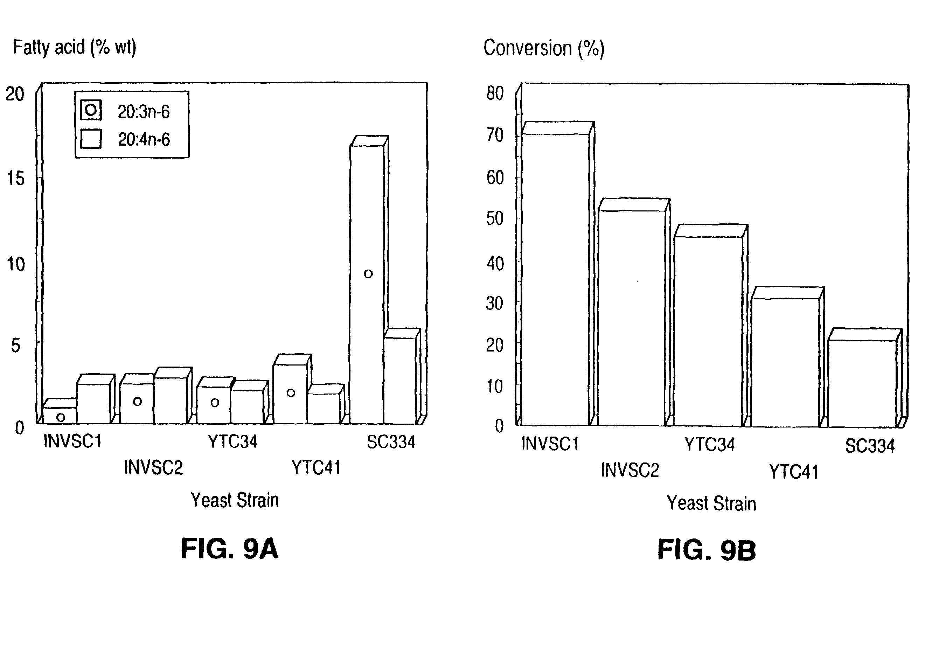

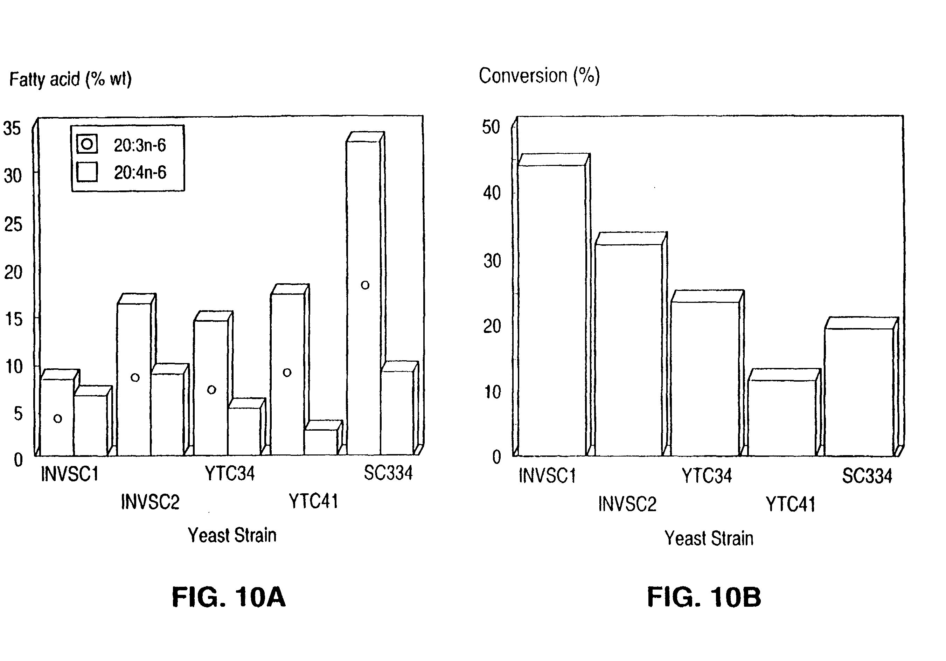

The effect of yeast strain on expression of the Δ5-desaturase gene was studied in 5 different host strains; INVSC1, INVSC2, YTC34, YTC41, and SC334, at 15°C and 30°C. At 15°C, SC334 has the highest percentage of ARA in total fatty acids, suggesting higher activity of Δ5-desaturase in SC334. The rate of conversion of substrate to product, however is lowest in SC334 and highest in INVSC1 (Fig. 9A and B). At 30°C, the highest percentage of product (ARA) in total fatty acids was observed in INVSC2, although the rate of conversion of substrate to product in INVSC2 was slightly lower than INVSC1 (Fig. 10A and B).

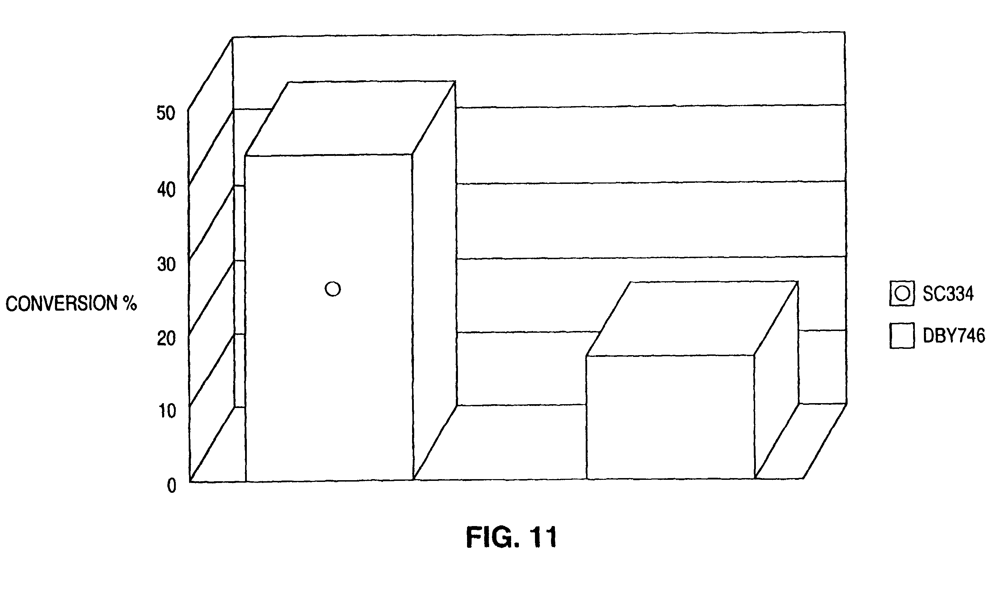

ARA, the product of Δ5-desaturase, is stored in the phospholipid faction (Example 4). Therefore the quantity of ARA produced in yeast is limited by the amount that can be stored in the phospholipid fraction. If ARA could also be stored in other fractions such as the triglyceride fraction, the quantity of ARA produced in yeast might be increased. To test this hypothesis, the Δ5-desaturase gene was expressed in the yeast host strain DBY746 (obtained from the Yeast Genetic Stock Centre, 1021 Donner Laboratory, Berkeley, CA 94720. The genotype of strain DBY746 is Matα, his3-Δ1, leu2-3, leu2-112, ura3-32, trp1-289, gal). The DBY746 yeast strain has an endogenous gene for choline transferase. The presence of this enzyme might enable the DBY746 strain to convert excess phospholipids into triglycerides fraction. Results in Fig. 11 show no increase in the conversion of substrate to product as compared to SC334, which does not have the gene for choline transferase.

To study the effect of media on expression of Δ5-desaturase, pCGR4/SC334 was grown in four different media at two different temperatures (15°C and 30°) and in two different host strains (SC334 and INVSC1). The composition of the media was as follows:
Media A: mm-Ura, + 2% galactose + 2% glucose.
Media B: mm-Ura, + 20% galactose + 2% Glucose + 1M sorbitol (pH5.8)
Media C: mm-Ura, + 2% galactose + 2% raffmose
Media D: mm-Ura, + 2% galactose +2% raffinose + 1M sorbitol (pH5.8)
mm=minimal media

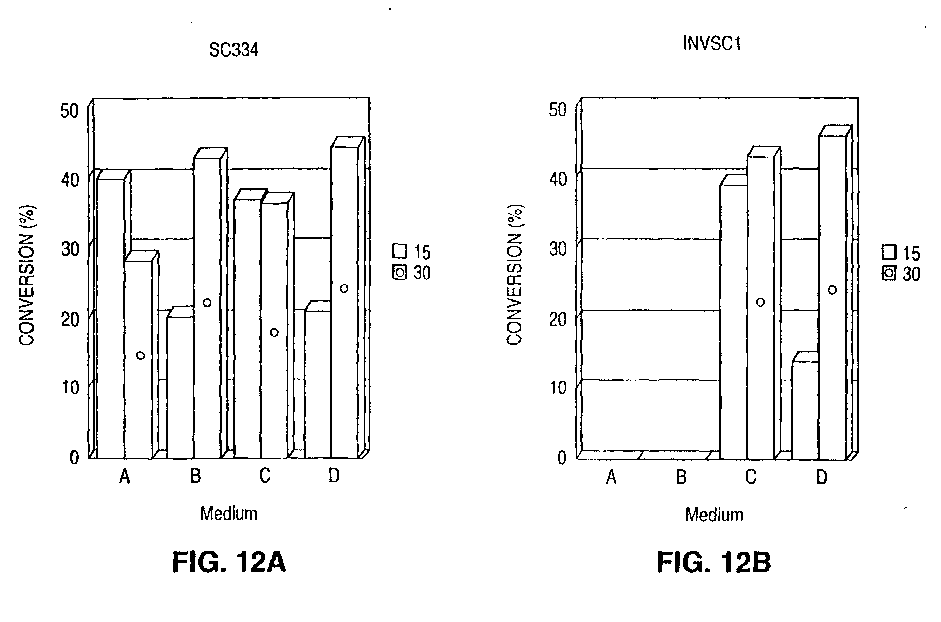

Results show that the highest conversion rate of substrate to product at 15°C in SC334 was observed in media A. The highest conversion rate overall for Δ5-desaturase in SC334 was at 30° in media D. The highest conversion rate of Δ5-desaturase in INVSC1 was also at 30° in media D (Figures 12A and 12B).

These data show that a DNA encoding a desaturase that can convert DGLA to ARA can be isolated from *Mortierella alpina* and can be expressed in a heterologous system and used to produce poly-unsaturated long chain fatty acids. Exemplified is the production of ARA from the precursor DGLA by expression of a Δ5-desaturase in yeast.

### Example 6

### Identification of Homologues to M. alpina Δ5 and Δ6 desaturases

A nucleic acid sequence that encodes a putative Δ5 desaturase was identified through a TBLASTN search of the est databases through NCBI using amino acids 100-446 of Ma29 as a query. The truncated portion of the Ma29 sequence was used to avoid picking up homologies based on the cytochrome b5 portion at the N-terminus of the desaturase. The deduced amino acid sequence of an est from *Dictyostelium discoideum* (accession # C25549) shows very significant homology to Ma29 and lesser, but still significant homology to Ma524. The DNA sequence is presented as SEQ ID NO:13. The amino acid sequence is presented as SEQ ID NO:14.

### Example 7

### Identification of M. alpina Δ5 and Δ6 homologues in other PUFA-producing organisms

To look for desaturases involved in PUFA production, a cDNA library was constructed from total RNA isolated from Phaeodactylum tricornutum. A plasmid-based cDNA library was constructed in pSPORT1 (GIBCO-BRL) following manufacturer's instructions using a commercially available kit (GIBCO-BRL). Random cDNA clones were sequenced and nucleic acid sequences that encode putative Δ5 or Δ6 desaturases were identified through BLAST search of the databases and comparison to Ma29 and Ma524 sequences.

One clone was identified from the *Phaeodactylum* library with homology to Ma29 and Ma524; it is called 144-011-B12. The DNA sequence is presented as SEQ ID NO:15. The amino acid sequence is presented as SEQ ID NO:16.

### Example 8

### Identification of M. alpina Δ5 and Δ6 homologues in other PUFA-producing organisms

To look for desaturases involved in PUFA production, a cDNA library was constructed from total RNA isolated from *Schizochytrium* species. A plasmid-based cDNA library was constructed in pSPORT1 (GIBCO-BRL) following manufacturer's instructions using a commercially available kit (GIBCO-BRL). Random cDNA clones were sequenced and nucleic acid sequences that encode putative Δ5 or Δ6 desaturases were identified through BLAST search of the databases and comparison to Ma29 and Ma524 sequences.

One clone was identified from the *Schizochytrium* library with homology to Ma29 and Ma524; it is called 81-23-C7. This clone contains a ∼1 kb insert. Partial sequence was obtained from each end of the clone using the universal forward and reverse sequencing primers. The DNA sequence from the forward primer is presented as SEQ ID NO:17. The peptide sequence is presented as SEQ ID NO:18. The DNA sequence from the reverse primer is presented as SEQ ID NO:19. The amino acid sequence from the reverse primer is presented as SEQ ID NO:20.

### Example 9

### Human Desaturase Gene Sequences

Human desaturase gene sequences potentially involved in long chain polyunsaturated fatty acid biosynthesis were isolated based on homology between the human cDNA sequences and *Mortierella alpina* desaturase gene sequences. The three conserved "histidine boxes" known to be conserved among membrane-bound desaturases were found. As with some other membrane-bound desaturases the final HXXHH histidine box motif was found to be QXXHH. The amino acid sequence of the putative human desaturases exhibited homology to *M*. *alpina* Δ5, Δ6, Δ9, and Δ12 desaturases.

The *M*. *alpina* Δ5 desaturase and Δ6 desaturase cDNA sequences were used to search the LifeSeq database of Incyte Pharmaceuticals, Inc., Palo Alto, California 94304. The Δ5 desaturase sequence was divided into fragments; 1) amino acid no. 1-150, 2) amino acid no. 151-300, and 3) amino acid no. 301-446. The Δ6 desaturase sequence was divided into three fragments; 1) amino acid no. 1-150, 2) amino acid no. 151-300, and 3) amino acid no. 301-457. These polypeptide fragments were searched against the database using the "tblastn" algorithm. This alogarithm compares a protein query sequence against a nucleotide sequence database dynamically translated in all six reading frames (both strands).

The polypeptide fragments 2 and 3 of *M. alpina* Δ5 and Δ6 have homologies with the CloneID sequences as outlined in Table 6. The CloneID represents an individual sequence from the Incyte LifeSeq database. After the "tblastn" results have been reviewed, Clone Information was searched with the default settings of Stringency of >=50, and Productscore <=100 for different CloneID numbers. The Clone Information Results displayed the information including the ClusterID, CloneID, Library, HitID, Hit Description. When selected, the ClusterID number displayed the clone information of all the clones that belong in that ClusterID. The Assemble command assembles all of the CloneID which comprise the ClusterID. The following default settings were used for GCG (Genetics Computer Group, University of Wisconsin Biotechnology Center, Madison, Wisconsin 53705) Assembly:

| | |
|---|---|
| Word Size | 7 |
| Minimum Overlap | 14 |
| Stringency | 0.8 |
| Minimum Identity | 14 |
| Maximum Gap | 10 |
| Gap Weight | 8 |
| Length Weight | 2 |

GCG Assembly Results displayed the contigs generated on the basis of sequence information within the CloneID. A contig is an alignment of DNA sequences based on areas of homology among these sequences. A new sequence (consensus sequence) was generated based on the aligned DNA sequences within a contig. The contig containing the CloneID was identified, and the ambiguous sites of the consensus sequence was edited based on the alignment of the CloneIDs (see SEQ ID NO:21 - SEQ ID NO:25) to generate the best possible sequence. The procedure was repeated for all six CloneID listed in Table 6. This produced five unique contigs. The edited consensus sequences of the 5 contigs were imported into the Sequencher software program (Gene Codes Corporation, Ann Arbor, Michigan 48 105). These consensus sequences were assembled. The contig 2511785 overlaps with contig 3506132, and this new contig was called 2535 (SEQ ID NO:27). The contigs from the Sequencher program were copied into the Sequence Analysis software package of GCG.

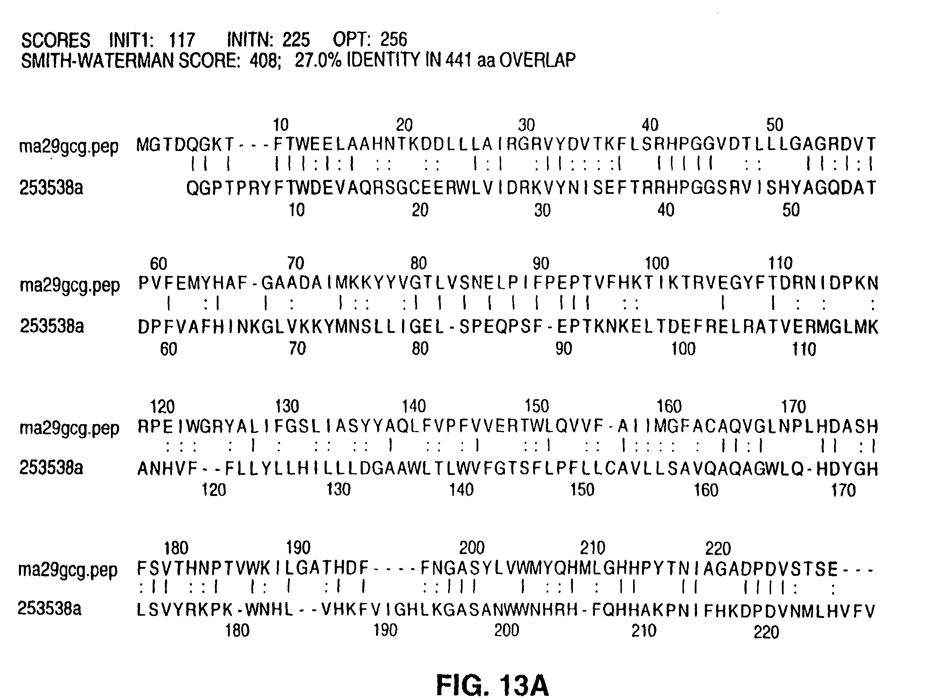

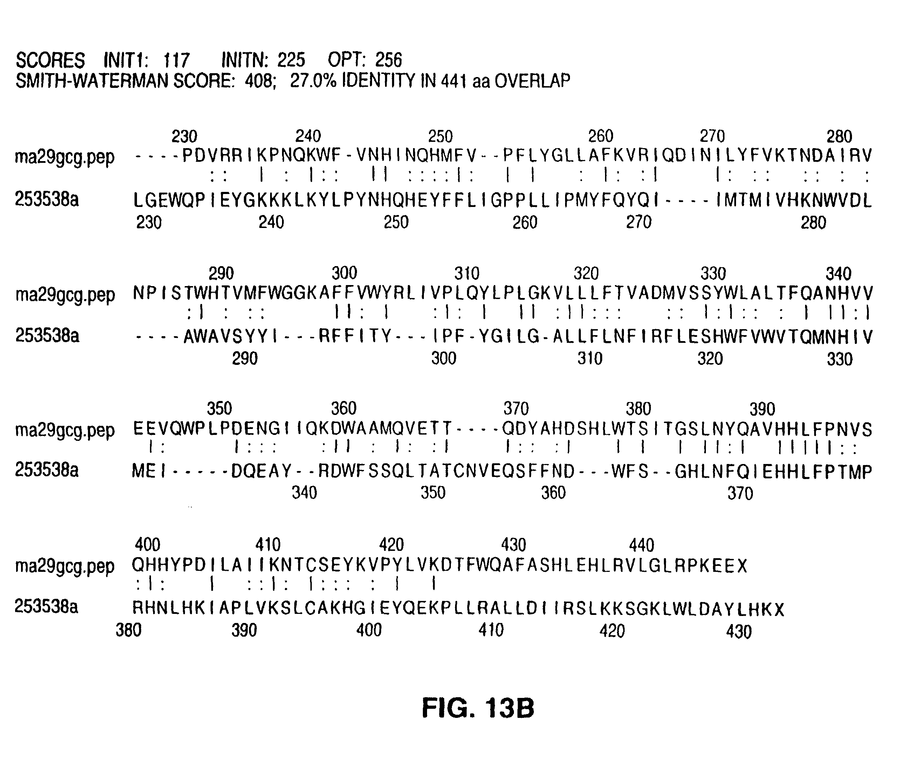

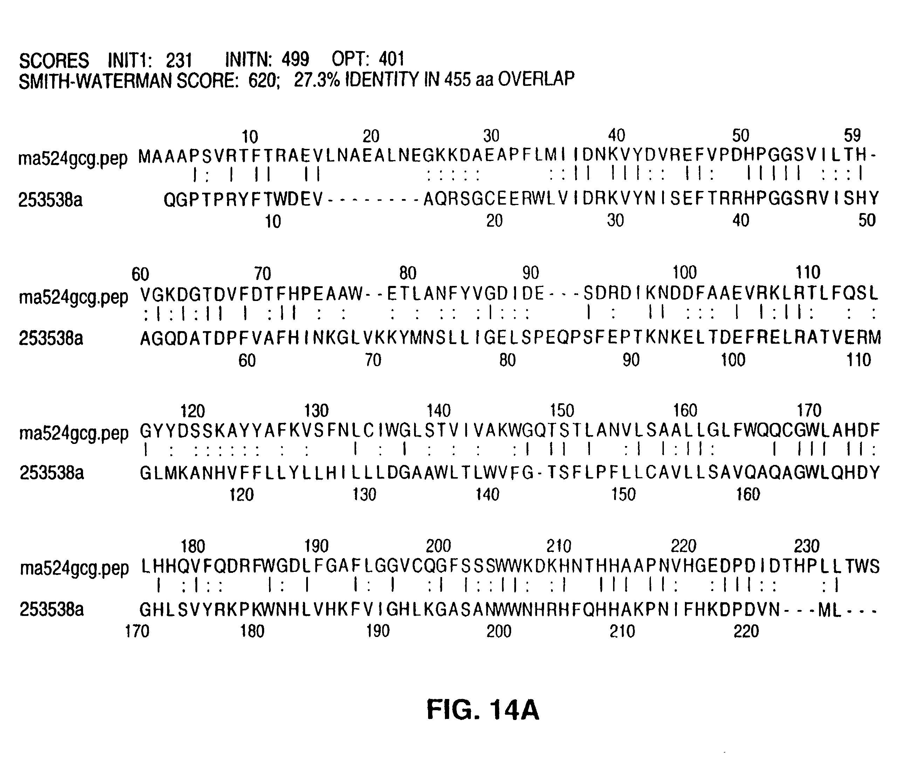

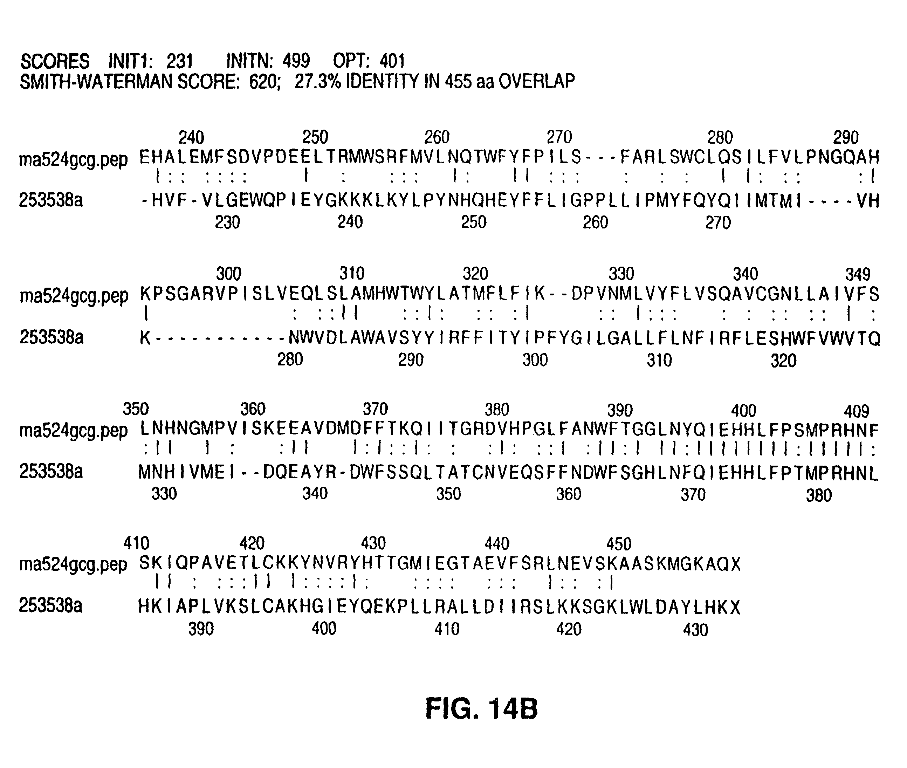

Each contig was translated in all six reading frames into protein sequences. The *M. alpina* Δ5 (MA29) and Δ6 (MA524) sequences were compared with each of the translated contigs using the FastA search (a Pearson and Lipman search for similarity between a query sequence and a group of sequences of the same type (nucleic acid or protein)). Homology among these sequences suggest the open reading frames of each contig. The homology among the *M. alpina* A5 and Δ6 to contigs 2535 and 3854933 were utilized to create the final contig called 253538a. Figure 13 is the FastA match of the final contig 253538a and MA29, and Figure 14 is the FastA match of the final contig 253538a and MA524. The DNA sequences for the various contigs are presented in SEQ ID NO:21 -SEQ ID NO:27. The various peptide sequences are shown in SEQ ID NO:28 - SEQ ID NO:34.

Although the open reading frame was generated by merging the two contigs, the contig 2535 shows that there is a unique sequence in the beginning of this contig which does not match with the contig 3854933. Therefore, it is possible that these contigs were generated from independent desaturase like human genes.

The contig 253538a contains an open reading frame encoding 432 amino acids. It starts with Gln (CAG) and ends with the stop codon (TGA). The contig 253538a aligns with both *M. alpina* Δ5 and Δ6 sequences, suggesting that it could be either of the desaturases, as well as other known desaturases which share homology with each other. The individual contigs listed in Table 6, as well as the intermediate contig 2535 and the final contig 253538a can be utilized to isolate the complete genes for human desaturases.

### Uses of the human desaturases

These human sequences can be expressed in yeast and plants utilizing the procedures described in the preceding examples. For expression in mammalian cells and transgenic animals, these genes may provide superior codon bias. These human sequences can also be used to identify related desaturase sequences.

**Table 6**

| **Sections of the Desaturases** | **Clone ID from LifeSeq Database** | **Keyword** |
|---|---|---|
| 151-300 Δ5 | 3808675 | Fatty acid desaturase |
| 301-446 Δ5 | 354535 | Δ6 |
| 151-300 Δ6 | 3448789 | Δ6 |
| 151-300 Δ6 | 1362863 | Δ6 |
| 151-300 Δ6 | 2394760 | Δ6 |
| 301-457 Δ6 | 3350263 | Δ6 |

### Example 10

### Nutritional Compositions

The PUFAs of the previous examples can be utilized in various nutritional supplements, infant formulations, nutritional substitutes and other nutrition solutions.

### I. INFANT FORMULATIONS

### A. Isomil® Soy Formula with Iron.

Usage: As a beverage for infants, children and adults with an allergy or sensitivity to cow's milk. A feeding for patients with disorders for which lactose should be avoided: lactase deficiency, lactose intolerance and galactosemia.

### Features:

- Soy protein isolate to avoid symptoms of cow's-milk-protein allergy or sensitivity
- Lactose-free formulation to avoid lactose-associated diarrhea
- Low osmolaity (240 mOsm/kg water) to reduce risk of osmotic diarrhea.
- Dual carbohydrates (corn syrup and sucrose) designed to enhance carbohydrate absorption and reduce the risk of exceeding the absorptive capacity of the damaged gut.
- 1.8 mg of Iron (as ferrous sulfate) per 100 Calories to help prevent iron deficiency.
- Recommended levels of vitamins and minerals.
- Vegetable oils to provide recommended levels of essential fatty acids.
- Milk-white color, milk-like consistency and pleasant aroma.

Ingredients: (Pareve, 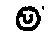 ) 85% water, 4.9% corn syrup, 2.6% sugar (sucrose), 2.1% soy oil, 1.9% soy protein isolate, 1.4% coconut oil, 0.15% calcium citrate, 0.11 % calcium phosphate tribasic, potassium citrate, potassium phosphate monobasic, potassium chloride, mono- and disglycerides, soy lecithin, carrageenan, ascorbic acid, L-methionine, magnesium chloride, potassium phosphate dibasic, sodium chloride, choline chloride, taurine, ferrous sulfate, m-inositol, alpha-tocopheryl acetate, zinc sulfate, L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D₃ and cyanocobalamin.

### B. Isomil® DF Soy Formula For Diarrhea.

Usage: As a short-term feeding for the dietary management of diarrhea in infants and toddlers.

### Features:

- First infant formula to contain added dietary fiber from soy fiber specifically for diarrhea management.
- Clinically shown to reduce the duration of loose, watery stools during mild to severe diarrhea in infants.
- Nutritionally complete to meet the nutritional needs of the infant.
- Soy protein isolate with added L-methionine meets or exceeds an infant's requirement for all essential amino acids.
- Lactose-free formulation to avoid lactose-associated diarrhea.
- Low osmolality (240 mOsm/kg water) to reduce the risk of osmotic diarrhea.
- Dual carbohydrates (corn syrup and sucrose) designed to enhance carbohydrate absorption and reduce the risk of exceeding the absorptive capacity of the damaged gut.
- Meets or exceeds the vitamin and mineral levels recommended by the Committee on Nutrition of the American Academy of Pediatrics and required by the Infant Formula Act.
- 1.8 mg of iron (as ferrous sulfate) per 100 Calories to help prevent iron deficiency.
- Vegetable oils to provide recommended levels of essential fatty acids.

Ingredients: (Pareve, 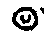 ) 86% water, 4.8% corn syrup, 2.5% sugar (sucrose), 2.1% soy oil, 2.0% soy protein isolate, 1.4% coconut oil, 0.77% soy fiber, 0.12% calcium citrate, 0.11 % calcium phosphate tribasic, 0.10% potassium citrate, potassium chloride, potassium phosphate monobasic, mono-and disglycerides, soy lecithin, carrageenan, magnesium chloride, ascorbic acid, L-methionine, potassium phosphate dibasic, sodium chloride, choline chloride, taurine, ferrous sulfate, m-inositol, alpha-tocopheryl acetate, zinc sulfate, L-camitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D₃ and cyanocobalamin.

### C. Isomil® SF Sucrose-Free Soy Formula With Iron.

Usage: As a beverage for infants, children and adults with an allergy or sensitivity to cow's-milk protein or an intolerance to sucrose. A feeding for patients with disorders for which lactose and sucrose should be avoided.

### Features:

- Soy protein isolate to avoid symptoms of cow's-milk-protein allergy or sensitivity.
- Lactose-free formulation to avoid lactose-associated diarrhea (carbohydrate source is Polycose® Glucose Polymers).
- Sucrose free for the patient who cannot tolerate sucrose.
- Low osmolality (180 mOsm/kg water) to reduce risk of osmotic diarrhea.
- 1.8 mg of iron (as ferrous sulfate) per 100 Calories to help prevent iron deficiency.
- Recommended levels of vitamins and minerals.
- Vegetable oils to provide recommended levels of essential fatty acids.
- Milk-white color, milk-like consistency and pleasant aroma.

Ingredients: (Pareve, 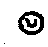 ) 75% water, 11.8% hydrolized cornstarch, 4.1 % soy oil, 4.1% soy protein isolate, 2.8% coconut oil, 1.0% modified cornstarch, 0.38% calcium phosphate tribasic, 0.17% potassium citrate, 0.13% potassium chloride, mono- and disglycerides, soy lecithin, magnesium chloride, abscorbic acid, L-methionine, calcium carbonate, sodium chloride, choline chloride, carrageenan, taurine, ferrous sulfate, m-inositol, alpha-tocopheryl acetate, zinc sulfate, L-camitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D₃ and cyanocobalamin

### D. Isomil® 20 Soy Formula With Iron Ready To Feed, 20 Cal/fl oz.

Usage: When a soy feeding is desired.

Ingredients: (Pareve, 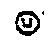 ) 85% water, 4.9% corn syrup, 2.6% sugar (sucrose), 2.1% soy oil, 1.9% soy protein isolate, 1.4% coconut oil, 0.15% calcium citrate, 0.11% calcium phosphate tribasic, potassium citrate, potassium phosphate monobasic, potassium chloride, mono- and disglycerides, soy lecithin, carrageenan, abscorbic acid, L-methionine, magnesium chloride, potassium phosphate dibasic, sodium chloride, choline chloride, taurine, ferrous sulfate, m-inositol, alpha-tocopheryl acetate, zinc sulfate, L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D₃ and cyanocobalamin.

### E. Similac® Infant Formula

Usage: When an infant formula is needed: if the decision is made to discontinue breastfeeding before age 1 year, if a supplement to breastfeeding is needed or as a routine feeding if breastfeeding is not adopted.

### Features:

- Protein of appropriate quality and quantity for good growth; heat-denatured, which reduces the risk of milk-associated enteric blood loss.
- Fat from a blend of vegetable oils (doubly homogenized), providing essential linoleic acid that is easily absorbed.
- Carbohydrate as lactose in proportion similar to that of human milk.
- Low renal solute load to minimize stress on developing organs.
- Powder, Concentrated Liquid and Ready To Feed forms.

Ingredients: ( 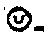 -D) Water, nonfat milk, lactose, soy oil, coconut oil, mono- and diglycerides, soy lecithin, abscorbic acid, carrageenan, choline chloride, taurine, m-inositol, alpha-tocopheryl acetate, zinc sulfate, niacinamid, ferrous sulfate, calcium pantothenate, cupric sulfate, vitamin A palmitate, thiamine chloride hydrochloride, riboflavin, pyridoxine hydrochloride, folic acid, manganese sulfate, phylloquinone, biotin, sodium selenite, vitamin D₃ and cyanocobalamin.

### F. Similac® NeoCare Premature Infant Formula With Iron

Usage: For premature infants' special nutritional needs after hospital discharge. Similac NeoCare is a nutritionally complete formula developed to provide premature infants with extra calories, protein, vitamins and minerals needed to promote catch-up growth and support development.

### Features:

- Reduces the need for caloric and vitamin supplementation. More calories (22 Cal/fl oz) then standard term formulas (20 Cal/fl oz).
- Highly absorbed fat blend, with medium-chain triglycerides (MCT oil) to help meet the special digestive needs of premature infants.
- Higher levels of protein, vitamins and minerals per 100 Calories to extend the nutritional support initiated in-hospital.
- More calcium and phosphorus for improved bone mineralization.

Ingredients: 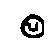 -D Corn syrup solids, nonfat milk, lactose, whey protein concentrate, soy oil, high-oleic safflower oil, fractionated coconut oil (medium-chain triglycerides), coconut oil, potassium citrate, calcium phosphate tribasic, calcium carbonate, ascorbic acid, magnesium chloride, potassium chloride, sodium chloride, taurine, ferrous sulfate, m-inositol, choline chloride, ascorbyl palmitate, L-carnitine, alpha-tocopheryl acetate, zinc sulfate, niacinamide, mixed tocopherols, sodium citrate, calcium pantothenate, cupric sulfate, thiamine chloride hydrochloride, vitamin A palmitate, beta carotene, riboflavin, pyridoxine hydrochloride, folic acid, manganese sulfate, phylloquinone, biotin, sodium selenite, vitamin D₃ and cyanocobalamin.

### G. Similac Natural Care Low-Iron Human Milk Fortifier Ready To Use, 24 Cal/fl oz.

Usage: Designed to be mixed with human milk or to be fed alternatively with human milk to low-birth-weight infants.

Ingredients: 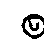 -D Water, nonfat milk, hydrolyzed cornstarch, lactose, fractionated coconut oil (medium-chain triglycerides), whey protein concentrate, soil oil, coconut oil, calcium phosphate tribasic, potassium citrate, magnesium chloride, sodium citrate, ascorbic acid, calcium carbonate, mono-and diglycerides, soy lecithin, carrageenan, choline chloride, m-inositol, taurine, niacinamide, L-carnitine, alpha tocopheryl acetate, zinc sulfate, potassium chloride, calcium pantothenate, ferrous sulfate, cupric sulfate, riboflavin, vitamin A palmitate, thiamine chloride hydrochloride, pyridoxine hydrochloride, biotin, folic acid, manganese sulfate, phylloquinone, vitamin D₃, sodium selenite and cyanocobalamin.

Various PUFAs of this invention can be substituted and/or added to the infant formulae described above and to other infant formulae known to those in the art..

### II. NUTRITIONAL FORMULATIONS

### A. ENSURE®

Usage: ENSURE is a low-residue liquid food designed primarily as an oral nutritional supplement to be used with or between meals or, in appropriate amounts, as a meal replacement. ENSURE is lactose- and gluten-free, and is suitable for use in modified diets, including low-cholesterol diets. Although it is primarily an oral supplement, it can be fed by tube.

### Patient Conditions:

- For patients on modified diets
- For elderly patients at nutrition risk
- For patients with involuntary weight loss
- For patients recovering from illness or surgery
- For patients who need a low-residue diet

### Ingredients:

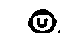 -D Water, Sugar (Sucrose), Maltodextrin (Corn), Calcium and Sodium Caseinates, High-Oleic Safflower Oil, Soy Protein Isolate, Soy Oil, Canola Oil, Potassium Citrate, Calcium Phosphate Tribasic, Sodium Citrate, Magnesium Chloride, Magnesium Phosphate Dibasic, Artificial Flavor, Sodium Chloride, Soy Lecithin, Choline Chloride, Ascorbic Acid, Carrageenan, Zinc Sulfate, Ferrous Sulfate, Alpha-Tocopheryl Acetate, Gellan Gum, Niacinamide, Calcium Pantothenate, Manganese Sulfate, Cupric Sulfate, Vitamin A Palmitate, Thiamine Chloride Hydrochloride, Pyridoxine Hydrochloride, Riboflavin, Folic Acid, Sodium Molybdate, Chromium Chloride, Biotin, Potassium Iodide, Sodium Selenate.

### B. ENSURE® BARS

Usage: ENSURE BARS are complete, balanced nutrition for supplemental use between or with meals. They provide a delicious, nutrient-rich alternative to other snacks. ENSURE BARS contain <1 g lactose/bar, and Chocolate Fudge Brownie flavor is gluten-free. (Honey Graham Crunch flavor contains gluten.)

### Patient Conditions:

- For patients who need extra calories, protein, vitamins and minerals
- Especially useful for people who do not take in enough calories and nutrients
- For people who have the ability to chew and swallow
- Not to be used by anyone with a peanut allergy or any type of allergy to nuts.

### Ingredients:

Honey Graham Crunch -- High-Fructose Corn Syrup, Soy Protein Isolate, Brown Sugar, Honey, Maltodextrin (Com), Crisp Rice (Milled Rice, Sugar [Sucrose], Salt [Sodium Chloride] and Malt), Oat Bran, Partially Hydrogenated Cottonseed and Soy Oils, Soy Polysaccharide, Glycerine, Whey Protein Concentrate, Polydextrose, Fructose, Calcium Caseinate, Cocoa Powder, Artificial Flafors, Canola Oil, High-Oleic Safflower Oil, Nonfat Dry Milk, Whey Powder, Soy Lecithin and Corn Oil. Manufactured in a facility that processes nuts.

### Vitamins and Minerals:

Calcium Phosphate Tribasic, Potassium Phosphate Dibasic, Magnesium Oxide, Salt (Sodium Chloride), Potassium Chloride, Ascorbic Acid, Ferric Orthophosphate, Alpha-Tocopheryl Acetate, Niacinamide, Zinc Oxide, Calcium Pantothenate, Copper Gluconate, Manganese Sulfate, Riboflavin, Beta-Carotene, Pyridoxine Hydrochloride, Thiamine Mononitrate, Folic Acid, Biotin, Chromium Chloride, Potassium Iodide, Sodium Selenate, Sodium Molybdate, Phylloquinone, Vitamin D₃ and Cyanocobalamin.

### Protein:

**Honey Graham Crunch** - The protein source is a blend of soy protein isolate and milk proteins.

| | |
|---|---|
| Soy protein isolate | 74% |
| Milk proteins | 26% |

### Fat:

Honey Graham Crunch - The fat source is a blend of partially hydrogenated cottonseed and soybean, canola, high oleic safflower, and com oils, and soy lecithin.

| | |
|---|---|
| Partially hydrogenated cottonseed and soybean oil | 76% |
| Canola oil | 8% |
| High-oleic safflower oil | 8% |
| Corn oil | 4% |
| Soy lecithin | 4% |

### Carbohydrate:

Honey Graham Crunch - The carbohydrate source is a combination of high-fructose com syrup, brown sugar, maltodextrin, honey, crisp rice, glycerine, soy polysaccharide, and oat bran.

| | |
|---|---|
| High-fructose com syrup | 24% |
| Brown sugar | 21% |
| Maltodextrin | 12% |
| Honey | 11% |
| Crisp rice | 9% |
| Glycerine | 9% |
| Soy polysaccharide | 7% |
| Oat bran | 7% |

### C. ENSURE® HIGH PROTEIN

Usage: ENSURE HIGH PROTEIN is a concentrated, high-protein liquid food designed for people who require additional calories, protein, vitamins, and minerals in their diets. It can be used as an oral nutritional supplement with or between meals or, in appropriate amounts, as a meal replacement. ENSURE HIGH PROTEIN is lactose- and gluten-free, and is suitable for use by people recovering from general surgery or hip fractures and by patients at risk for pressure ulcers.

### Patient Conditions

- For patients who require additional calories, protein, vitamins, and minerals, such as patients recovering from general surgery or hip fractures, patients at risk for pressure ulcers, and patients on low-cholesterol diets

### Features

- Low in saturated fat
- Contains 6 g of total fat and < 5 mg of cholesterol per serving
- Rich, creamy taste
- Excellent source of protein, calcium, and other essential vitamins and minerals
- For low-cholesterol diets
- Lactose-free, easily digested

### Ingredients:

**Vanilla Supreme:** - 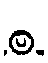 -D Water, Sugar (Sucrose), Maltodextrin (Corn), Calcium and Sodium Caseinates, High-Oleic Safflower Oil, Soy Protein Isolate, Soy Oil, Canola Oil, Potassium Citrate, Calcium Phosphate Tribasic, Sodium Citrate, Magnesium Chloride, Magnesium Phosphate Dibasic, Artificial Flavor, Sodium Chloride, Soy Lecithin, Choline Chloride, Ascorbic Acid, Carrageenan, Zinc Sulfate, Ferrous Suffate, Alpha-Tocopheryl Acetate, Gellan Gum, Niacinamide, Calcium Pantothenate, Manganese Sulfate, Cupric Sulfate, Vitamin A Palmitate, Thiamine Chloride Hydrochloride, Pyridoxine Hydrochloride, Riboflavin, Folio Acid, Sodium Motybdate, Chromium Chloride, Biotin, Potassium Iodide, Sodium Selenate, Phylloquinone, Vitamin D.3 and Cyanocobalamin.

### Protein:

The protein source is a blend of two high-biologic-value proteins: casein and soy.

| | |
|---|---|
| Sodium and calcium caseinates | 85% |
| Soy protein isolate | 15% |

### Fat:

The fat source is a blend of three oils: high-oleic safflower, canola, and soy.

| | |
|---|---|
| High-oleic safflower oil | 40% |
| Canola oil | 30% |
| Soy oil | 30% |

The level of fat in ENSURE HIGH PROTEIN meets American Heart Association (AHA) guidelines. The 6 grams of fat in ENSURE HIGH PROTEIN represent 24% of the total calories, with 2.6% of the fat being from saturated fatty acids and 7.9% from polyunsaturated fatty acids. These values are within the AHA guidelines of ≤ 30% of total calories from fat, < 1 0% of the calories from saturated fatty acids, and ≤ 1 0% of total calories from polyunsaturated fatty acids.

### Carbohydrate:

ENSURE HIGH PROTEIN contains a combination of maltodextrin and sucrose. The mild sweetness and flavor variety (vanilla supreme, chocolate royal, wild berry, and banana), plus VARI-FLAVORSO® Flavor Pacs in pecan, cherry, strawberry, lemon, and orange, help to prevent flavor fatigue and aid in patient compliance.

| **Vanilla and other nonchocolate flavors** | |
|---|---|
| Sucrose | 60% |
| Maltodextrin | 40% |

| **Chocolate** | |
|---|---|
| Sucrose | 70% |
| Maltodextrin | 30% |

### D. ENSURE ® LIGHT

Usage: ENSURE LIGHT is a low-fat liquid food designed for use as an oral nutritional supplement with or between meals. ENSURE LIGHT is lactose- and gluten-free, and is suitable for use in modified diets, including low-cholesterol diets.

### Patient Conditions:

- For normal-weight or overweight patients who need extra nutrition in a supplement that contains 50% less fat and 20% fewer calories than ENSURE
- For healthy adults who don't eat right and need extra nutrition

### Features:

- Low in fat and saturated fat
- Contains 3 g of total fat per serving and < 5 mg cholesterol
- Rich, creamy taste
- Excellent source of calcium and other essential vitamins and minerals
- For low-cholesterol diets
- Lactose-free, easily digested

### Ingredients:

**French Vanilla:** 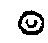 -D Water, Maltodextrin (Corn), Sugar (Sucrose), Calcium Caseinate, High-Oleic Safflower Oil, Canola Oil, Magnesium Chloride, Sodium Citrate, Potassium Citrate, Potassium Phosphate Dibasic, Magnesium Phosphate Dibasic, Natural and Artificial Flavor, Calcium Phosphate Tribasic, Cellulose Gel, Choline Chloride, Soy Lecithin, Carrageenan, Salt (Sodium Chloride), Ascorbic Acid, Cellulose Gum, Ferrous Sulfate, Alpha-Tocopheryl Acetate, Zinc Sulfate, Niacinamide, Manganese Sulfate, Calcium Pantothenate, Cupric Sulfate, Thiamine Chloride Hydrochloride, Vitamin A Palmitate, Pyridoxine Hydrochloride, Riboflavin, Chromium Chloride, Folic Acid, Sodium Molybdate, Biotin, Potassium Iodide, Sodium Selenate, Phylloquinone, Vitamin D₃ and Cyanocobalamin.

### Protein:

The protein source is calcium caseinate.

| | |
|---|---|
| Calcium caseinate | 100% |

### Fat

The fat source is a blend of two oils: high-oleic safflower and canola.

| | |
|---|---|
| High-oleic safflower oil | 70% |
| Canola oil | 30% |

The level of fat in ENSURE LIGHT meets American Heart Association (AHA) guidelines. The 3 grams of fat in ENSURE LIGHT represent 13.5% of the total calories, with 1.4% of the fat being from saturated fatty acids and 2.6% from polyunsaturated fatty acids. These values are within the AHA guidelines of ≤ 30% of total calories from fat, < 10% of the calories from saturated fatty acids, and ≤ 10% of total calories from polyunsaturated fatty acids.

### Carbohydrate

ENSURE LIGHT contains a combination of maltodextrin and sucrose. The chocolate flavor contains corn syrup as well. The mild sweetness and flavor variety (French vanilla, chocolate supreme, strawberry swirl), plus VARI-FLAVORS® Flavor Pacs in pecan, cherry, strawberry, lemon, and orange, help to prevent flavor fatigue and aid in patient compliance.

| **Vanilla and other nonchocolate flavors** | |
|---|---|
| Sucrose | 51 % |
| Maltodextrin | 49% |

| **Chocolate** | |
|---|---|
| Sucrose | 47.0% |
| Corn Syrup | 26.5% |
| Maltodextrin | 26.5% |

### Vitamins and Minerals

An 8-fl-oz serving of ENSURE LIGHT provides at least 25% of the RDIs for 24 key vitamins and minerals.

### Caffeine

Chocolate flavor contains 2.1 mg caffeine/8 fl oz.

### E. ENSURE PLUS®

Usage: ENSURE PLUS is a high-calorie, low-residue liquid food for use when extra calories and nutrients, but a normal concentration of protein, are needed. It is designed primarily as an oral nutritional supplement to be used with or between meals or, in appropriate amounts, as a meal replacement. ENSURE PLUS is lactose- and gluten-free. Although it is primarily an oral nutritional supplement, it can be fed by tube.

### Patient Conditions:

- For patients who require extra calories and nutrients, but a normal concentration of protein, in a limited volume
- For patients who need to gain or maintain healthy weight

### Features

- Rich, creamy taste
- Good source of essential vitamins and minerals

### Ingredients

**Vanilla:** 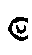 -D Water, Com Syrup, Maltodextrin (Corn), Corn Oil, Sodium and Calcium Caseinates, Sugar (Sucrose), Soy Protein Isolate, Magnesium Chloride, Potassium Citrate, Calcium Phosphate Tribasic, Soy Lecithin, Natural and Artificial Flavor, Sodium Citrate, Potassium Chloride, Choline Chloride, Ascorbic Acid, Carrageenan, Zinc Sulfate, Ferrous Sulfate, Alpha-Tocopheryl Acetate, Niacinamide, Calcium Pantothenate, Manganese Sulfate, Cupric Sulfate, Thiamine Chloride Hydrochloride, Pyridoxine Hydrochloride, Riboflavin, Vitamin A Palmitate, Folic Acid, Biotin, Chromium Chloride, Sodium Molybdate, Potassium Iodide, Sodium Selenite, Phylloquinone, Cyanocobalamin and Vitamin D₃.

### Protein

The protein source is a blend of two high-biologic-value proteins: casein and soy.

| | |
|---|---|
| Sodium and calcium caseinates | 84% |
| Soy protein isolate | 16% |

### Fat

The fat source is corn oil.

| | |
|---|---|
| Corn oil | 100% |

### Carbohydrate

ENSURE PLUS contains a combination of maltodextrin and sucrose. The mild sweemess and flavor variety (vanilla, chocolate, strawberry. coffee, buffer pecan, and eggnog), plus VARI-FLAVORS® Flavor Pacs in pecan, cherry, strawberry. lemon, and orange, help to prevent flavor fatigue and aid in patient compliance.

| **Vanilla, strawberry, butter pecan, and coffee flavors** | |
|---|---|
| Corn Syrup | 39% |
| Maltodextrin | 38% |
| Sucrose | 23% |

| **Chocolate and eggnog flavors** | |
|---|---|
| Corn Syrup | 36% |
| Maltodextrin | 34% |
| Sucrose | 30% |

### Vitamins and Minerals

An 8-fl-oz serving of ENSURE PLUS provides at least 15% of the RDIs for 25 key Vitamins and minerals.

### Caffeine

Chocolate flavor contains 3.1 mg Caffeine/8 fl oz. Coffee flavor contains a trace amount of caffeine.

### F. ENSURE PLUS® HN

Usage: ENSURE PLUS HN is a nutritionally complete high-calorie, high-nitrogen liquid food designed for people with higher calorie and protein needs or limited volume tolerance. It may be used for oral supplementation or for total nutritional support by tube. ENSURE PLUS HN is lactose- and gluten-free.

### Patient Conditions:

- For patients with increased calorie and protein needs, such as following surgery or injury
- For patients with limited volume tolerance and early satiety

### Features

- For supplemental or total nutrition
- For oral or tube feeding
- 1.5 CaVmL
- High nitrogen
- Calorically dense

### Ingredients

**Vanilla:** 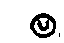 -D Water, Maltodextrin (Corn), Sodium and Calcium Caseinates, Corn Oil, Sugar (Sucrose), Soy Protein Isolate, Magnesium Chloride, Potassium Citrate, Calcium Phosphate Tribasic, Soy Lecithin, Natural and Artificial Flavor, Sodium Citrate, Choline Chloride, Ascorbic Acid, Taurine, L-Carnitine, Zinc Sulfate, Ferrous Sulfate, Alpha-Tocopheryl Acetate, Niacinamide, Carrageenan, Calcium Pantothenate, Manganese Sulfate, Cupric Sulfate, Thiamine Chloride Hydrochloride, Pyridoxine Hydrochloride, Riboflavin, Vitamin A Palmitate, Folic Acid, Biotin, Chromium Chloride, Sodium Molybdate, Potassium Iodide, Sodium Selenite, Phylloquinone, Cyanocobalamin and Vitamin D₃.

### G. ENSURE® POWDER

Usage: ENSURE POWDER (reconstituted with water) is a low-residue liquid food designed primarily as an oral nutritional supplement to be used with or between meals. ENSURE POWDER is lactose- and gluten-free, and is suitable for use in modified diets, including low-cholesterol diets.

### Patient Conditions:

- For patients on modified diets
- For elderly patients at nutrition risk
- For patients recovering from illness/surgery
- For patients who need a low-residue diet

### Features

- Convenient, easy to mix
- Low in saturated fat
- Contains 9 g of total fat and < 5 mg of cholesterol per serving
- High in vitamins and minerals
- For low-cholesterol diets
- Lactose-free, easily digested
**Ingredients:** 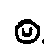 -D Corn Syrup, Maltodextrin (Corn), Sugar (Sucrose), Com Oil, Sodium and Calcium Caseinates, Soy Protein Isolate, Artificial Flavor, Potassium Citrate, Magnesium Chloride, Sodium Citrate, Calcium Phosphate Tribasic, Potassium Chloride, Soy Lecithin, Ascorbic Acid, Choline Chloride, Zinc Sulfate, Ferrous Sulfate, Alpha-Tocopheryl Acetate, Niacinamide, Calcium Pantothenate, Manganese Sulfate, Thiamine Chloride Hydrochloride, Cupric Sulfate, Pyridoxine Hydrochloride, Riboflavin, Vitamin A Palmitate, Folic Acid, Biotin, Sodium Molybdate, Chromium Chloride, Potassium Iodide, Sodium Selenate, Phylloquinone, Vitamin D₃ and Cyanocobalamin.

### Protein

The protein source is a blend of two high-biologic-value proteins: casein and soy.

| | |
|---|---|
| Sodium and calcium caseinates | 84% |
| Soy protein isolate | 16% |

### Fat

The fat source is corn oil.

| | |
|---|---|
| Corn oil | 100% |

### Carbohydrate

ENSURE POWDER contains a combination of corn syrup, maltodextrin, and sucrose. The mild sweetness of ENSURE POWDER, plus VARI-FLAVORS® Flavor Pacs in pecan, cherry, strawberry, lemon, and orange, helps to prevent flavor fatigue and aid in patient compliance.

| **Vanilla** | |
|---|---|
| Corn Syrup | 35% |
| Maltodextrin | 35% |
| Sucrose | 30% |

### H. ENSURE® PUDDING

Usage: ENSURE PUDDING is a nutrient-dense supplement providing balanced nutrition in a nonliquid form to be used with or between meals. It is appropriate for consistency-modified diets (e.g., soft, pureed, or full liquid) or for people with swallowing impairments. ENSURE PUDDING is gluten-free.

### Patient Conditions:

- For patients on consistency-modified diets (e.g., soft, pureed, or full liquid)
- For patients with swallowing impairments
- **Features**
- Rich and creamy, good taste
- Good source of essential vitamins and minerals Convenient-needs no refrigeration
- Gluten-free
**Nutrient Profile per 5 oz:** Calories 250, Protein 10.9%, Total Fat 34.9%, Carbohydrate 54.2%

### Ingredients:

**Vanilla:** 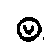 -D Nonfat Milk, Water, Sugar (Sucrose), Partially Hydrogenated Soybean Oil, Modified Food Starch, Magnesium Sulfate. Sodium Stearoyl Lactylate, Sodium Phosphate Dibasic, Artificial Flavor, Ascorbic Acid, Zinc Sulfate, Ferrous Sulfate, Alpha-Tocopheryl Acetate, Choline Chloride, Niacinamide, Manganese Sulfate, Calcium Pantothenate, FD&C Yellow #5, Potassium Citrate, Cupric Sulfate, Vitamin A Palmitate, Thiamine Chloride Hydrochloride, Pyridoxine Hydrochloride, Riboflavin, FD&C Yellow #6, Folic Acid, Biotin, Phyiloquinone, Vitamin D3 and Cyanocobalamin.

### Protein

The protein source is nonfat milk.

| | |
|---|---|
| Nonfat milk | 100% |

### Fat

The fat source is hydrogenated soybean oil.

| | |
|---|---|
| Hydrogenated soybean oil | 100% |

x

### Carbohydrate

ENSURE PUDDING contains a combination of sucrose and modified food starch. The mild sweetness and flavor variety (vanilla, chocolate, butterscotch, and tapioca) help prevent flavor fatigue. The product contains 9.2 grams of lactose per serving.

| **Vanilla and other nonchocolate flavors** | |
|---|---|
| Sucrose | 56% |
| Lactose | 27% , |
| Modified food starch | 17% |

| **Chocolate** | |
|---|---|
| Sucrose | 58% |
| Lactose | 26% |
| Modified food starch | 16% |

### I. ENSURE® WITH FIBER

Usage: ENSURE WITH FIBER is a fiber-containing, nutritionally complete liquid food designed for people who can benefit from increased dietary fiber and nutrients. ENSURE WITH FIBER is suitable for people who do not require a low-residue diet. It can be fed orally or by tube, and can be used as a nutritional supplement to a regular diet or, in appropriate amounts, as a meal replacement. ENSURE WITH FIBER is lactose- and gluten-free, and is suitable for use in modified diets, including low-cholesterol diets.

### Patient Conditions

- For patients who can benefit from increased dietary fiber and nutrients

### Features

- New advanced formula-low in saturated fat, higher in vitamins and minerals
- Contains 6 g of total fat and < 5 mg of cholesterol per serving
- Rich, creamy taste
- Good source of fiber
- Excellent source of essential vitamins and minerals
- For low-cholesterol diets
- Lactose- and gluten-free

### Ingredients

**Vanilla:** 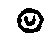 -D Water, Maltodextrin (Corn), Sugar (Sucrose), Sodium and Calcium Caseinates, Oat Fiber, High-Oleic Safflower Oil, Canola Oil, Soy Protein Isolate, Corn Oil, Soy Fiber, Calcium Phosphate Tribasic, Magnesium Chloride, Potassium Citrate. Cellulose Gel, Soy Lecithin, Potassium Phosphate Dibasic, Sodium Citrate, Natural and Artificial Flavors, Choline Chloride, Magnesium Phosphate, Ascorbic Acid, Cellulose Gum, Potassium Chloride, Carrageenan, Ferrous Sulfate, Alpha-Tocopheryl Acetate, Zinc Sulfate, Niacinamide, Manganese Sulfate, Calcium Pantothenate, Cupric Sulfate, Vitamin A Palmitate, Thiamine Chloride Hydrochloride, Pyridoxine Hydrochloride, Riboflavin, Folic Acid, Chromium Chloride, Biotin, Sodium Molybdate, Potassium Iodide, Sodium Selenate, Phylloquinone, Vitamin D₃ and Cyanocobalamin.

### Protein

The protein source is a blend of two high-biologic-value proteins- casein and soy.

| | |
|---|---|
| Sodium and calcium caseinates | 80% |
| Soy protein isolate | 20% |

### Fat

The fat source is a blend of three oils: high-oleic safflower, canola, and corn.

| | |
|---|---|
| High-oleic safflower oil | 40% |
| Canola oil | 40% |
| Corn oil | 20% |

The level of fat in ENSURE WITH FIBER meets American Heart Association (AHA) guidelines. The 6 grams of fat in ENSURE WITH FIBER represent 22% of the total calories, with 2.01 % of the fat being from saturated fatty acids and 6.7% from polyunsaturated fatty acids. These values are within the AHA guidelines of ≤ 30% of total calories from fat, < 10% of the calories from saturated fatty acids, and ≤ 1 0% of total calories from polyunsaturated fatty acids.

### Carbohydrate

ENSURE WITH FIBER contains a combination of maltodextrin and sucrose. The mild sweetness and flavor variety (vanilla, chocolate, and butter pecan), plus VARI-FLAVORS® Flavor Pacs in pecan, cherry, strawberry, lemon, and orange, help to prevent flavor fatigue and aid in patient compliance.

| **Vanilla and other nonchocolate flavors** | |
|---|---|
| Maltodextrin | 66% |
| Sucrose | 25% |
| Oat Fiber | 7% |
| Soy Fiber | 2% |

| **Chocolate** | |
|---|---|
| Maltodextrin | 55% |
| Sucrose | 36% |
| Oat Fiber | 7% |
| Soy Fiber | 2% |

### Fiber

The fiber blend used in ENSURE WITH FIBER consists of oat fiber and soy polysaccharide. This blend results in approximately 4 grams of total dietary fiber per 8-fl-oz can. The ratio of insoluble to soluble fiber is 95:5.

The various nutritional supplements described above and known to others of skill in the art can be substituted and/or supplemented with the PUFAs of this invention.

### J. Oxepa™ Nutritional Product

Oxepa is low-carbohydrate, calorically dense enteral nutritional product designed for the dietary management of patients with or at risk for ARDS. It has a unique combination of ingredients, including a patented oil blend containing eicosapentaenoic acid (EPA from fish oil), γ-linolenic acid (GLA from borage oil), and elevated antioxidant levels.

### Caloric Distribution:

- Caloric density is high at 1.5 Cal/mL (355 Cal/8 fl oz), to minimize the volume required to meet energy needs.
- The distribution of Calories in Oxepa is shown in Table 7.

**Table 7.**

| **Caloric Distribution of Oxepa** | | | |
|---|---|---|---|
| | **per 8 fl oz.** | **per liter** | **% of Cal** |
| Calories | 355 | 1,500 | -- |
| Fat (g) | 22.2 | 93.7 | 55.2 |
| Carbohydrate (g) | 25 | 105.5 | 28.1 |
| Protein (g) | 14.8 | 62.5 | 16.7 |
| Water (g) | 186 | 785 | -- |

### Fat:

- Oxepa contains 22.2 g of fat per 8-fl oz serving (93.7 g/L).
- The fat source is a oil blend of 31.8% canola oil, 25% medium-chain triglycerides (MCTs), 20% borage oil, 20% fish oil, and 3.2 % soy lecithin. The typical fatty acid profile of Oxepa is shown in Table 8.
- Oxepa provides a balanced amount of polyunsaturated, monounsaturated, and saturated fatty acids, as shown in Table 10.
- Medium-chain trigylcerides (MCTs) -- 25% of the fat blend -- aid gastric emptying because they are absorbed by the intestinal tract without emulsification by bile acids.

The various fatty acid components of Oxepa™ nutritional product can be substituted and/or supplemented with the PUFAs of this invention.

**Table 8.**

| **Typical Fatty Acid Profile** | | | |
|---|---|---|---|
| | % Total Fatty Acids | g/8 fl oz* | g/L* |
| Caproic (6:0) | 0.2 | 0.04 | 0.18 |
| Caprylic (8:0) | 14.69 | 3.1 | 13.07 |
| Capric (10:0) | 11.06 | 2.33 | 9.87 |
| Palmitic (16:0) | 5.59 | 1.18 | 4.98 |
| Palmitoleic (16:1n-7) | 1.82 | 0.38 | 1.62 |
| Stearic (18:0) | 1.84 | 0.39 | 1.64 |
| Oleic (18:1n-9) | 24.44 | 5.16 | 21.75 |
| Linoleic (18:2n-6) | 16.28 | 3.44 | 14.49 |
| α-Linolenic (18:3n-3) | 3.47 | 0.73 | 3.09 |
| γ-Linolenic (18:3n-6) | 4.82 | 1.02 | 4.29 |
| Eicosapentaenoic (20:5n-3) | 5.11 | 1.08 | 4.55 |
| n-3-Docosapentaenoic (22:5n-3) | 0.55 | 0.12 | 0.49 |
| Docosahexaenoic (22:6n-3) | 2.27 | 0.48 | 2.02 |
| Others | 7.55 | 1.52 | 6.72 |

| | | | |
|---|---|---|---|
| * Fatty acids equal approximately 95% of total fat. | | | |

**Table 9.**

| **Fat Profile of Oxepa.** | |
|---|---|
| % of total calories from fat | 55.2 |
| Polyunsaturated fatty acids | 31.44 g/L |
| Monounsaturated fatty acids | 25.53 g/L |
| Saturated fatty acids | 32.38 g/L |
| n-6 to n-3 ratio | 1.75:1 |
| Cholesterol | 9.49 mg/8 fl oz 40.1 mg/L |

### Carbohydrate:

- The carbohydrate content is 25.0 g per 8-fl-oz serving (105.5 g/L).
- The carbohydrate sources are 45% maltodextrin (a complex carbohydrate) and 55% sucrose (a simple sugar), both of which are readily digested and absorbed.
- The high-fat and low-carbohydrate content of Oxepa is designed to minimize carbon dioxide (CO₂) production. High CO₂ levels can complicate weaning in ventilator-dependent patients. The low level of carbohydrate also may be useful for those patients who have developed stress-induced hyperglycemia.
- Oxepa is lactose-free.

Dietary carbohydrate, the amino acids from protein, and the glycerol moiety of fats can be converted to glucose within the body. Throughout this process, the carbohydrate requirements of glucose-dependent tissues (such as the central nervous system and red blood cells) are met. However, a diet free of carbohydrates can lead to ketosis, excessive catabolism of tissue protein, and loss of fluid and electrolytes. These effects can be prevented by daily ingestion of 50 to 100 g of digestible carbohydrate, if caloric intake is adequate. The carbohydrate level in Oxepa is also sufficient to minimize gluconeogenesis, if energy needs are being met.

### Protein:

- Oxepa contains 14.8 g of protein per 8-fl-oz serving (62.5 g/L).
- The total calorie/nitrogen ratio (150:1) meets the need of stressed patients.
- Oxepa provides enough protein to promote anabolism and the maintenance of lean body mass without precipitating respiratory problems. High protein intakes are a concern in patients with respiratory insufficiency. Although protein has little effect on CO₂ production, a high protein diet will increase ventilatory drive.
- The protein sources of Oxepa are 86.8% sodium caseinate and 13.2% calcium caseinate.

All publications and patent applications mentioned in this specification are indicative of the level of skill of those skilled in the art to which this invention pertains.

The invention now being fully described, it will be apparent to one of ordinary skill in the art that many changes and modifications can be made thereto without departing from the spirit or scope of the appended claims.

## Claims

1. A purified or isolated polypeptide having Δ5 desaturase activity, which polypeptide is capable of desaturating a fatty acid molecule at carbon 5 from the carboxyl end of said fatty acid, said polypeptide having an amino acid sequence which has at least 60 % homology to the 446 amino acid sequence of SEQ ID NO:2.

2. A polypeptide according to claim 1 having an amino acid sequence which has at least 80% homology to the 446 amino acid sequence of SEQ ID NO:2.

3. A polypeptide according to claim 1 having an amino acid sequence which has at least 90% homology to the 446 amino acid sequence of SEQ ID NO:2.

4. A purified or isolated polypeptide having Δ5 desaturase activity, which polypeptide is a deletion mutant of SEQ ID NO: 2, said polypeptide being capable of desaturating a fatty acid molecule at carbon 5 from the carboxyl end of said fatty acid.

5. A polypeptide according to claim 1, 2, 3 or 4 wherein said polypeptide includes an amino acid motif selected from the group consisting of residues 30-38, 41-44, 171-175, 203-212 and 387-394 of SEQ ID NO:2.

6. A polypeptide according to claim 5 wherein said polypeptide comprises residues 30-38, 41-44, 171-175, 203-212 and 387-394 of SEQ ID NO:2.

7. A polypeptide according to any one of the preceding claims comprising SEQ ID NO:2.

8. An isolated nucleic acid encoding a polypeptide as defined in any one of claims 1 to 7.

9. An isolated nucleic acid according to claim 8 which comprises SEQ ID NO:1.

10. A nucleic acid construct comprising a nucleic acid as defined in claim 8 or 9 linked to a heterologous nucleic acid.

11. A nucleic acid construct comprising a nucleic acid as defined in claim 10 operably linked to a promoter.

12. A recombinant host cell transformed with the nucleic acid construct of claim 10 or 11.

13. A host cell according to claim 12 which is a microbial host cell.

14. A host cell according to claim 13 which is a yeast cell.

15. A host cell according to any one of claims 12 to 14 which is enriched for 20:3, 20:4 or ω-3 20:4 fatty acids compared to a cell untransformed with said nucleic acid construct of claim 10 or 11.

16. A method for the production of the fatty acid arachidonic acid which method comprises:
growing the host cell of any one of claims 12 to 15 in the presence of dihomo-γ-linolenic acid, under conditions wherein said acid is converted to arachidonic acid by the expression of the polypeptide of any one of claims 1 to 7;
recovering the fatty acid arachidonic acid from the culture.

17. A method for obtaining a long chain polyunsaturated fatty acid, which method comprises:
growing the host cell of any one of claims 12 to 15 in the presence of dihomo-γ-linolenic acid, under conditions wherein the polypeptide of any one of claims 1 to 7 is produced, resulting in the biosynthesis of a long chain polyunsaturated fatty acid; and
recovering the fatty acid from the culture.

18. A method according to claim 17 wherein said fatty acid is selected from the group consisting of ARA, DGLA or EPA.

19. A method according to any one of claims 16 to 18 which further comprises formulating the fatty acid into a product selected from the group:
a pharmaceutical composition comprising a pharmaceutically acceptable carrier;
a nutritional formula;
an infant formula;
a dietary supplement;
a dietary substitute;
a cosmetic; and
an animal feed.

20. A method according to claim 19 wherein the nutritional formula, infant formula, dietary supplement or dietary substitute contains at least one macronutrient selected from the group consisting of coconut oil, soy oil, canola oil, mono- and di-glycerides, glucose, edible lactose, electrodialysed whey, electrodialysed skimmed milk, milk whey, soy protein, and other protein hydrolysates.

21. A method according to claim 20 wherein said nutritional formula, infant formula, dietary supplement or dietary substitute contains at least one vitamin selected from the group consisting of vitamins A, C, D, E and B complex; and at least one mineral selected from the group consisting of calcium, magnesium, zinc, manganese, sodium, potassium, phosphorus, copper, chloride, iodine, selenium and iron.

22. The use of a microbial host cell of claim 13 or 14 for the production of a fatty acid.

23. The use according to claim 22 for the production of a product comprising said fatty acid, said product being selected from the group:
a pharmaceutical composition comprising said fatty acid and a pharmaceutically acceptable carrier;
a nutritional formula;
an infant formula;
a dietary supplement;
a dietary substitute;
a cosmetic; and
an animal feed.

## Patentansprüche

1. Gereinigtes oder isoliertes Polypeptid mit Δ5-Desaturase-Aktivität, wobei das Polypeptid zur Desaturierung eines Fettsäuremoleküls am 5. Kohlenstoff vom Carboxylende der Fettsäure fähig ist und das Polypeptid eine Aminosäuresequenz aufweist, die zumindest 60 % Homologie zur 446-Aminosäuresequenz von Seq.-ID Nr. 2 aufweist.

2. Polypeptid nach Anspruch 1, das eine Aminosäuresequenz aufweist, die zumindest 80 % Homologie zur 446-Aminosäuresequenz von Seq.-ID Nr. 2 aufweist.

3. Polypeptid nach Anspruch 1, das eine Aminosäuresequenz aufweist, die zumindest 90 % Homologie zur 446-Aminosäuresequenz von Seq.-ID Nr. 2 aufweist.

4. Gereinigtes oder isoliertes Polypeptid mit Δ5-Desaturaseaktivität, wobei das Polypeptid eine Deletionsmutante von Seq.-ID Nr. 2 ist und zur Desaturierung eines Fettsäuremoleküls am 5. Kohlenstoff vom Carboxylende der Fettsäure fähig ist.

5. Polypeptid nach Anspruch 1, 2, 3 oder 4, worin das Polypeptid ein Aminosäuremotiv umfasst, das aus der aus den Resten 30-38, 41-44, 171-175, 203-212 und 387-394 von Seq.-ID Nr. 2 bestehenden Gruppe ausgewählt ist.

6. Polypeptid nach Anspruch 5, worin das Polypeptid die Reste 30-38, 41-44, 171-175, 203-212 und 387-394 von Seq.-ID Nr. 2 umfasst.

7. Polypeptid nach einem der vorangegangenen Ansprüche, das die Seq.-ID Nr. 2 umfasst.

8. Isolierte Nucleinsäure, die für ein Polypeptid nach einem der Ansprüche 1 bis 7 kodiert.

9. Isolierte Nucleinsäure nach Anspruch 8, welche die Seq.-ID Nr. 1 umfasst.

10. Nucleinsäurekonstrukt, umfassend eine Nucleinsäure nach Anspruch 8 oder 9, die an eine heterologe Nucleinsäure gebunden ist.

11. Nucleinsäurekonstrukt, umfassend eine Nucleinsäure nach Anspruch 10, die operabel an einen Promotor gebunden ist.

12. Rekombinante Wirtszelle, die mit einem Nucleinsäurekonstrukt nach Anspruch 10 oder 11 transformiert ist.

13. Wirtszelle nach Anspruch 12, die eine Mikrobenwirtszelle ist.

14. Wirtszelle nach Anspruch 13, die eine Hefezelle ist.

15. Wirtszelle nach einem der Ansprüche 12 bis 14, die im Vergleich zu einer Zelle, die nicht mit dem Nucleinsäurekonstrukt nach Anspruch 10 oder 11 transformiert ist, mit 20:3-, 20:4- oder ω-3-20:4-Fettsäuren angereichert ist.

16. Verfahren zur Herstellung einer Arachidonfettsäure, wobei das Verfahren Folgendes umfasst:
Züchten einer Wirtszelle nach einem der Ansprüche 12 bis 15 in Gegenwart einer Dihomo-γ-linolensäure unter Bedingungen, unter denen die Säure durch Expression eines Polypeptids nach einem der Ansprüche 1 bis 7 in Arachidonsäure übergeführt wird;
Gewinnen der Arachidonfettsäure aus der Kultur.

17. Verfahren zur Herstellung einer langkettigen, mehrfach ungesättigten Fettsäure, wobei das Verfahren Folgendes umfasst:
Züchten einer Wirtszelle nach einem der Ansprüche 12 bis 15 in Gegenwart einer Dihomo-γ-linolensäure unter Bedingungen, unter denen das Polypeptid nach einem der Ansprüche 1 bis 7 gebildet wird, was zur Biosynthese einer langkettigen, mehrfach ungesättigten Fettsäure führt; und
Gewinnen der Fettsäure aus der Kultur.

18. Verfahren nach Anspruch 17, worin die Fettsäure aus der aus ARA, DGLA und EPA bestehenden Gruppe ausgewählt ist.

19. Verfahren nach einem der Ansprüche 16 bis 18, das außerdem die Formulierung der Fettsäure zu einem Produkt umfasst, das aus der folgenden Gruppe ausgewählt ist:
einen pharmazeutisch annehmbaren Träger umfassende pharmazeutische Zusammensetzung;
Nahrungsmittelformulierung;
Kindernahrung;
Nahrungsmittelergänzung;
Nahrungsmittelersatz;
Kosmetikum; und
Tierfutter.

20. Verfahren nach Anspruch 19, worin die Nahrungsmittelformulierung, die Kindernahrung, die Nahrungsmittelergänzung oder der Nahrungsmittelersatz zumindest einen Makronährstoff umfasst, der aus der aus Kokosnussöl, Sojaöl, Canolaöl, Monound Diglyceriden, Glucose, essbarer Lactose, elektrodialysierter Molke, elektrodialysierter Magermilch, Molke, Sojaprotein und anderen Proteinhydrolysaten bestehenden Gruppe ausgewählt ist.

21. Verfahren nach Anspruch 20, worin die Nahrungsmittelformulierung, die Kindernahrung, die Nahrungsmittelergänzung oder der Nahrungsmittelersatz zumindest ein Vitamin, das aus der aus Vitamin A, C, D, E und Vitamin-B-Komplexen bestehenden Gruppe ausgewählt ist; und zumindest ein Mineral, das aus der aus Calcium, Magnesium, Zink, Mangan, Natrium, Kalium, Phosphor, Kupfer, Chlorid, lod, Selen und Eisen bestehenden Gruppe ausgewählt ist, umfassen.

22. Verwendung einer Mikrobenwirtszelle nach Anspruch 13 oder 14 zur Herstellung einer Fettsäure.

23. Verwendung nach Anspruch 22 zur Herstellung eines Produkts, das die Fettsäure umfasst, wobei das Produkt aus der folgenden Gruppe ausgewählt ist:
einen pharmazeutisch annehmbaren Träger umfassende pharmazeutische Zusammensetzung;
Nahrungsmittelformulierung;
Kindernahrung;
Nahrungsmittelergänzung;
Nahrungsmittelersatz;
Kosmetikum; und
Tierfutter.

## Revendications

1. Polypeptide purifié ou isolé ayant une activité de Δ5 désaturase, lequel polypeptide est capable de désaturation d'une molécule d'acide gras au carbone 5 à partir de l'extrémité carboxyle dudit acide gras, ledit polypeptide ayant une s équence d'acides aminés qui a au moins 60% d'homologie avec la séquence de 446 acides aminés de SEQ ID NO:2.

2. Polypeptide selon la revendication 1 ayant une séquence d'acides aminés qui a au moins 80% d'homologie avec la séquence de 446 acides aminés de SEQ ID NO:2.

3. Polypeptide selon la revendication 1 ayant une séquence d'acides aminés qui a au moins 90% d'homologie avec la séquence de 446 acides aminés de SEQ ID NO:2.

4. Polypeptide purifié ou isolé ayant une activité de Δ5 désaturase, lequel polypeptide est un mutant par délétion de SEQ ID NO:2, ledit polypeptide étant capable de désaturation d'une molécule d'acide gras au carbone 5 à partir de l'extrémité carboxyle dudit acide gras.

5. Polypeptide selon la revendication 1, 2, 3 ou 4 où ledit po lypeptide contient un motif d'acides aminés sélectionné dans le groupe consistant en résidus 30-38, 41-44, 171-175, 203-212 et 387-394 de SEQ ID NO:2.

6. Polypeptide selon la revendication 5 où ledit polypeptide comprend des résidus 30-38, 41 -44, 171 -175, 203-212 et 387-394 de SEQ ID NO:2.

7. Polypeptide selon l'une quelconque des revendications précédentes, comprenant SEQ ID NO:2.

8. Acide nucléique isolé codant pour un polypeptide tel que défini dans l'une quelconque des revendications 1 à 7.

9. Acide nucléique isolé selon la revendication 8 qui comprend SEQ ID NO:1.

10. Construction d'acide nucléique comprenant un acide nucléique tel que défini à la revendication 8 ou la revendication 9 enchaîné à un acide nucléique hétérologue.

11. Construction d'acide nucléique comprenant un acide nucléique tel que défini à la revendication 10 opérativement enchaîné à un promoteur.

12. Cellule hôte recombinante transformée par la construction d'acide nucléique de la revendication 10 ou 11.

13. Cellule hôte selon la revendication 12 qui est une cellule hôte microbienne.

14. Cellule hôte selon la revendication 13 qui est une cellule de levure.

15. Celle hôte selon l'une quelconque des revendications 12 à 14 qui est enrichie pour des acides gras 20:3, 20:4 ou ω-3 20:4 en comparaison avec une cellule non transformée par ladite construction d'acide nucléique de la revendication 10 ou 11.

16. Méthode pour la production de l'acide gras acide arachidonique laquelle méthode comprend:
la croissance de la cellule h ôte de l'une quelconque des revendications 12 à 15 en présence de l'acide dihomo-γ-linolénique, dans des conditions où ledit acide est converti en acide arachidonique par l'expression du polypeptide de l'une quelconque des revendications 1 à 7;
la récupération de l'acide gras acide arachidonique de la culture.

17. Méthode d'obtention d'un acide gras polyinsaturé à chaîne longue, laquelle méthode comprend:
la croissance de la cellule hôte de l'une quelconque des revendications 12 à 15 en présence d'acide dihomo -γ-linolénique dans des conditions où le polypeptide de l'une quelconque des revendications 1 à 7 est produit avec pour résultat la biosynthèse d'un acide gras insaturé à chaîne longue; et
la récupération de l'acide gras de la culture.

18. Méthode selon la revendication 17 où ledit acide gras est sélectionné dans le groupe consistant en ARA, DGLA ou EPA.

19. Méthode selon l'une quelconque des revendications 16 à 18 qui comprend de plus la formulation de l'acide gras en un produit sélectionné dans le groupe:
composition pharmaceutique comprenant un
support pharmaceutiquement acceptable;
formule nutritionnelle;
formule infantile;
complément diététique;
substitut diététique
cosmétique; et
alimentation animale.

20. Méthode selon la revendication 19 où la formule nutritionnelle, la formule pour enfant, le complément diététique ou le substitut diététique contient au moins un macronutrient sélectionné dans le groupe consistant en huile de noix de coco, huile de soja, huile de canola , mono- et di -glycérides, glucose, lactose comestible, lactosérum électrodialysé, lait écrémé- électrodialysé, lactosérum électrodialysé, lait écrémé électrodialysé, lactosérum de lait, protéine du soja et autres hydrolysats de protéines.

21. Méthode selon la revendication 20 où ladite formule nutritionnelle, formule pour enfant, complément diététique ou substitut diététique contient au moins une vitamine sélectionnée dans le groupe consistant en vitamines A, C, D, E et B complexe; et au moins un minéral sélectionné dans le groupe consistant en calcium, magnésium, zinc, manganèse, sodium, potassium, phosphore, cuivre, chlore, iode, sélénium et fer.

22. Utilisation d'une cellule hôte microbienne de la revendication 13 ou 14 pour la production d'un acide gras.

23. Utilisation selon la revendication 22 pour la production d'un produit comprenant ledit acide gras, ledit produit étant sélectionné dans le groupe:
une composition pharmaceutique comprenant ledit
acide gras et un support pharmaceutiquement acceptable;
une formule nutritionnelle;
une formule pour enfant;
un complément diététique;
un substitut diététique;
un cosmétique; et
un aliment pour animal.
